# EUROPEAN PATENT APPLICATION

(11) **EP 3 381 455 A1**
(43) Date of publication of application: **03.10.2018**
(21) Application number: 18152818.3
(22) Date of filing: 13.02.2013
(51) Int. Cl.: A61K 31/505, A61P 25/14, A61K 31/4409, A61K 31/44, A61K 9/28, A61K 9/48

(54) **METHODS FOR TREATING AN IMPAIRMENT IN GAIT AND/OR BALANCE IN PATIENTS WITH MULTIPLE SCLEROSIS USING AN AMINOPYRIDINE**

(30) Priority: 13.02.2012 US 201261598332 P; 30.07.2012 US 201261677466 P
(62) Divisional of application: 13706858.1
(71) Applicant: Acorda Therapeutics, Inc., Ardsley, NY 10502 (US)
(72) Inventor: PARDO, Gabriel, Oklahoma City, OK Oklahoma 73103 (US); SUAREZ ZAMBRANO, Gustavo, Adolfo, Lincoln Park, NJ New Jersey 07035 (US); FJELDSTAD, Cecilie, Oklahoma City, OK Oklahoma 73102 (US)
(74) Representative: Carpmaels & Ransford LLP

(57) **Abstract**

Disclosed herein is use of one or more aminopyridines in methods and compositions for treatment of impairments in gait or balance in patients with multiple sclerosis.

## Description

### PRIORITY BENEFIT

This application claims the benefit of U.S. provisional application No. 61/598,332, filed on February 13, 2012, and U.S. provisional application No. 61/677,466, filed on July 30, 2012, each of which is incorporated herein by reference in its entirety.

### 1. FIELD OF INVENTION

The invention relates to improvement of gait or balance in patients with multiple sclerosis using one or more aminopyridines.

### 2. BACKGROUND

### 2.1 Multiple Sclerosis

Multiple Sclerosis (MS) is a chronic immune-mediated disease of the central nervous system (CNS) with both inflammatory and degenerative components. It is the most common neurologic, disabling disease in young adults (Frohman, 2003, The Medical Clinics of North America, 87(4): 867-897, viii-ix). Permanent neurological dysfunction can result from incomplete recovery from acute relapses or as a consequence of slow progression of disability.

MS may affect different neurological systems such as visual, sensory, cerebellar and/or motor. Deficits secondary to the involvement of any of these areas can result in alteration of gait, often seen through postural imbalance. Walking impairment and inactivity are primary concerns for individuals with MS. This was verified by Harris Interactive survey which reported 64% of individuals with MS experienced trouble walking, and 54% reported losing balance at least twice a week. Approximately 94% found the walking and balance problems to be somewhat disruptive to their overall daily living.

The effect of ambulatory dysfunction on regular activities is present in MS individuals with various degrees of disability. The functional implication varies from limiting the length of jogging for someone with minimal deficits, to impairing the ability to transfer to the toilet for others with more significant disability. Postural imbalance and the resulting gait alteration are significant problems for many MS patients, limiting their regular activities and increasing the risk of injury through fails. In clinical practice, balance dysfunction is often reported by patients before it is clinically evident on a conventional physical examination.

Prompt identification, characterization and treatment of balance alteration can be beneficial.

### 2.2 Aminopyridines

An exemplary property of certain aminopyridines is that they are potassium channel blockers.

4-aminopyridine (4-AP) is an example of an aminopyridine with such potassium channel blocking properties. At 4-AP plasma concentrations obtained in clinical studies, which are typically <1 microM (94 ng/mL⁻¹), the potassium channel blocking activity of 4-AP appears to be selective for certain types of these channels. Interestingly, at high concentration (such as at millimolar concentrations) 4-AP is a broad-spectrum blocker of potassium channels. The clinical neurologic effects of 4-AP are consistent with the molecular mechanism of potassium channel blockade. At the cellular level, this action may increase neuronal excitability, relieve conduction block in demyelinated axons, and potentiate synaptic and neuromuscular transmission.

Studies of 4-aminopyridine have been conducted using intravenous (i.v.) administration and oral administration with immediate-release (IR), controlled-release (CR) or sustained-release (SR) formulations. Administration of IR capsules resulted in rapid and shortlasting peaks of 4-aminopyridine in the plasma. Early pharmacokinetic studies were conducted using an immediate release (IR) formulation for oral administration, which consisted of 4-aminopyridine powder in a gelatin-based capsule or oral solution. Administration resulted in rapidly changing 4-aminopyridine plasma levels that were not well tolerated. A sustained-release matrix tablet was then developed. The 4-aminopyridine-SR matrix tablet showed improved stability and an appropriate pharmacokinetic profile for dosing twice daily.

Sustained release compositions of 4-aminopyridine and related use of such compositions are set forth, e.g., in US Patent 5,370,879, US Patent 5,540,938; US Patent 8,007,826; and US Patent Publication US2005-0228030. For example, suitable formulations, methods of manufacture, pharmacokinetic characteristics of sustained release aminopyridine compositions and methods of treating various neurological disorders are further described in U.S. Patent No. 8,007,826 entitled "Sustained Release Aminopyridine Composition" issued on August 30, 2011; and U.S. Patent Publication No. 2005-0228030 entitled "Methods of Using Sustained Release Aminopyridine Compositions" published on October 13, 2005; the contents of each of which are incorporated herein by reference in their entireties.

Dalfampridine is the United States Adopted Name (USAN) for the chemical 4-aminopyridine (4-AP). It is FDA-approved as an extended release (ER), 10 mg tablet (see Ampyra® package insert) indicated to improve walking in subjects with multiple sclerosis (MS), as demonstrated by an increase in walking speed. The approved therapeutic dose of Dalfampridine is a 10 mg extended release tablet to be taken twice daily, approximately 12 hours apart, with or without food.

The effectiveness of dalfampridine in improving walking in patients with multiple sclerosis was evaluated in two double-blind, placebo-controlled phase 3 trials involving a total of 540 patients (Goodman et al., 2009, Lancet 373: 732-738; Goodman et al., 2010, Ann Neurol 68:494-502. The primary measure of efficacy in both trials was walking speed (in feet per second) as measured by the Timed 25-foot Walk (T25FW), using a responder analysis. A Responder was defined as a patient who showed faster walking speed for at least three visits out of a possible four during the double-blind period than the maximum value achieved in the five non-treatment visits. A statistically significantly greater proportion of patients taking dalfampridine-ER 10 mg twice daily were Responders, compared to patients taking placebo. During the double-blind treatment period, a statistically significantly greater proportion of patients taking dalfampridine had increases in walking speed of at least 10%, 20%, or 30% from baseline, compared to placebo. In both trials, consistent improvements in walking speed were shown to be associated with improvements on a patient self-assessment of ambulatory disability, the 12-item Multiple Sclerosis Walking Scale (MSWS-12).

Leg strength, assessed using the Lower Extremity Manual Muscle Test (LEMMT) was also studied in patients with multiple sclerosis. Leg strength was found to be statistically significantly improved with dalfampridine relative to placebo (p<0.05) (Goodman et al., 2008, Neurology 71: 1134-1141; Goodman et al., 2009, Lancet 373: 732-738; Goodman et al., 2010, Ann Neurol 373: 494-502).

There is a long-standing unmet need in the art to effectively treat impairments in balance and/or gait in patients with multiple sclerosis.

Citation of a reference herein shall not be construed as an admission that such is prior art to the present invention.

### 3. BRIEF SUMMARY OF THE INVENTION

Provided herein are methods for treatment of a balance impairment or a gait impairment in a patient with multiple sclerosis by administering an aminopyridine or a pharmaceutically acceptable salt thereof to the patient. In particular, disclosed herein is treatment that causes improvement in gait or balance in a patient with multiple sclerosis by administering an aminopyridine or a pharmaceutically acceptable salt thereof to the patient.

In specific embodiments, the methods described herein comprise administering a therapeutically effective amount of an aminopyridine or a pharmaceutically acceptable salt thereof to a patient with multiple sclerosis. In some embodiments, the described methods include a step of assessing balance or gait in a patient before administration of an aminopyridine or a pharmaceutically acceptable salt thereof, and/or a step of assessing balance or gait after administration of the aminopyridine or a pharmaceutically acceptable salt thereof. In specific embodiments, a composition administered to a patient does not comprise a pharmaceutically acceptable salt of an aminopyridine. In one embodiment, the aminopyridine is 4-aminopyridine. In another embodiment, the aminopyridine is 3-aminopyridine. In yet another embodiment, the aminopyridine is 3,4-diaminopyridine.

In certain embodiments, the patient treated in accordance with the methods described herein is a mammal. In a preferred embodiment, the patient treated in accordance with the methods described herein is a human.

In some embodiments, an aminopyridine or a pharmaceutically acceptable salt thereof is administered to a patient in a sustained release composition. In other embodiments, an aminopyridine or a pharmaceutically acceptable salt thereof is administered to a patient in an immediate release composition. In some embodiments, an aminopyridine or a pharmaceutically acceptable salt thereof is administered to the patient once daily. In other embodiments, an aminopyridine or a pharmaceutically acceptable salt thereof is administered to the patient twice daily. In specific embodiments of all the dosage regimens described herein, an aminopyridine or a pharmaceutically acceptable salt thereof is administered to the patient orally (e.g., as a tablet, a pill or a capsule). In one embodiment, an aminopyridine or a pharmaceutically acceptable salt thereof is formulated in a form of a tablet for administration to a patient. In one embodiment, an aminopyridine or a pharmaceutically acceptable salt thereof is formulated in a form of a capsule for administration to a patient.

In specific embodiments, an aminopyridine itself, and not a pharmaceutically acceptable salt thereof, is used in any of the methods described herein.

In a specific embodiment, an aminopyridine or a pharmaceutically acceptable salt thereof is administered to the patient orally, in a sustained release composition b.i.d. (i.e., twice daily). In certain embodiments, twice daily administration comprises administration of an aminopyridine or a pharmaceutically acceptable salt thereof every 12 hours. In a specific embodiment, an aminopyridine or a pharmaceutically acceptable salt thereof in a sustained release composition provides a Tₘₐₓ of about 2 hours to about 6 hours in a human. In another specific embodiment, an aminopyridine or a pharmaceutically acceptable salt thereof is administered to the patient orally, in a sustained release composition once daily.

In particular embodiments, an aminopyridine or a pharmaceutically acceptable salt thereof is administered to a patient in an amount in the range of 5 to 20 mg, 5 to 15 mg, 5 to 10 mg, or 7.5 to 10 mg once or twice daily, preferably in a sustained release composition. For example, 5, 6, 7, 7.5, 8, 9, 10, 11, 12, 12.5, 13, 14, 15, 16, 17, 17.5, 18, 19, 20, 25, 30, 35, or 40 mg of an aminopyridine or a pharmaceutically acceptable salt thereof can be administered to a patient once daily, preferably in a sustained release composition, or 5, 6, 7, 7.5, 8, 9, 10, 11, 12, 12.5, 13, 14, 15, 16, 17, 17.5, 18, 19, or 20 mg of an aminopyridine or a pharmaceutically acceptable salt thereof can be administered to a patient twice daily, preferably in a sustained release composition. In one embodiment, an aminopyridine or a pharmaceutically acceptable salt thereof is administered in the amount of 10 mg once daily, preferably in a sustained release composition. In another embodiment, an aminopyridine or a pharmaceutically acceptable salt thereof is administered in the amount of 10 mg twice daily, preferably in a sustained release composition. In one embodiment, an aminopyridine or a pharmaceutically acceptable salt thereof is administered in the amount of 15 mg once daily, preferably in a sustained release composition. In another embodiment, an aminopyridine or a pharmaceutically acceptable salt thereof is administered in the amount of 7.5 mg twice daily, preferably in a sustained release composition.

In specific embodiments, a therapeutically effective amount of an aminopyridine or a pharmaceutically acceptable salt thereof is in the range of 5 to 20 mg, 5 to 15 mg, 5 to 10 mg, or 7.5 to 10 mg once or twice daily, preferably in a sustained release composition. For example, a therapeutically effective amount of an aminopyridine or a pharmaceutically acceptable salt can be 5, 6, 7, 7.5, 8, 9, 10, 11, 12, 12.5, 13, 14, 15, 16, 17, 17.5, 18, 19, 20, 25, 30, 35, or 40 mg once daily, preferably in a sustained release composition, or 5, 6, 7, 7.5, 8, 9, 10, 11, 12, 12.5, 13, 14, 15, 16, 17, 17.5, 18, 19, or 20 mg twice daily, preferably in a sustained release composition.

In certain embodiments, treatment in accordance with the invention continues for more than 1, 2, 3, 4, 5, 6, 7, 8, 9, 10, 11, or 12 weeks; 1, 2, 3, 4, 5, 6, 7, 8, 9, 10, 11, or 12 months; or 1, 2, 3, 4, 5, 6, 7, 8, 9, or 10 years since the commencement of treatment.

In specific embodiments, the methods provided herein are for treating a balance impairment or dysfunction, or an abnormal gait in a patient with multiple sclerosis. In one embodiment, provided herein are methods for treating a balance impairment during motion, i.e., impairment observed when the patient is in motion (e.g., walking, jogging, or running). In another embodiment, provided herein are methods for treating a balance impairment observed when the patient is not in motion, such as a balance impairment in a stationary position (i.e., stationary balance). Stationary balance can be, e.g., balance when sitting still, standing still, or in another fixed position, or when rotating about a fixed axis. As used herein, stationary balance means balance in a stationary position that is not locomotion from one point to another, but stationary balance can be balance during rotational movement. In particular embodiments, the methods provided herein are for treating an impairment in stationary balance in a patient when the patient is sitting, standing, reaching, maintaining single-leg stance or turning. In some embodiments, the methods provided herein are for treating a postural imbalance (e.g., a postural imbalance in a stationary position) in a patient with multiple sclerosis. In some embodiments, the methods provided herein are for treating an impairment in postural balance in a patient when the patient is sitting, standing, reaching (e.g., reaching while in a stationary position), maintaining single-leg stance or turning (e.g., turning while in a stationary position).

In specific embodiments, the methods provided herein are for treating an impairment in stationary balance in a patient with multiple sclerosis, said method comprising administering to the patient an aminopyridine (e.g., 4-aminopyridine) in a sustained release composition. In a specific embodiment, the methods provided herein are for treating an impairment in stationary balance in a patient with multiple sclerosis, said method comprising administering to the patient 10 mg of 4-aminopyridine in a sustained release composition twice daily. In a specific embodiment, the balance impairment is an impairment that can be assayed by the Berg Balance Scale.

In specific embodiments, the patient treated in accordance with the methods provided herein has relapsing remitting, secondary progressive, primary progressive, or progressive relapsing type of multiple sclerosis. In a specific embodiment, the patient has relapsing remitting type of multiple sclerosis.

In certain embodiments, provided herein are methods for treating an impairment in one, two, three or more than three parameters of gait and/or balance in a patient with multiple sclerosis. In one embodiment, provided herein are methods for treating an impairment in gait and/or balance in a patient with multiple sclerosis, wherein the impairment is diagnosed or efficacy of treatment is assessed using one, two, three or more tests described herein or known in the art. In some of these embodiments, the impairment in gait is determined by measuring at least one parameter of gait that is not walking speed.

In particular embodiments, provided herein are methods for treating an impairment in one, two, three or more than three parameters of gait, wherein the parameters of gait are selected from the group consisting of step length, step width, step speed and turn sway, and at least one of the parameters of gait is not step speed. In one embodiment, provided herein are methods for treating an impairment in step length (such as a decreased step length), an impairment in step width (such as an increased step width), an impairment in step speed (such as a decreased step speed), and/or an impairment in turn sway (such as an increased turn sway). In other words, in one specific embodiment, provided herein are methods for increasing step length in a patient with multiple sclerosis. In another specific embodiment, provided herein are methods for decreasing step width in a patient with multiple sclerosis. In yet another specific embodiment, provided herein are methods for decreasing turn sway in a patient with multiple sclerosis. In yet another specific embodiment, provided herein are methods for increasing step speed in a patient with multiple sclerosis. In some embodiments, provided herein are methods for treating an impairment in gait (such as decreased step length, increased step width, decreased step speed, or increased turn sway), wherein the impairment is diagnosed or efficacy of treatment is assessed using NeuroCom SMART Balance Master®. In a specific aspect of these embodiments, the impairment in gait is an impairment in at least one parameter of gait that is not step speed.

In specific embodiments, the treatment in accordance with the invention is effective to treat (e.g., improve, ameliorate, or reduce the severity of) the symptoms of gait impairment or balance impairment in a patient with multiple sclerosis. In some embodiments, the treatment in accordance with the invention improves gait or balance in a patient with multiple sclerosis. In specific embodiments, the treatment in accordance with the invention improves postural balance in a patient with multiple sclerosis. In other specific embodiments, the treatment in accordance with the invention improves step length, step width, step speed, and/or turn sway in a patient with multiple sclerosis. In one embodiment, the treatment in accordance with the invention improves at least one parameter of gait that is not step speed, e.g., step length, step width, or turn sway. In certain embodiments, further provided are methods for assessing the level of said balance or gait impairment after, or before and after, repeated administration of an aminopyridine or a pharmaceutically acceptable salt thereof. Such method can be any method for evaluating balance or gait described herein or known in the art.

In certain embodiments of the methods described herein, wherein a patient is administered an aminopyridine (e.g., 3-aminopyridine, 4-aminopyridine or 3,4-diaminopyridine), the aminopyridine is administered in an amount that elicits a Cₘᵢₙₛₛ of at least 10, 11, 12, 13, 14, 15, 16, 17, 18, 19 or 20 ng/ml. In some embodiments wherein a patient is administered an aminopyridine (e.g., 3-aminopyridine, 4-aminopyridine or 3,4-diaminopyridine), the aminopyridine is administered in an amount that elicits a Cₘₐₓₛₛ of less than 30, 35, 40, 45, 50, 55, 60, 65, 70 75 or 80 ng/ml. In other embodiments wherein a patient is administered an aminopyridine (e.g., 3-aminopyridine, 4-aminopyridine or 3,4-diaminopyridine), the aminopyridine is administered in an amount that elicits a Cₘᵢₙₛₛ selected from the group consisting of at least any of 10, 11, 12, 13, 14, 15, 16, 17, 18, 19 or 20 ng/ml, and which elicits a Cₘₐₓₛₛ selected from the group consisting of less than any of 30, 35, 40, 45, 50, 55, 60, 65, 70 75 or 80 ng/ml.

### 3.1 Terminology

In order to provide a clear and consistent understanding of the specification and claims, the following definitions are provided:

As used herein, if no fluid is mentioned or the context does not indicate otherwise, Cₘᵢₙₛₛ, Cₘₐₓₛₛ, Cₐᵥₛₛ values generally relate to blood plasma.

The term "gait," as used herein, refers to the pattern of movement of the limbs during locomotion over a solid substrate. In one embodiment, gait is the manner and style of walking.

The term "improvement" with respect to an impairment designates an alteration in a parameter in a therapeutic direction. As used herein, "improvement" also comprises stabilization of a parameter that would otherwise be deteriorating or moving in a non-therapeutic direction.

By "pharmaceutically acceptable", with respect to a carrier, diluent or excipient, is meant the carrier, diluent or excipient must be compatible with the other ingredients of the formulation and not prohibited for human or veterinary administration (as the case may be) by a regulatory agency such as the Food and Drug Administration or European Medicines Agency.

The term "pharmaceutically acceptable salt(s)," with reference to an aminopyridine, as used herein, refers to a salt prepared from a pharmaceutically acceptable non-toxic acid or base, including an inorganic acid or base, or an organic acid or base. In one embodiment, the pharmaceutically acceptable salt is prepared from a pharmaceutically acceptable non-toxic acid which can be an inorganic or organic acid. In one embodiment, non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. In one embodiment, the non-toxic acid is hydrochloric acid. Suitable pharmaceutically acceptable salts will be apparent to those skilled in the art and include those described in S.M. Barge et al., "Pharmaceutical Salts," 1977, J. Pharm. Sci. 66:1-19, which is incorporated herein by reference in its entirety.

Other terms and/or abbreviations are provided below:

| **Abbreviation or Specialist Term** | **Explanation** |
|---|---|
| b.i.d. (bid) | Twice daily |
| Cm | Centimeter |
| Cₘₐₓ | Maximum measured plasma concentration |
| Cₘₐₓₛₛ | Maximum measured plasma concentration at steady state |
| Cₘᵢₙ | Minimum measured plasma concentration |
| Cₘᵢₙₛₛ | Minimum measured plasma concentration at steady state |
| CNS | Central nervous system |
| Dalfampridine | Fampridine, 4-aminopyridine |
| DAP | di-aminopyridine |
| Dalfampridine-ER, D-ER, DER | Dalfampridine extended-release (i.e., sustained release formulation of dalfampridine) |
| Fampridine | Dalfampridine, 4-aminopyridine |
| g, kg, mg, µg, ng | Gram, kilogram, milligram, microgram, nanogram |
| GLP | Good Laboratory Practice |
| h, hr | Hour |
| HPLC | High performance liquid chromatography |
| IR | Immediate-release |
| IV, i.v., or iv | Intravenous |
| K⁺ | Ionic Potassium |
| L, mL | Liter, milliliter |
| LEMMT | Lower Extremity Manual Muscle Test |
| LCMS, LC/MS/MS | Liquid chromatography/ mass spectrometry |
| MCAO | Middle Cerebral Artery Occlusion |
| Min | Minute |
| mM, µM | Millimolar, micromolar |
| MS | Multiple sclerosis |
| MSWS-12 | 12-item Multiple Sclerosis Walking Scale |
| NF | National Formulary |
| p.o. | Oral |
| q.d. (qd) | Once a day |
| SR | Sustained-release |
| SS | Steady state |
| T25FW | Timed 25 Foot Walk |
| t.i.d. (tid) | Three times daily |
| Tₘₐₓ | Time of the maximum measured plasma concentration post-dose |
| USP | United States Pharmacopeia |
| WS | Walking speed |
| 3AP, or 3-AP | 3-aminopyridine |
| 4AP, or 4-AP | 4-aminopyridine |
| 3,4 DAP, or 3,4-DAP | 3,4, di-aminopyridine |

### 4. BRIEF DESCRIPTION OF DRAWINGS

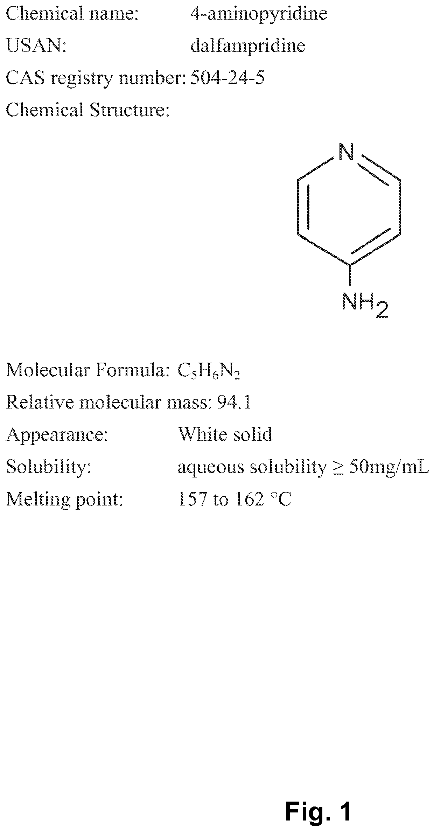

**Figure 1** shows information regarding 4-aminopyridine.

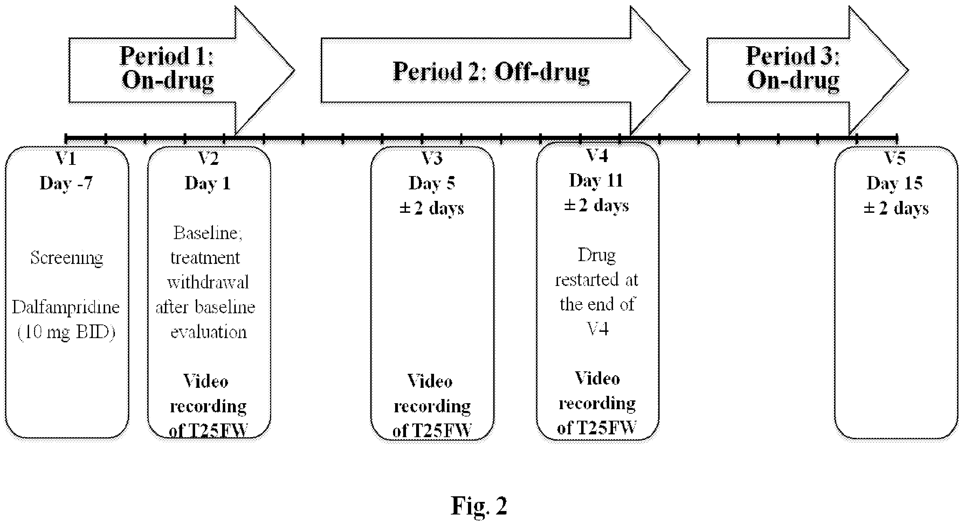

**Figure 2** shows the Treatment Schedule of an open-label, single-center, 3-study period study in T25FW Improvers, which is described in detail in the Examples. T25FW Improver is defined as a patient who showed an improvement on the T25FW between an off-drug and on-drug evaluation prior to entry in the study. Video recording was optional.

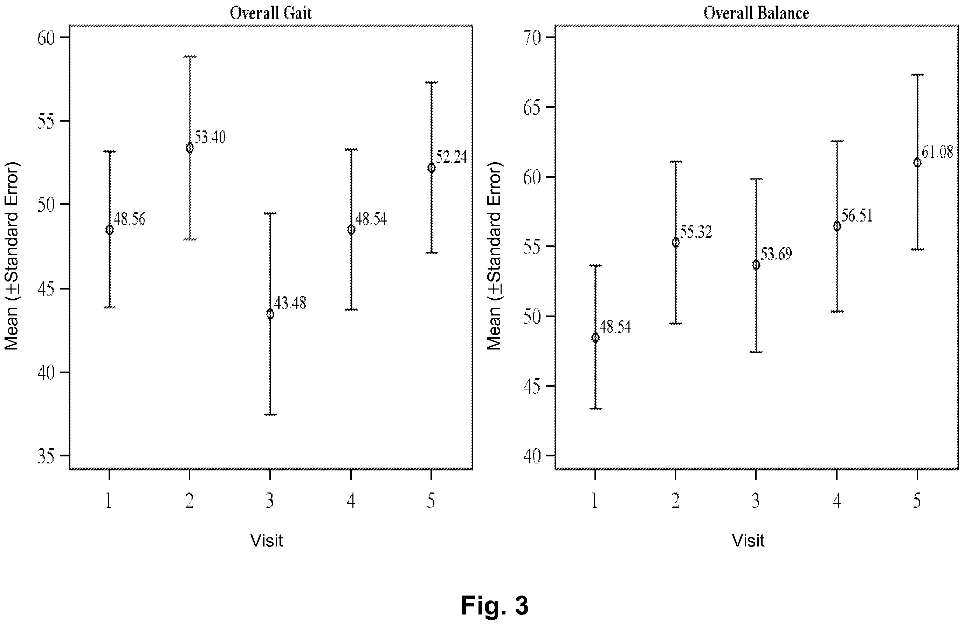

**Figure 3****:** NeuroCom-Overall Gait and Overall Balance by Visit (Full Analysis Population). This figure shows overall gait and overall balance scores during Visits 1, 2, 3, 4 and 5, as measured using NeuroCom SMART Balance Master®) (for more detailed description see Example 3). Visits 1, 2, and 5 were on-drug (dalfampridine-ER) visits; Visits 3 and 4 were off-drug (withdrawal) visits. For both composite scores, a higher score is indicative of better performance.

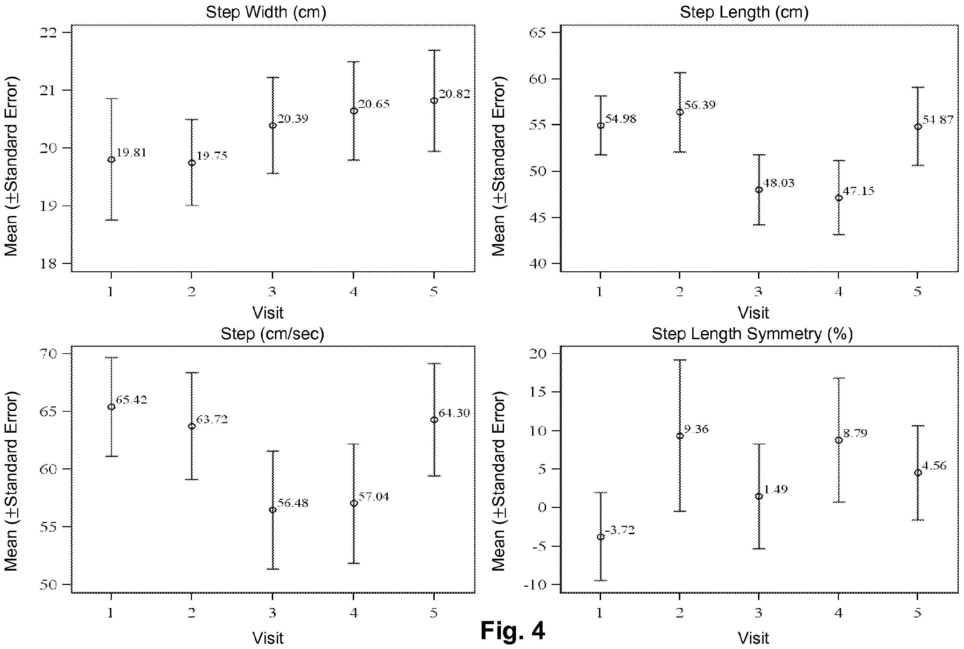

**Figure 4****:** NeuroCom-Walk Across Test Results by Visit (Full Analysis Population). This figure shows scores for the Walk Across Test variables during Visits 1, 2, 3, 4 and 5, as measured using NeuroCom SMART Balance Master® (for more detailed description see Example 3). Scores for the following variables were measured: step width (cm), step length (cm), speed (cm/sec), and step length symmetry (%). Visits 1, 2, and 5 were on-drug (dalfampridine-ER) visits; Visits 3 and 4 were off-drug (withdrawal) visits. For step width, a lower score is indicative of better performance. For all other variables, a higher score is better.

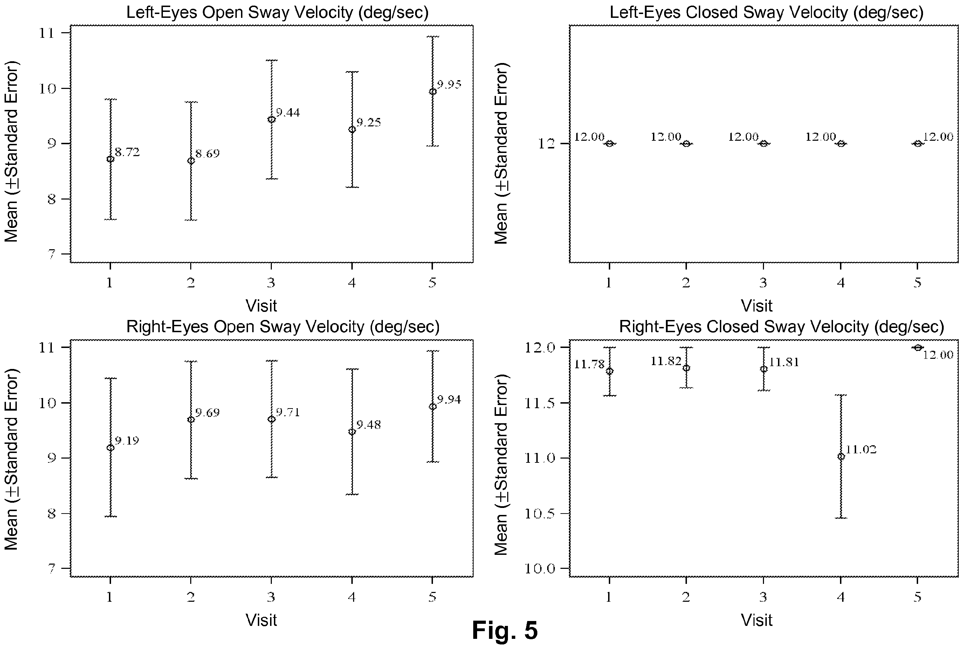

**Figure 5****:** NeuroCom-Unilateral Stance Variables by Visit (Full Analysis Population). This figure shows scores for the Unilateral Stance Test variables during Visits 1, 2, 3, 4 and 5, as measured using NeuroCom SMART Balance Master® (for more detailed description see Example 3). Scores for the following variables were measured: left-eyes open sway velocity (deg/sec), left-eyes closed sway velocity (deg/sec), right-eyes open sway velocity (deg/sec), and right-eyes closed sway velocity (deg/sec). Visits 1, 2, and 5 were on-drug (dalfampridine-ER) visits, while Visits 3 and 4 were off-drug (withdrawal) visits. For each variable, a lower score is more indicative of better performance.

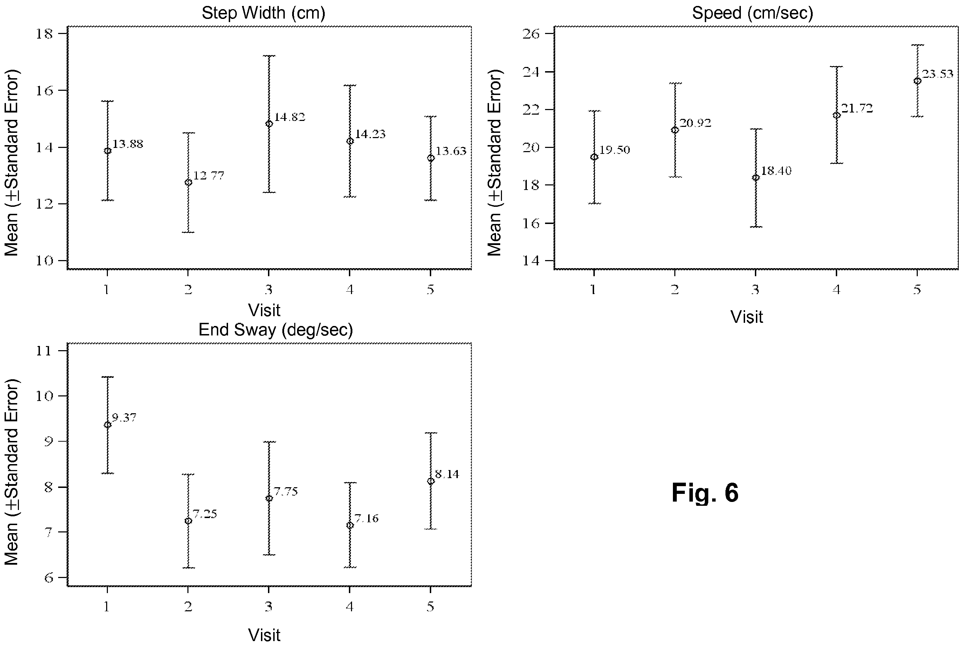

**Figure 6****:** NeuroCom-Tandem Walk Variables by Visit (Full Analysis Population). This figure shows scores for the Tandem Walk Test variables during Visits 1, 2, 3, 4 and 5, as measured using NeuroCom SMART Balance Master® (for more detailed description see Example 3). Scores for the following variables were measured: step width (cm), speed (cm/sec), and end sway (deg/sec). Visits 1, 2, and 5 were on-drug (dalfampridine-ER) visits, while Visits 3 and 4 were off-drug (withdrawal) visits. For step width and end sway, a lower score is indicative of better performance. For speed, a higher score is better.

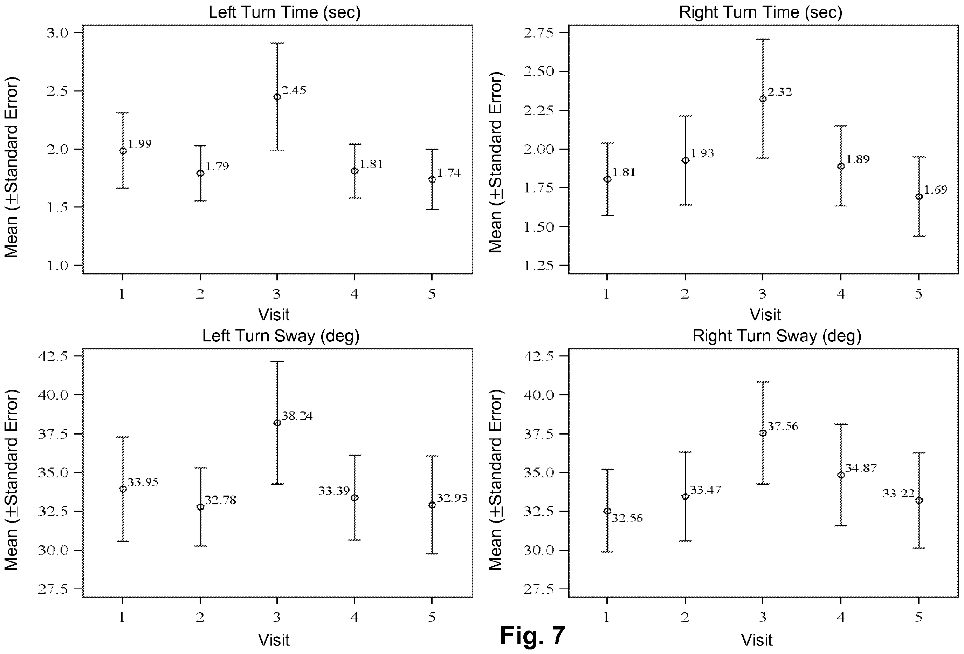

**Figure 7****:** NeuroCom-Step/Quick Turn Variables by Visit (Full Analysis Population). This figure shows scores for the Step/Quick Turn Test variables during Visits 1, 2, 3, 4 and 5, as measured using NeuroCom SMART Balance Master® (for more detailed description see Example 3). Scores for the following variables were measured: left turn time (sec), right turn time (sec), left turn sway (deg), and right turn sway (deg). Visits 1, 2, and 5 were on-drug (dalfampridine-ER) visits, while Visits 3 and 4 were off-drug (withdrawal) visits. For each variable, a lower score is indicative of better performance.

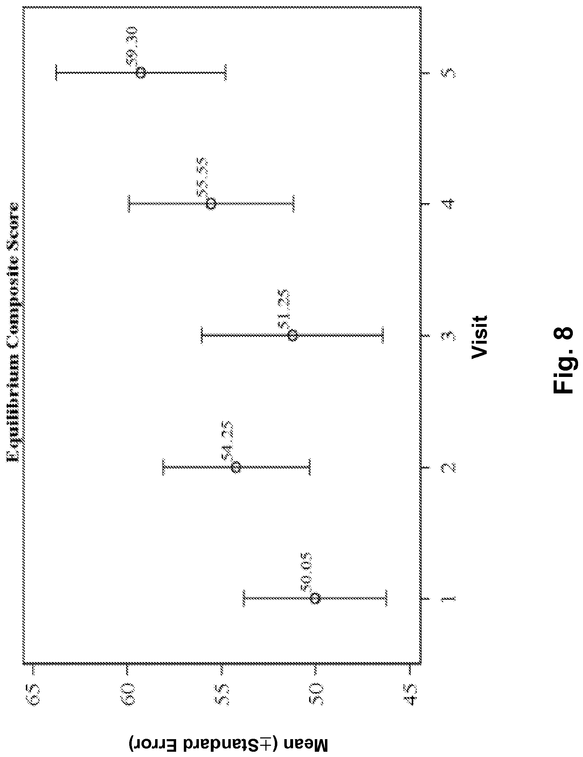

**Figure 8****:** NeuroCom-Sensory Organization Test Variable by Visit (Full Analysis Population). This figure shows Sensory Organization Test equilibrium composite score during Visits 1, 2, 3, 4 and 5, as measured using NeuroCom SMART Balance Master® (for more detailed description see Example 3). Visits 1, 2, and 5 were on-drug (dalfampridine-ER) visits, while Visits 3 and 4 were off-drug (withdrawal) visits. A higher equilibrium composite score is indicative of better performance.

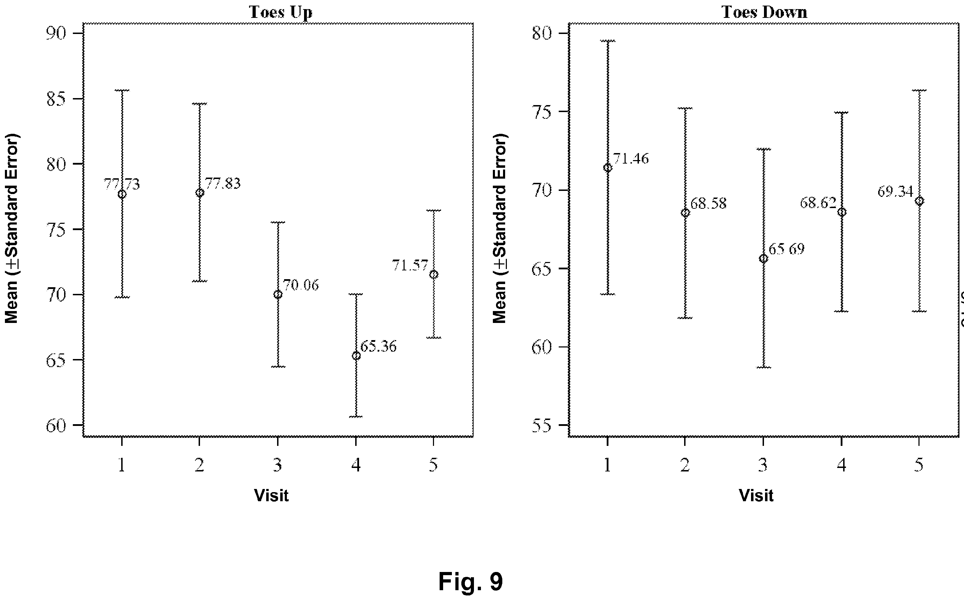

**Figure 9****:** NeuroCom-Adaptation Test Variables by Visit (Full Analysis Population). This figure shows scores for the Adaptation Test variables during Visits 1, 2, 3, 4 and 5, as measured using NeuroCom SMART Balance Master® (for more detailed description see Example 3). Scores for the following variables were measured: toes up score and toes down score. Visits 1, 2, and 5 were on-drug (dalfampridine-ER) visits, while Visits 3 and 4 were off-drug (withdrawal) visits. For each variable, a lower score is indicative of better performance.

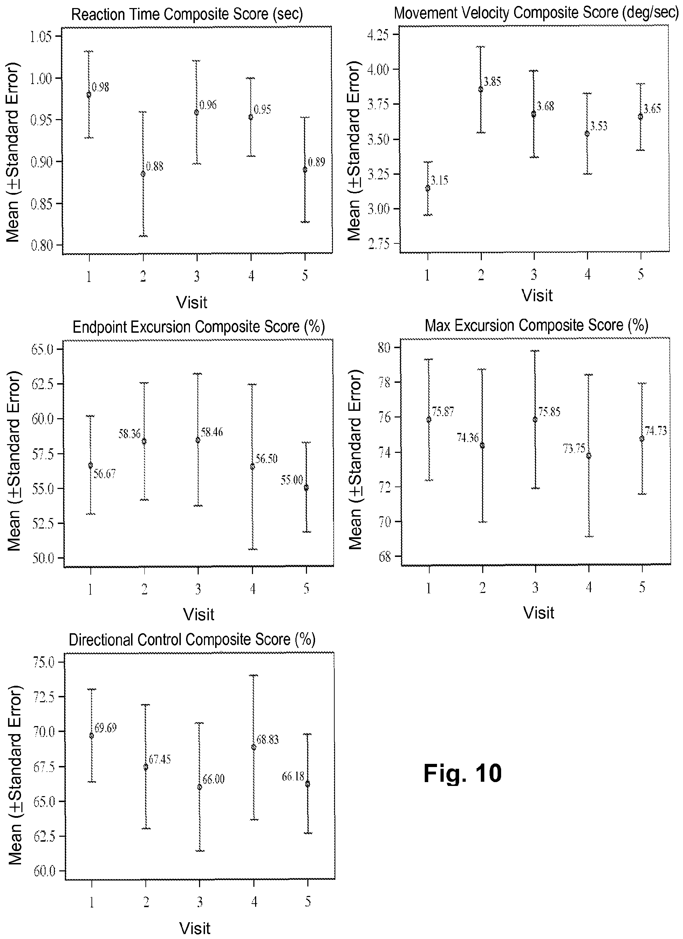

**Figure 10****:** NeuroCom-Limits of Stability Variables by Visit (Full Analysis Population). This figure shows scores for the Limits of Stability Test variables during Visits 1, 2, 3, 4 and 5, as measured using NeuroCom SMART Balance Master® (for more detailed description see Example 3). Scores for the following variables were measured: reaction time composite score (sec), movement velocity composite score (deg/sec), endpoint excursion composite score (%), max excursion composite score (%), and directional control composite score (%). Visits 1, 2, and 5 were on-drug (dalfampridine-ER) visits, while Visits 3 and 4 were off-drug (withdrawal) visits. For reaction time, a lower score is indicative of better performance. For all other variables, a higher score is better.

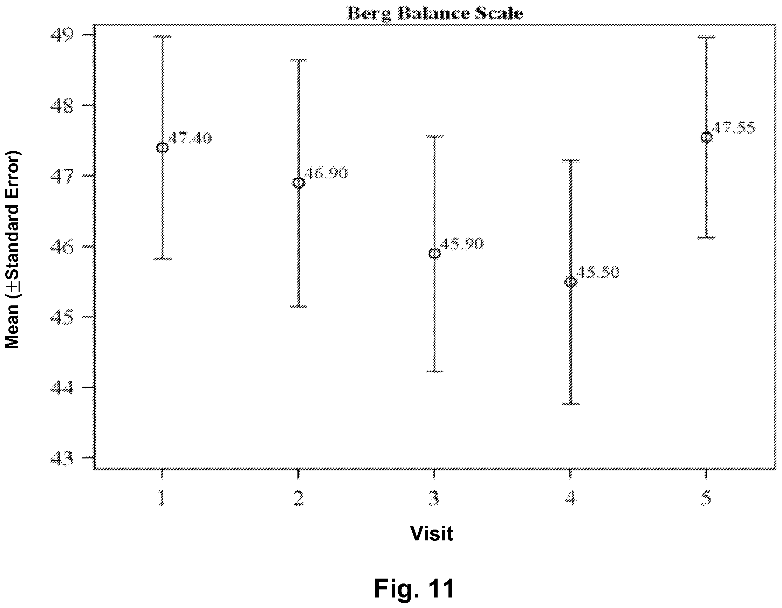

**Figure 11****:** Berg Balance Scale-Total Score by Visit (Full Analysis Population). This figure shows total balance scores during Visits 1, 2, 3, 4 and 5, as measured using Berg Balance Scale (for more detailed description see Example 3). Visits 1, 2, and 5 were on-drug (dalfampridine-ER) visits, while Visits 3 and 4 were off-drug (withdrawal) visits. A higher total score is indicative of better performance.

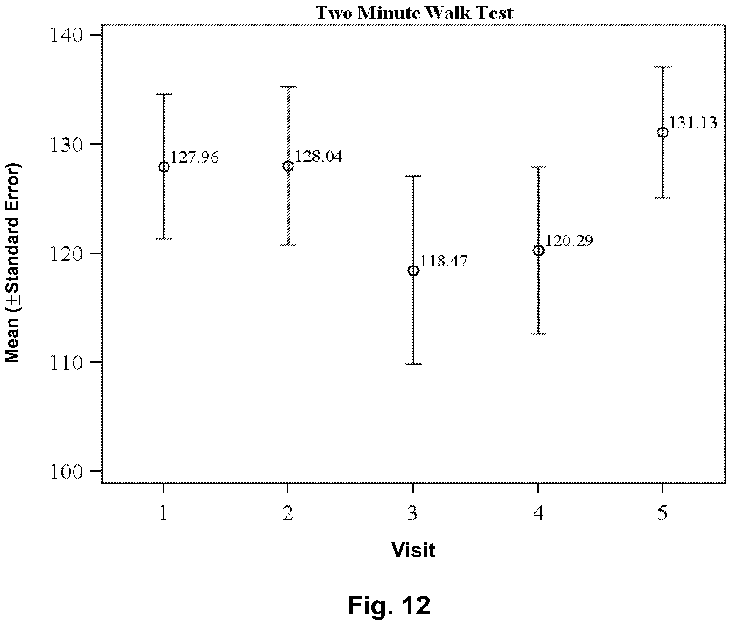

**Figure 12****:** Two Minute Walk Test-Walking Distance (m) by Visit (Full Analysis Population). This figure shows walking distance (m) during Visits 1, 2, 3, 4 and 5, as measured using Two Minute Walk Test (for more detailed description see Example 3). Visits 1, 2, and 5 were on-drug (dalfampridine-ER) visits, while Visits 3 and 4 were off-drug (withdrawal) visits. A larger walking distance is indicative of better performance.

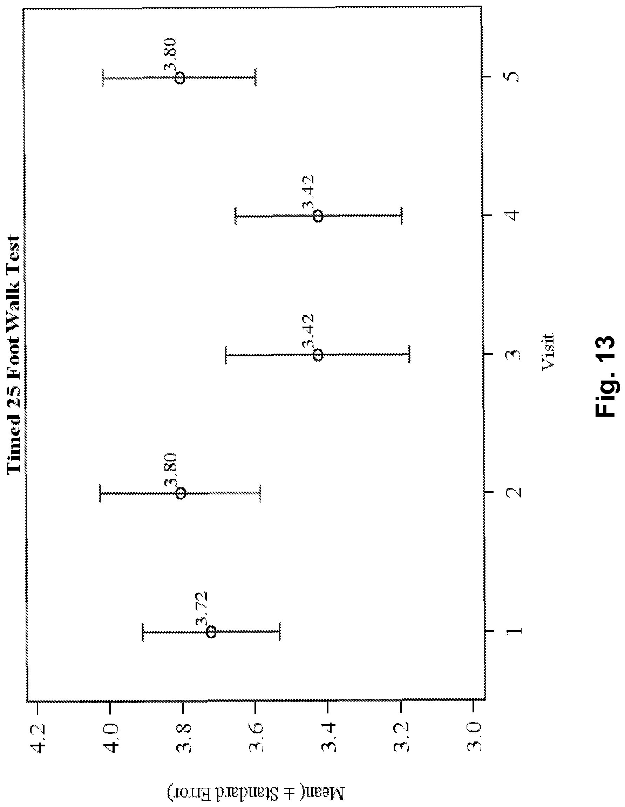

**Figure 13****:** Timed 25 Foot Walk-Walking Speed Distance (ft/s) by Visit (Full Analysis Population). This figure shows walking speed (ft/s) during Visits 1, 2, 3, 4 and 5, as measured using Timed 25 Foot Walk Test (for more detailed description see Example 3). Visits 1, 2, and 5 were on-drug (dalfampridine-ER) visits, while Visits 3 and 4 were off-drug (withdrawal) visits. A higher walking speed is indicative of better performance.

### 5. DETAILED DESCRIPTION

### 5.1 Aminopyridines for Use in the Methods of the Invention

The invention provides use of an aminopyridine or a pharmaceutically acceptable salt thereof for treating certain specific impairments described herein, such as impairments in gait and/or balance, in a patient with multiple sclerosis. In particular embodiments, disclosed herein are uses of an aminopyridine or a pharmaceutically acceptable salt thereof for treating an impairment in gait and/or balance in a patient with multiple sclerosis. The invention provides methods for treating an impairment in gait and/or balance in a patient with multiple sclerosis in need thereof, wherein the methods comprise administering to the patient an aminopyridine or a pharmaceutically acceptable salt thereof.

The structure of an aminopyridine is well known in the art. As shown in U.S. Patent No. 5,952,357, a mono- or diaminopyridine has the following structure: 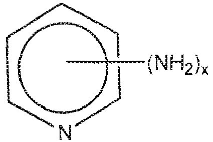 , wherein x is 1 or 2.

Aminopyridines having the above structural formula wherein x is 1 are, e.g., 2-aminopyridine, 3- aminopyridine and 4- aminopyridine. Aminopyridine compounds having the above structural formula wherein x is 2 are, e.g., 2,3-diaminopyridine; 2,5-diaminopyridine; 2,6-diaminopyridine; 3,4-diaminopyridine; 4,5-diaminopyridine and 4,6-diaminopyridine. Aminopyridine compounds having the above structural formula wherein x is 2 are, e.g., 2,3-diaminopyridine; 2,5-diaminopyridine; 2,6-diaminopyridine; 3,4-diaminopyridine; 3,5-diaminopyridine; and 2,4-diaminopyridine.

In one embodiment, the aminopyridine is a mono- or di-aminopyridine. In one embodiment, the mono- aminopyridine is 3-aminopyridine or 4-aminopyridine. In one embodiment the di- aminopyridine is 3,4-diaminopyridine.

As will be appreciated, a pharmaceutically acceptable salt of an aminopyridine may be used instead of or in addition to an aminopyridine in any or all of the methods of treating discussed herein. Thus, in specific embodiments, a pharmaceutically acceptable salt of an aminopyridine (i.e., any pharmaceutically acceptable salt of any of the aminopyridine compounds listed above) is used in the methods of treating an impairment in gait and/or balance in a patient with multiple sclerosis. These salts can be prepared, for example, in situ during the final isolation and purification of the compounds or by separately reacting the purified compound in its free base form with a suitable organic or inorganic acid and isolating the salt thus formed. In some embodiments, a salt of a mono- or di-aminopyridine is used in the methods of the invention. In another embodiment, a salt of 3-aminopyridine or 4-aminopyridine is used. In yet another embodiment, a salt of 3,4-diaminopyridine is used. In some embodiments, the pharmaceutically acceptable salt of an aminopyridine is prepared using acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, or p-toluenesulfonic acid. In one embodiment, one equivalent of an aminopyridine, as used herein, may form an acid salt with less than one or with one or more than one equivalent of an acid. In one embodiment an aminopyridine, as used herein, may form a dihydrochloride salt. In one embodiment an aminopyridine, as used herein, may form a phosphate salt. For further description of pharmaceutically acceptable salts that can be used in the methods described herein see, for example, S.M. Barge et al., "Pharmaceutical Salts," 1977, J. Pharm. Sci. 66:1-19, which is incorporated herein by reference in its entirety.

In preferred embodiments, an aminopyridine itself, and not a pharmaceutically acceptable salt thereof, is used in any of the methods described herein.

### 5.2 Impairments Treated in Accordance with the Invention

In particular, provided herein are methods for treatment of an impairment in gait and/or balance in a patient with multiple sclerosis comprising administering an aminopyridine or a pharmaceutically acceptable salt thereof. In a specific embodiment, the methods are effective to improve the impairment in gait and/or balance in a patient with multiple sclerosis. In certain embodiments, disclosed herein is treatment that causes improvement in one or more parameters of gait and/or balance in a patient with multiple sclerosis.

In certain embodiments, the impairment treated in accordance with the methods described herein is balance impairment or balance dysfunction in a patient with multiple sclerosis. In some embodiments, the methods provided herein are for treating imbalance in a patient with multiple sclerosis. In one embodiment, the methods provided herein are for treating a balance impairment observed when the patient is in motion, such as during locomotion over a solid substrate (e.g., walking, jogging, or running), in other words, an impairment in balance during motion (i.e., dynamic balance). Accordingly, in one embodiment, the methods provided herein are for treating an impairment in dynamic balance in a patient with multiple sclerosis. In another embodiment, the methods provided herein are for treating a balance impairment observed when the patient is not in motion, for example not in locomotion over a solid substrate, such as sitting, standing, or in another stationary or spatially fixed position (i.e., static balance). Accordingly, in one embodiment, the methods provided herein are for treating an impairment in static balance in a patient with multiple sclerosis. In one embodiment, the methods provided herein are for treating a postural imbalance (i.e., an impairment in postural balance) in a patient with multiple sclerosis. In one embodiment, an impairment in postural balance treated in accordance with the methods described herein can be an impairment in postural balance while not in locomotion over a solid substrate. In a specific embodiment, the impairment in postural balance is assayed or assayable by the Berg Balance Scale. In particular embodiments, the methods provided herein are for treating a postural balance impairment in an MS patient when the patient is sitting, standing, reaching, maintaining single-leg stance, and/or turning. In some embodiments, the methods provided herein are for treating an impairment in stationary balance in an MS patient when the patient is sitting, standing, reaching, maintaining single-leg stance, and/or turning. In one embodiment, the methods provided herein are for treating an impairment in balance in a patient when the patient is sitting. In one embodiment, the methods provided herein are for treating an impairment in balance in a patient when the patient is standing. In one embodiment, the methods provided herein are for treating an impairment in balance in a patient when the patient is reaching (e.g., while in a stationary position). In one embodiment, the methods provided herein are for treating an impairment in balance in a patient when the patient is maintaining single-leg stance. In one embodiment, the methods provided herein are for treating an impairment in turning or rotational balance in a patient (e.g., while in a stationary position with respect to locomotion over a solid substrate).

In certain embodiments, the impairment treated in accordance with the methods described herein is an abnormal gait in a patient with multiple sclerosis. In some embodiments, the impairment treated in accordance with the methods described herein is an abnormal manner and style of walking. In specific embodiments, the impairment treated in accordance with the methods described herein is an impairment in gait during walking, jogging, running, and/or turning, in a patient with multiple sclerosis. In particular embodiments, the methods provided herein are for treating a gait impairment observed when the patient is in motion, such as during locomotion over a solid substrate (such as during any ambulation, e.g., walking, jogging, or running). In one embodiment, the impairment treated in accordance with the methods described herein is an impairment in gait during walking in a patient with multiple sclerosis.

In certain embodiments, provided herein are methods for treating an impairment in one or more (e.g., one, two, three, four or more than four) parameters of gait and/or balance in a patient with multiple sclerosis. At least one or at least two of the one or more (e.g., one, two, three, four or more than four) parameters of gait and/or balance is not walking speed. In specific embodiments, parameters of balance include, without limitation, ability to sit, ability to stand, ability to reach, ability to maintain single-leg stance and ability to turn. Ability of a patient not to lose balance or fall, e.g., when exposed to surface irregularities or unexpected changes in support surface inclination, or when leaning their body in a given direction, can also be evaluated as a parameter of balance. In one aspect, mitigation (e.g., decrease) in the incidence of falls is evaluated as a parameter of balance. Balance can also be measured by a test quantifying postural sway velocity with the subject standing on one foot, with eyes open or eyes closed, in which less sway indicates greater stability. Parameters of gait include, without limitation, step width, step or stride length, step speed, step time, step length symmetry, step length/leg length ratio, velocity (e.g., endpoint sway velocity), base of support (e.g., dynamic base of support) and percentage of gait cycle spent in double or single support per walking trial. In some embodiments, parameters of gait include, without limitation, posture, length of stride, width of base, speed and/or fluidity of motion, arm swing, bilateral symmetry of motor activity, and neurological deficits or signs. In certain embodiments, parameters of gait include step width, step length, step speed, step length symmetry, sway velocity (e.g., left-eyes open sway velocity, left-eyes closed sway velocity, right-eyes open sway velocity, right-eyes closed sway velocity), end sway, turn time (e.g., left-turn time, right turn time), and turn sway (e.g., left-turn sway, right-turn sway). In specific embodiments, parameters of gait include postural sway velocity and endpoint sway velocity. In particular embodiments, parameters of gait include parameters that can be assessed using Walk Across Test Unilateral Stance Test, Tandem Walk Test, and/or Step/Quick Turn Test, for example, when such tests are performed using NeuroCom SMART Balance Master®.

In specific embodiments, provided herein are methods for treating an impairment in one, two, three or more than three parameters of gait (e.g., walking gait) in a patient with multiple sclerosis, wherein the parameters of gait are selected from the group consisting of step length, step width, step speed and turn sway, and at least one of the parameters of gait is not step speed. In one embodiment, provided herein are methods for increasing step length in a patient with multiple sclerosis. In another embodiment, provided herein are methods for decreasing step width in a patient with multiple sclerosis. In another embodiment, provided herein are methods for increasing step speed in a patient with multiple sclerosis. In yet another embodiment, provided herein are methods for decreasing turn sway (e.g., left turn sway and/or right turn sway) in a patient with multiple sclerosis. In preferred embodiments, the methods described herein are for treating at least one parameter of gait that is not step speed. In some embodiments, the treatment in accordance with the invention is effective to improve one, two, three or more than three parameters of gait (e.g., walking gait) in a patient with multiple sclerosis, wherein the parameters of gait are selected from the group consisting of step length, step width, step speed and turn sway, and at least one of the parameters of gait is not step speed. In particular embodiments, the treatment in accordance with the invention is effective to increase step length, decrease step width, increase step speed, and/or decrease turn sway in a patient with multiple sclerosis. In preferred embodiments, the treatment in accordance with the invention is effective to improve at least one parameter of gait that is not step speed.

One or more (e.g., one, two, three or more) tests described herein or known in the art can be used to assess gait and/or balance, and/or a parameter thereof, e.g., in order to diagnose an impairment in gait and/or balance and/or to monitor treatment efficacy (the latter when the test is administered after treatment). The one or more test(s) comprise at least one test that measures a parameter other than walking speed which is used for the assessment. In a specific embodiment, the one or more test(s) comprise at least one test that measures a parameter other than walking speed, lower extremity muscle strength, and lower extremity muscle tone. Thus, at least one measure other than walking speed (and, optionally, also other than lower extremity muscle strength and tone) is used for the assessment of gait and/or balance and/or a parameter thereof. In a particular embodiment, gait is assessed by one or more tests described herein or known in the art, wherein the results of at least one test that does not measure walking speed (and, optionally, other than lower extremity muscle strength and tone) are used for the assessment.

The gait or balance and parameters thereof in a patient, as well as an impairment or an improvement in gait or balance in a patient, can be assessed using any method known in the art, provided that at least one measure other than walking speed (and, optionally, other than lower extremity muscle strength and tone) is used for the assessment. For example, assessment tests can include, without limitation, tests that can be performed using NeuroCom SMART Balance Master®. NeuroCom SMART Balance Master® is a machine that provides objective assessments and retraining of the sensory and voluntary motor control of balance with visual feedback on either a stable or unstable support surface and in a stable or dynamic visual environment, and gait and balance parameters can be measured using such machine. Tests that can be performed using NeuroCom SMART Balance Master® include, without limitation, Walk Across test (evaluating characteristics of gait by measuring such parameters as step width, step length, speed and/or step length symmetry), Unilateral Stance test (evaluating postural sway velocity with a subject standing on one foot, with eyes open or closed, and specifically, measuring mean center of gravity sway velocity), Tandem Walk test (evaluating characteristics of gait as the subject walks heel to toe, and specifically, measuring such parameters as step width, speed and/or end sway), Step/Quick turn test (evaluating characteristic turn performance as a subject takes two steps forward and then quickly turns 180° and then returns to the starting point, and specifically measuring such parameters as turn time and/or turn sway), Sensory Organization Test (assessing a subject's ability to effectively process individual sensory system input cues to maintain balance control, and specifically, such test may include one or more of the following conditions: fixed surface eyes open, fixed surface eyes closed, walls moving eyes open, surface moving eyes open, surface moving eyes closed, and surface and walls moving eyes open), Adaptation Test (assessing a subject" s ability to minimize sway when exposed to surface irregularities or unexpected changes in support surface inclination, and specifically, measuring such parameters as the averaged, raw sway and/or center of force during rotational disturbances), and Limits of Stability Test (assessing the maximum distance a person can intentionally displace their center of gravity without losing balance, stepping or reaching for assistance, and specifically, measuring such parameters as reaction time, movement velocity, endpoint excursion, maximum excursion and/or directional control). The above-listed tests can be performed using NeuroCom SMART Balance Master® or a different assessment tool or protocol measuring one or more of the above-listed parameters of gait or balance. In addition, gait or balance in a patient (e.g., an impairment or improvement in gait or balance) can be assessed using Berg's Balance Scale, two minute walk test (2MWT), or timed 25 foot walk test (T25FW), provided that at least one measure other than walking speed (and, optionally, other than lower extremity muscle strength and tone) is used for the assessment. In one embodiment, Berg Balance Scale is used to assess an impairment or improvement in balance parameters in a patient. In some embodiments, Berg's Balance Scale test is used to measure a patient's ability to sit, stand, reach, maintain single-leg stance, or turn; or used to measure the integrity of the following functions in a patient: ability to change position from sitting to standing, ability to change position from standing to sitting, or ability to reach forward with an out-stretched arm. In some embodiments, gait or balance is assessed by observing or video recording gait and/or balance of a patient during 2MWT or T25FW. In some embodiments, 6 minute walk (6MW), Postural Stability Test, or Timed 10-meter Gait Test can be used to evaluate gait and/or balance in a patient, provided that at least one measure other than walking speed (and, optionally, other than lower extremity muscle strength and tone) is used for the assessment. In one embodiment, gait in a patient with MS is assessed using GAITRite™ technology (e.g., 26 foot GAITRite™), in which functional ambulatory profile (FAP) score, velocity, stride length, step time, base of support, and/or percentage of gait cycle spent in double or single support per walking trial can be evaluated (see Sosnoff et al., 2011, Gait&Posture 34:145-147). The FAP score obtained using GAITRite™ technology is based on the step length/leg length ratio, step time, normalized velocity and dynamic base of support (see Sosnoff et al., 2011, Gait&Posture 34:145-147). In yet another embodiment, gait can be evaluated during a physical exam by directly observing one or more parameters of gait, e.g., posture, length of stride, width of base, speed of motion, fluidity of motion, arm swing, bilateral symmetry of motor activity, or neurological deficits or signs. In addition, any test or method known in the art can be used to assess one or more parameters of gait or balance in a patient with multiple sclerosis, provided that at least one measure other than walking speed is used for the assessment. Such assessments can be performed before and after administration of an aminopyridine to a patient in accordance with the methods disclosed herein. For example, gait or balance in a patient with multiple sclerosis can be assessed before administering an aminopyridine and/or after administering an aminopyridine, e.g., at or after 1, 2, 3, 4, 5, 6, 7, 8, 9, 10 days; 1, 2, 3, 4, 5, 6, 7, 8 weeks; 1, 2, 3, 4, 5, 6, 7, 8, 9, 10 months; or 1, 2, 3, 4, 5 years since the commencement of treatment in accordance with the methods described herein.

In specific embodiments, the gait impairment or a parameter thereof that is treated or improved according to the methods of the invention is gait that is assessed as described herein. In specific embodiments, the balance impairment or a parameter thereof that is treated or improved according to the methods of the invention is balance that is assessed as described herein.

In specific embodiments, the treatment in accordance with the invention is to improve, ameliorate, or reduce the severity of the symptoms of gait impairment or balance impairment in a patient with multiple sclerosis. In certain embodiments, the treatment in accordance with the invention is to improve postural imbalance in a patient with multiple sclerosis. In some embodiments, the treatment in accordance with the invention is to improve gait and/or balance (e.g., to improve one, two or more parameters of gait and/or balance) in a patient with multiple sclerosis.

In certain embodiments, the level of balance or gait impairment can be assessed (e.g., by assessing one, two or more parameters of gait or balance) after, or before and after, repeated administration of an aminopyridine or a pharmaceutically acceptable salt thereof. Such method can be any method for evaluating balance or gait described herein or known in the art.

In specific embodiments, treating a MS patient by administering an amount of an aminopyridine or a pharmaceutically acceptable salt thereof is effective to treat (e.g., improve, ameliorate, alleviate the symptoms of, or reduce the severity of) an impairment in gait or balance in a patient with multiple sclerosis. In a specific embodiment, treating a MS patient by administering an amount of an aminopyridine or a pharmaceutically acceptable salt thereof is effective to eliminate an impairment in gait or balance in a patient with multiple sclerosis.

In another embodiment, a method for maintaining improvement of gait and/or balance in a patient with multiple sclerosis is provided, said method comprising: administering an amount (e.g., a therapeutically effective amount) of an aminopyridine (such as 3,4-diaminopyridine, 4-aminopyridine and the like) or a pharmaceutically acceptable salt thereof to said patient after previously achieving an improvement in gait and/or balance in said patient during administration of an aminopyridine.

In one embodiment, a method for treating an impairment in gait and/or balance or maintaining an improvement in gait and/or balance in a patient with multiple sclerosis comprises administering an amount (e.g., a therapeutically effective amount) of an aminopyridine or a pharmaceutically acceptable salt thereof to said patient over an extended period of time. In another embodiment, a method for achieving sustained improvement of gait and/or balance in a patient with multiple sclerosis comprises continuing administration of an amount (e.g., a therapeutically effective amount) of an aminopyridine (such as 3,4-diaminopyridine, 4-aminopyridine and the like) or a pharmaceutically acceptable salt thereof to said patient over an extended period of time. In a specific embodiment, the extended period of time is at least, or more than, 1, 2, 3, 4, 5, 6, 7, 8, 9, 10, 11, or 12 months.

In certain embodiments, an aminopyridine or a pharmaceutically acceptable salt thereof is administered to a patient for at least or more than: 1, 2, 3, 4, 5, 6, 7, 8, 9, 10, 11, 12, 13, 14, 15,16, 17, 18, 19, 20, 21 days; 1, 2, 3, 4, 5, 6, 7, 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 10 or greater than 5 or 10 years.

In specific embodiments, the improvement(s) in gait and/or balance among patients with multiple sclerosis occur over periods of at least or more than: 1, 2, 3, 4, 5, 6, 7, 8, 9, 10, 11, 12, 13, 14, 15, 16, 17, 18, 19, 20, 21 days; 1, 2, 3, 4, 5, 6, 7, 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 10, or greater than 5 or 10 years of treatment with an aminopyridine.

In a specific embodiment, an aminopyridine of the invention or a pharmaceutically acceptable salt thereof is administered at a therapeutically effective dosage sufficient to treat an impairment in gait and/or balance in a patient with multiple sclerosis. In certain embodiments, the treatment reduces the amount of symptoms of the impairment in the patient by at least about 10%, more preferably 20%, more preferably by at least about 40%, even more preferably by at least about 60%, and still more preferably by at least about 80% relative to untreated subjects. Such percent change quantification is preferably applied to assays of gait or balance that provide measurements of results in continuous linear scales, such as T25FW, etc. Other tests of gait and/or balance will not be expressed as percent change but would be predicted to result in a significant change with the appropriate statistical comparison. Such tests include semiquantitative measures that assign values to the ability to perform certain skills. In some embodiments, treatment in accordance with the invention results in a statistically significant improvement in a gait and/or balance impairment (e.g., as measured by the patient's ability to perform certain task or skill) in comparison to a control. Such control can be the patient's ability to perform the assessed task or skill prior to the commencement of treatment.

In a specific embodiment, a therapeutic outcome of treatment in accordance with the methods described herein is assayed for and detected at any one, two, three, four, five or more, or each, of the following time points, and/or at a time point later than any one of the following time points: 1, 2, 3, 4, 5, 6, 7, 8, 9, 10, 11, 12, 13, 14, 15, 16, 17, 18, 19, 20, 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, and 66 months; .5, 1, 1.5, 2, 2.5, 3, 3.5, 4, 4.5, 5, 5.5, 6 and 6.5 years post-commencement of treatment with an aminopyridine or a pharmaceutically acceptable salt thereof.

Example 2 set forth herein demonstrates that a sustained release formulation of 4-aminopyridine had positive and significant effects on gait. Observed trends for improvement in balance with time shown in Example 2 may reflect a previously reported NeuroCom SMART Balance Master® learning effect in addition to pharmacologic effects. Example 2 also shows that subjects performed significantly better in Timed 25 Foot Walk Test (T25FW), Two Minute Walk Test (2MWT), and Berg Balance Scale (BBS) while on drug than off drug.

Example 3 set forth herein represents a more detailed description of the experimental design of the study presented in Example 2 and the data obtained therein. Examples 2 and 3 demonstrate that a sustained release formulation of 4-aminopyridine had positive and significant effects on overall impairment in gait in patients with multiple sclerosis. Examples 2 and 3 demonstrate that a sustained release formulation of 4-aminopyridine had positive and significant effects on gait as measured by tests performed using NeuroCom SMART Balance Master1®. In addition, Example 3 shows that treatment of patients with multiple sclerosis with a sustained release formulation of 4-aminopyridine had significant positive effects on a number of specific gait parameters, such as step length, step width, step speed and turn sway, as measured by tests performed using NeuroCom SMART Balance Master®.

Examples 2 and 3 also demonstrate that a sustained release formulation of 4-aminopyridine had positive and significant effects on balance as measured by Berg Balance Scale (BBS). In particular, Examples 2 and 3 demonstrate that a sustained release formulation of 4-aminopyridine had positive and significant effects on postural balance in patients with multiple sclerosis as measured by BBS. Examples 2 and 3 further show that balance improved in patients upon re-initiation of treatment with a sustained release formulation of 4-aminopyridine after a period of withdrawal as measured by NeuroCom SMART Balance Master®, although no deterioration in overall balance during the period of withdrawal was observed. It is likely that both a learning effect and a drug effect contributed to the significant improvement in balance upon re-initiation of the sustained release 4-aminopyridine administration as measured by NeuroCom SMART Balance Master®.

Overall, the data presented in Examples 2 and 3 show that a sustained release formulation of 4-aminopyridine is effective to treat an impairment in gait (including impairments in such parameters of gait as step length, step width, step speed and turn sway) and an impairment in balance (including impairment in postural balance).

### 5.3 Patient identification or selection

In a preferred embodiment of the invention, a patient is selected, identified or diagnosed with multiple sclerosis and with an impairment in gait and/or balance.

In certain embodiments, a patient treated in accordance with the methods described herein has multiple sclerosis and an impairment in balance (i.e., imbalance). In a specific embodiment, the impairment in balance is manifested as an increased incidence of falls. The MS patient treated in accordance with the methods described herein can be diagnosed with balance or coordination impairment or dysfunction, a global body control impairment, an impairment in body sense, an abnormal gait, or a postural imbalance. In one embodiment, the MS patient treated in accordance with the methods described herein is diagnosed with a balance impairment observed when the patient is in motion (e.g., walking, jogging, or running). In another embodiment, the MS patient treated in accordance with the methods described herein is diagnosed with a balance impairment observed when the patient is in a stationary position and not in motion (e.g., sitting, standing, or in another stationary or spatially fixed position).

In certain embodiments, a patient treated in accordance with the methods described herein has multiple sclerosis and an impairment in gait (such as an impairment in gait during walking, jogging, running, and/or turning). In some embodiments, the MS patient treated in accordance with the methods described herein has (e.g., is diagnosed with) an impairment in one, two, three or more than three parameters of gait. In specific embodiments, the MS patient treated in accordance with the methods described herein has (e.g., is diagnosed with) an impairment in one, two or all of the following parameters of gait: step length (e.g., diagnosed with a decreased step length), step width (e.g., diagnosed with an increased step width), and turn sway (e.g., diagnosed with an increased turn sway). In particular embodiments, the MS patient treated in accordance with the methods described herein has (e.g., is diagnosed with) an impairment in at least one parameter of gait that is not walking speed or step speed.

The patients or subjects that are treated by the methods of the invention include, but are not limited to, humans and non-human vertebrates such as wild, domestic and farm animals. In certain embodiments, the patient treated in accordance with the invention is a mammal, e.g., a human, a cow, a dog, a cat, a goat, a horse, a sheep, or a pig. In a preferred embodiment, the patient to whom an aminopyridine or a pharmaceutically acceptable salt thereof is administered is a human. In one embodiment, the patient is 18 to 55 years old. In another embodiment, the patient is 18 to 70 years old. In certain embodiments, the patient is 40 to 70 years old. In some embodiments, the patient is 16 to 40 years old. In other embodiments, the patient is greater than 55 years old. In a specific embodiment, the patient is a female. In yet another specific embodiment, the patient is a male. In some embodiments, the patient has body mass index (BMI) of 16 to 35. In another embodiment, the patient has BMI of 18 to 30.

In certain embodiments, the patient treated in accordance with the methods described herein has (e.g., is diagnosed with) any type of multiple sclerosis, e.g., relapsing remitting, secondary progressive, primary progressive, or progressive relapsing. In one embodiment, the patient is diagnosed with relapsing remitting MS. In another embodiment, the patient is diagnosed with secondary progressive MS In yet another embodiment, the patient is diagnosed with primary progressive MS. In yet another embodiment, the patient is diagnosed with progressive relapsing MS. Patients with an atypical type of MS, such as Devic's disease, Balo concentric sclerosis, Schilder's diffuse sclerosis or Marburg multiple sclerosis, can also be treated in accordance with the methods described herein.

In some embodiments, the patients treated in accordance with the methods provided herein do not have a clinical history of seizures and/or epilepsy. In specific embodiments, the patients treated in accordance with the methods provided herein do not have a clinical history of seizures and/or epilepsy , with the exception of febrile seizures. For example, the patients have not experienced seizures and/or epilepsy in their lifetime, or have not experienced seizures and/or epilepsy 1, 2, 3, 4 or 5 years, or more than 5 years, prior to administration of an aminopyridine or a pharmaceutically acceptable salt thereof. In yet other embodiments, the patients treated in accordance with the methods provided herein have a clinical history of seizures and/or epilepsy.

### 5.4 Dosing Regimens

Any of the therapeutic methods described above can be carried out using any of the following dosing regimens. In specific embodiments, the aminopyridine used in such methods is 4-aminopyridine in a sustained release composition.

In some embodiments, an aminopyridine or a pharmaceutically acceptable salt thereof is administered in a sustained release composition. In other embodiments, an aminopyridine or a pharmaceutically acceptable salt thereof is administered in an immediate release composition. In certain embodiments, the method in accordance with the invention comprises administering an aminopyridine or a pharmaceutically acceptable salt thereof once daily, twice daily or thrice daily. In a specific embodiment, an aminopyridine (e.g., 4-AP), or a pharmaceutically acceptable salt thereof, is in a sustained release composition, and is administered once or twice daily, for example, orally. In a specific embodiment, the daily dose of 4-AP is once a day, or is given as two, three, or four equally divided subdoses. In another specific embodiment, an aminopyridine (e.g., 4-AP), or a pharmaceutically acceptable salt thereof, is in an immediate release composition, and is administered one, two, three times or more than three times daily, for example, orally. In one embodiment, a single dose of an aminopyridine or a pharmaceutically acceptable salt thereof (e.g., in an immediate release composition or in a sustained release composition) is administered to a patient.

In a specific embodiment, an aminopyridine or a pharmaceutically acceptable salt thereof is administered to the patient orally, in a sustained release composition b.i.d. (i.e., twice daily). In certain embodiments, twice daily administration comprises administration of a compound every 12 hours.

In some embodiments, an aminopyridine in a sustained release composition provides a Tₘₐₓ of about 2 hours to about 6 hours in a human.

In a specific embodiment, an aminopyridine or a pharmaceutically acceptable salt thereof is administered to the patient orally, in a sustained release composition once daily.

In certain embodiments, an aminopyridine or a pharmaceutically acceptable salt thereof is administered in an amount ranging from about 4 mg to about 20 mg (e.g., about 4, 5, 6, 7, 7.5, 8, 9, 10, 11, 12, 12.5, 13, 14, 15, 16, 17, 17.5 or 20 mg) twice daily, preferably in a sustained release composition. In certain embodiments, an aminopyridine or a pharmaceutically acceptable salt thereof is administered in an amount ranging from about 4 mg to about 35 mg (e.g., about 4, 5, 6, 7, 7.5, 8, 9, 10, 11, 12, 12.5, 13, 14, 15, 16, 17, 17.5, 20, 22.5, 25, 27.5, 30, 32.5, or 35 mg) once daily, preferably in a sustained release composition. In some embodiments, an aminopyridine or a pharmaceutically acceptable salt thereof is administered in an amount ranging from about 5 mg to 20 mg, 5 mg to 15 mg, 5 mg to 10 mg, 5 mg to 7.5 mg, 7.5 mg to 12.5 mg, or 7.5 mg to 10 mg twice daily, or about 7.5 mg to 20 mg, 7.5 mg to 15 mg, 10 mg to 30 mg, 10 mg to 20 mg, 10 mg to 15 mg, 15 mg to 30 mg, 15 mg to 40 mg, or 20 mg to 40 mg once daily, preferably in a sustained release composition. In one embodiment, an aminopyridine or a pharmaceutically acceptable salt thereof is administered at a dose of 5 mg once daily or twice daily, preferably in a sustained release composition. In one embodiment, an aminopyridine or a pharmaceutically acceptable salt thereof is administered at a dose of 7.5 mg once daily or twice daily, preferably in a sustained release composition. In another embodiment, an aminopyridine or a pharmaceutically acceptable salt thereof is administered at a dose of 10 mg once daily or twice daily, preferably in a sustained release composition. In some of these embodiments, the aminopyridine is 4-aminopyridine. In specific embodiments, an aminopyridine or a pharmaceutically acceptable salt thereof is administered in an amount ranging from about 5 mg to 20 mg, 8 mg to 15 mg, 7.5 mg to 12.5 mg, or 10 mg to 15 mg once daily (e.g., in a sustained release composition).

In some embodiments, a patient is treated in accordance with the methods described herein for a period of time that is, e.g., for at least 1 week, at least 2 weeks, at least 3 weeks, at least 4 weeks, at least 5 weeks, at least 6 weeks, at least 1 month, at least 2 months, at least 3 months, at least 4 months, at least 5 months, at least 6 months, at least 7 months, at least 8 months, at least 9 months, at least 10 months, at least 11 months, at least 1 year, at least 2 years, at least 3 years, at least 4 years, at least 5 years, at least 10 years, or more than 5 or 10 years. In certain embodiments, the treatment regimen (a particular dose and frequency of administration, which can be selected from any described herein) is stable over a period of time, e.g., for at least 1 week, at least 2 weeks, at least 3 weeks, at least 1 month, at least 2 months, at least 3 months, at least 4 months, at least 6 months, or at least 1 year.

In one embodiment, a patient with multiple sclerosis and an impairment in gait and/or balance is treated with 4-aminopyridine-SR. In one embodiment, the patient is instructed to take the drug twice daily. In one embodiment, the patient is instructed to take 4-aminopyridine-SR in a dose of 4-aminopyridine selected from 3.0, 3.5, 4.0, 4.5, 5.0, 5.5, 6.0, 6.5, 7.0, 7.5, 8.0, 85, 9.0, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, or 25 mg bid. In one embodiment, one of the daily doses of 4-aminopyridine-SR is different from the other dose, and in a particular embodiment a morning dose is higher than the evening dose. In one embodiment, at least one of the twice daily doses of 4-aminopyridine-SR is 10 mg 4-AP.

In another embodiment, the patient is instructed to take 4-aminopyridine-SR once daily. In one embodiment, the patient is instructed to take 4-aminopyridine-SR in a dose of 4-aminopyridine selected from 5.0, 5.5, 6.0, 6.5, 7.0, 7.5, 8.0, 8.5, 9.0, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 mg once daily. In one embodiment, the once daily dose of 4-aminopyridine-SR is 10 mg 4-AP.

In one dosing embodiment, a sufficient amount of an aminopyridine, such as 4-aminopyridine, is provided such that it elicits the steady state levels that are within the range obtained by use of 4-aminopyridine-SR; in one embodiment these steady state values are a maximum concentration at steady state (Cₘₐₓₛₛ) and minimum concentration at steady state (Cₘᵢₙₛₛ). The steady state values can be plasma levels, levels on the brain side of the blood:brain barrier, or levels in the CSF. Preferably, these are plasma levels.

In another embodiment a sufficient amount of aminopyridine, such as 4-aminopyridine, is provided that it elicits the steady state levels that differ not more than about 15, 14, 13, 12, 11, 10, 9, 8, 7, 6, 5, 4, 3, 2, or 1% from the average steady state level (Cₐᵥₛₛ) obtained by use of 4-aminopyridine-SR. The steady state values can be plasma levels, levels on the brain side of the blood:brain barrier, or levels in the CSF. Preferably, these are plasma levels.

### 5.5 Pharmaceutical compositions

The invention also provides pharmaceutical compositions comprising an aminopyridine or a pharmaceutically acceptable salt thereof as described herein. Such pharmaceutical compositions can comprise an amount (e.g., a therapeutically effective amount) of an aminopyridine or a pharmaceutically acceptable salt thereof and a pharmaceutically acceptable carrier. In one embodiment, the pharmaceutical composition is suitable for oral administration and can be, for example, a pill, tablet or capsule. Pharmaceutical compositions can be as described, for example, in U.S. Patent Application Publication No. 2005/0276851, published December 15, 2005 and U.S. Patent Application Publication No. 2005/0228030, published October 13, 2005, the contents of each of which are incorporated by reference herein in their entireties. A pharmaceutical composition can be, for example, an immediate release composition, a controlled release composition, or a sustained release composition. In one embodiment, the pharmaceutical composition comprises a sustained release composition of 4-aminopyridine. The pharmaceutical compositions of the invention are administered to a patient for any of the uses described herein.

An aminopyridine or a pharmaceutically acceptable salt thereof is preferably administered to a patient orally or parenterally in the conventional form of preparations, such as capsules, microcapsules, tablets, granules, powder, troches, pills, suppositories, injections, suspensions, or syrups. Suitable formulations can be prepared by methods commonly employed using conventional, organic or inorganic additives, such as one or more of: an excipient (e.g., sucrose, starch, mannitol, sorbitol, lactose, glucose, cellulose, talc, calcium phosphate or calcium carbonate), a binder (e.g., cellulose, methylcellulose, hydroxymethylcellulose, polypropylpyrrolidone, polyvinylpyrrolidone, gelatin, gum arabic, polyethyleneglycol, sucrose or starch), a disintegrator (e.g., starch, carboxymethylcellulose, hydroxypropylstarch, low substituted hydroxypropylcellulose, sodium bicarbonate, calcium phosphate or calcium citrate), a lubricant (e.g., magnesium stearate, light anhydrous silicic acid, talc or sodium lauryl sulfate), a flavoring agent (e.g., citric acid, menthol, glycine or orange powder), a preservative (e.g., sodium benzoate, sodium bisulfite, methylparaben or propylparaben), a stabilizer (e.g., citric acid, sodium citrate or acetic acid), a suspending agent (e.g., methylcellulose, polyvinyl pyrroliclone or aluminum stearate), a dispersing agent (e.g., hydroxypropylmethylcellulose), a diluent (e.g., water), and base wax (e.g., cocoa butter, white petrolatum or polyethylene glycol). In some embodiments, suitable formulations of an aminopyridine or a pharmaceutically acceptable salt thereof can be prepared using one, two, three or more, or all, of the following additives: colloidal silicon dioxide, hydroxypropyl methylcellulose, magnesium stearate, microcrystalline cellulose, polyethylene glycol, and titanium dioxide.

A pharmaceutically acceptable carrier or vehicle can comprise an excipient, diluent, or a mixture thereof. In some embodiments, suitable formulations (e.g., suitable formulations for oral administration) of an aminopyridine or a pharmaceutically acceptable salt thereof are prepared using one or more of the following excipients: hydroxypropyl methylcellulose, USP; microcrystalline cellulose, USP; colloidal silicon dioxide, NF; magnesium stearate, USP; and Opadry White.

The amount of an aminopyridine or a pharmaceutically acceptable salt thereof that is present in the pharmaceutical composition is preferably an amount that will exercise the desired effect.

An aminopyridine or a pharmaceutically acceptable salt thereof can be administered orally. In some of the embodiments wherein an aminopyridine or a pharmaceutically acceptable salt thereof is administered orally, the composition is formulated in a form of a tablet, a pill or a capsule. An aminopyridine or a pharmaceutically acceptable salt thereof can also be administered intradermally, intramuscularly, intraperitoneally, percutaneously, intravenously, subcutaneously, intranasally, epidurally, sublingually, intracerebrally, intravaginally, transdermally, rectally, by inhalation, or topically to the ears, nose, eyes, or skin. In one embodiment, an aminopyridine or a pharmaceutically acceptable salt thereof is administered to the patient intravenously. The mode of administration is left to the discretion of the health-care practitioner.

The compositions can be in the form of tablets, chewable tablets, capsules, solutions, parenteral solutions, troches, suppositories and suspensions and the like. Compositions can be formulated to contain a daily dose, or a convenient fraction of a daily dose, in a dosage unit, which may be, e.g., a single tablet or capsule or convenient volume of a liquid.

Capsules can be prepared by any known method, such as mixing an aminopyridine or a pharmaceutically acceptable salt thereof with a suitable carrier or diluent and tilling the proper amount of the mixture in capsules. Carriers and diluents include, but are not limited to, inert powdered substances such as starch of many different kinds, powdered cellulose, especially crystalline and microcrystalline cellulose, sugars such as fructose, mannitol and sucrose, grain flours and similar edible powders.

Tablets can be prepared by known methods such as direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

In a specific embodiment, the pharmaceutical composition is a sustained release tablet or capsule of 4-AP.

### 5.6 Combination Treatments

In a specific embodiment, one can combine an aminopyridine or a pharmaceutically acceptable salt thereof with one or more other agents and/or physical or occupational therapies for the treatment of an impairment in gait and/or balance in a patient with multiple sclerosis. In some embodiments, an aminopyridine or a pharmaceutically acceptable salt thereof is administered to a patient concomitantly or sequentially with one or more additional drug or therapy. For example, an aminopyridine or a pharmaceutically acceptable salt thereof can be administered to a patient at the same time, before, or after administration of a drug that controls seizures, a drug that alleviates pain, a drug that reduces fatigue, a drug that relaxes muscle spasms (e.g. benzodiazepienes, baclofen, tizanadine and intrathecal phenol/baclofen), a drug that reduces inflammation (e.g., a corticosteroid), or another drug that is approved for treatment of multiple sclerosis. In particular embodiments, the combination of an aminopyridine or a pharmaceutically acceptable salt thereof and one, two or more additional drug(s) is a fixed dose combination. For example, an aminopyridine or a pharmaceutically acceptable salt thereof and one or more additional drug(s) can be formulated in one composition, such as a pill, a tablet or a capsule. In other embodiments, an aminopyridine or a pharmaceutically acceptable salt thereof is administered to a patient concomitantly (e.g., at the same time, before or after) with physical therapy, occupational therapy, or speech therapy, or plasmapheresis. In some embodiments, an aminopyridine or a pharmaceutically acceptable salt thereof is administered to a patient with multiple sclerosis who uses an assistive device (e.g., cane crutches, or a wheeled walker). In a specific embodiment, the aminopyridine (or salt thereof) and other drug or therapy is administered at the same doctor's visit, or within 1, 2, 3, 4, 5, 6, or 12 hours, or within 1, 2, 3, 4, 5, 6, or 7 days, of each other.

In yet other embodiments, an aminopyridine or a pharmaceutically acceptable salt thereof is administered to a patient without an additional drug or therapy, or without one or more of additional treatments (such as those described above). In certain embodiments, treatment in accordance with the invention (either with or without use of an additional drug or therapy), is more effective than treatment with another drug or therapy known to be used for the treatment of impairments in gait and/or balance in patients with multiple sclerosis.

### 6. EXAMPLES

### 6.1 Example 1: A Study Evaluating the Effects of Dalfampridine on Gait and Balance Parameters in Subjects with Multiple Sclerosis (MS)

### 6.1.1 LIST OF ABBREVIATIONS

The following abbreviations and specialist terms are used in this study protocol (see Table 1).

**Table 1: Abbreviations and Specialist Terms**

| **Abbreviation or Specialist Term** | **Explanation** |
|---|---|
| 2MWT | Two Minute Walk test |
| ADT | Adaptation Test |
| AE | Adverse event |
| BBS | Berg's Balance Scale |
| C | Celsius |
| CFR | Code of Federal Regulations |
| CNS | Central nervous system |
| COG | Center of Gravity |
| CrCl | Creatinine clearance |
| CRF | Case Report Form |
| DCL | Directional Control |
| EC | Eyes Closed |
| EO | Eyes Open |
| EMA | European Medicines Agency |
| EPE | Endpoint Excursion |
| ER | Extended release |
| F | Fahrenheit |
| FAP | Full Analysis Population |
| FDA | Food and Drug Administration |
| GCP | Good Clinical Practices |
| ICH | International Conference on Harmonization |
| 1RB | Institutional Review Board |
| Kg | Kilogram |
| LOS | Limits of Stability Test |
| M | Meter |
| Mg | Milligram |
| MS | Multiple sclerosis |
| MVL | Movement Velocity |
| MXE | Maximum Excursion |
| NDC | National Drug Code |
| NeuroCom or NeuroCom SMART system | NeuroCom SMART Balance Master® |
| PPP | Per-Protocol Population |
| RT | Reaction time |
| SAE | Serious adverse event |
| SAP | Statistical Analysis Plan |
| SQT | Step/Quick turn |
| SOT | Sensory Organization Test |
| SOC | System Organ Class |
| T25FW | Timed 25 Foot Walk |
| TW | Tandem Walk |
| US | Unilateral Stance |
| WA | Walk Across |

### 6.1.2 STUDY OBJECTIVES

The primary objective of this study is to determine changes in overall gait as well as in multiple gait and balance parameters after withdrawal of dalfampridine-ER 10 mg (i.e., a sustained release formulation of 10 mg 4-aminopyridine) in subjects who are receiving the medication consistently for at least two weeks prior to the screening visit as part of their regular clinical care and are considered Improvers based on treatment, defined as having an improvement on the T25FW between an off-drug and on-drug evaluation prior to entry into the study.

Gait and balance parameters will be measured using the NeuroCom SMART Balance Master® using the following tests: Walk Across (WA) measuring step width, step length, speed and step length symmetry; Unilateral Stance (US) measuring mean center of gravity sway velocity; Tandem Walk (TW) measuring step width, speed and end sway; Step/Quick turn (SQT) measuring turn time and turn sway; Sensory Organization Test (SOT) (fixed surface eyes open, fixed surface eyes closed, walls moving eyes open, surface moving eyes open, surface moving eyes closed, surface and walls moving eyes open); Adaptation Test (ADT) measuring the averaged, raw sway and center of force during rotational disturbances; and Limits of Stability Test (LOS) measuring reaction time, movement velocity, endpoint excursion, maximum excursion and directional control.

The secondary objectives of this study is to evaluate gait and balance parameters by measuring changes on the Berg's Balance Scale (BBS), two minute walk test (2MWT), and timed 25 foot walk test (T25FW) after dalfampridine withdrawal.

### 6.1.3 INVESTIGATIONAL PLAN

This is a study to evaluate the effects of dalfampridine withdrawal on gait and postural balance parameters in subjects diagnosed with MS and Improvers in response to treatment with dalfampridine as defined above.

All subjects have to provide written informed consent and then be evaluated by their MS physician before being either included or excluded from the study. If subjects are eligible at screening (day -7, Visit 1) they will be included in the study and the following tests will be administered: the NeuroCom gait and balance tests, BBS, 2MWT, and the T25FW.

The subjects will return to the MS Center one week later (day 1, Visit 2). At this visit, subjects will go through safety and tolerability assessments, a brief physical evaluation, NeuroCom gait and balance testing, BBS, 2MWT and T25FW. A blood sample will be drawn to determine concentration of dalfampridine. Subjects will then be asked to stop taking Dalfampridine. Visits 1 and 2 correspond to period 1, which is defined as the first on-drug period for statistical purposes.

On day 5 (Visit 3), subjects will go through a brief physical examination, NeuroCom gait and balance testing, BBS, 2MWT and T25FW. Blood sample will be drawn to determine the concentration of dalfampridine.

On day 11 (Visit 4), ten days following dalfampridine withdrawal, subjects will return to the clinic and undergo a physical evaluation, NeuroCom gait and balance testing, BBS, 2MWT and T25FW. A blood sample will be drawn to determine the concentration of dalfampridine. Once safety and tolerability evaluation is completed, they will be instructed to re-start dalfampridine. Visits 3 and 4 correspond to period 2, which is defined as the off-drug period for statistical purposes.

The final visit will be on day 15 (Visit 5), period 3, which is defined as the second on-drug period for statistical purposes, and the subjects will undergo physical evaluation, NeuroCom gait and balance testing, BBS, 2MWT, T25FW and final status assessment. Blood will be drawn to determine concentration of dalfampridine and subjects will terminate participation in the study.

Subjects may be video recorded during the T25FW evaluation, but it will not be a requirement to participate in the study. See Section 6.1.7(a) for details of video recordings.

Table 2 provides outline of the schedule of assessments.

**Table 2: Study Schedule and Procedures**

| | **Period 1** | | **Period 2** | | **Period 3** |
|---|---|---|---|---|---|
| | **Visit 1 (screening)** | **Visit 2** | **Visit 3** | **Visit 4** | **Visit 5** |
| | **Day -7** | **Day 1 ± 2** | **Day 5 ± 2** | **Day 11 ± 2** | **Day 15 ± 2** |
| Written Informed Consent | X | | | | |
| Medical History | X | | | | |
| Concomitant Meds/Therapy | X | X | X | X | X |
| Godin Leisure-Time Exercise Questionnaire | X | | | | |
| Physical Examination¹ | X | X | X | X | X |
| Vital Signs | X | X | X | X | X |
| NeuroCom gait and balance tests | X | X | X | X | X |
| BBS | X | X | X | X | X |
| 2MWT | X | X | X | X | X |
| T25FW² | X | X | X | X | X |
| Dalfampridine withdrawal³ | | X | | | |
| Dalfampridine re-start⁴ | | | | X | |
| Final status assessment | | | | | X |
| Blood sample for dalfampridine concentration | | X | X | X | X |
| Video recording of T25FW⁵ | | X | X | X | |
| Review and record adverse events | X | X | X | X | X |

| | | | | | |
|---|---|---|---|---|---|
| Full physical only at screening. Brief physical for the rest of the visits in the study. ² T25FW: Result will be the average of two trials separated by a rest period of 5 minutes. ³ After administration of all battery of tests. ⁴ After administration of all battery of tests. ⁵ Video recording is voluntary and additional consent is required | | | | | |

### 6.1.4 SELECTION AND WITHDRAWAL OF SUBJECTS

Each subject must meet the following criteria for eligibility (inclusion and exclusion criteria) before enrollment in the study.

### (a) Inclusion Criteria

- Diagnosis of multiple sclerosis
- Man or woman 18 to 70 years of age, inclusive
- Receiving dalfampridine-ER consistently for at least 2 weeks prior to the screening visit and are considered Improvers based on treatment, defined as having an improvement on the T25FW between an off-drug and on-drug evaluation prior to entry into the study
- Subjects may be eligible if they have no moderate or severe renal impairment (CrCl > 50 mL/min) as estimated using the Cockcroft-Gault Equation
- No history of seizures except simple febrile seizures
- No urinary tract infection within 4 weeks of screening
- For any concomitant medications, including disease-modifying therapies ("DMT") or other symptomatic treatment, the participant must be on stable dosing regimen during the 4 weeks prior to screening

### (b) Exclusion Criteria

- If any of the above criteria is not met
- Sexually active woman of childbearing potential who are not surgically sterile, < two years post-menopause or are not using effective birth control methods
- Subject that is pregnant or breastfeeding, as confirmed and documented by Evaluator (c) **Withdrawal Criteria**

The withdrawal criteria, which are optional, include one or more of the following reasons:
- Subject experiences an adverse event
- Pregnancy
- Subject is non-compliant with the protocol
- Subject is lost to follow-up
- Subject no longer meets an eligibility criterion, and, in the judgment of the Evaluator, this would affect assessments of clinical status to a significant degree

### 6.1.5 TREATMENT OF SUBJECTS

Dalfampridine-ER tablets, 10 mg given twice daily approximately 12 hours apart will be re-started on day 11 (Visit 4) after drug withdrawal on day 1 (Visit 2), and after all required evaluations are performed.

### 6.1.6 DESCRIPTION OF INVESTIGATIONAL PRODUCT

The investigational product in this study will be commercial drug. AMPYRA® (dalfampridine) Extended Release tablets are available in a 10 mg strength and are a white to off-white, biconvex, oval shaped, film-coated, non-scored tablet with flat edge, debossed with "A10" on one side, containing 10 mg of dalfampridine. Inactive ingredients consist of colloidal silicon dioxide, hydroxypropyl methylcellulose, magnesium stearate, microcrystalline cellulose, polyethylene glycol, and titanium dioxide.

### 6.1.7 STUDY PROCEDURES

### (a) Assessment of Efficacy

### Timed 25 Foot Walk Test (T25FW)

The T25FW test is a measure of ambulatory function that provides quantitative data and is used widely in the MS population. If needed the subject can use their assistive device (cane, crutches, wheeled walker) that is used on a regular basis while walking as quickly as he or she can from one end to the other on a 25-foot (7.62 meters) course. Non-wheeled walkers should generally not be used. This test will be performed in a clearly marked course and will be used for every T25FW test.

For the test the individual will be asked to be lined up with their toes of their shoes on the marked spot on the starting line and as soon as the foot crosses the line, timing using a stopwatch will begin. Timing will conclude when the first foot crosses the 25 foot line. Time will be recorded in seconds and if appropriate rounded in seconds to the nearest tenth of a second. There will be a maximum 5 minutes rest between trials and then the subject will walk the same distance again.

The T25FW will be performed once at Visits 1, 2, 3, 4 and 5.

Video recording: Subjects may be video recorded during the T25FW evaluation, but it will not be a requirement to participate in the study. For subjects who volunteer, video recording of the T25FW will be conducted at Visits 2, 3, and 4. The video footage will be captured using a fixed camera mount to insure consistent perspective and angle. The raw video footage will be edited to ensure that the subjects are unidentifiable. This will be accomplished by using a visual effects software program, Adobe After Effects, to track the face or any personally distinguishing features of the subject and obscure it for the entire length of the segment. The final videos will include title graphics for each segment including the Subject Number, the phase of the study, and the elapsed time for the T25FW. These video recordings will be viewed by the Evaluator to provide a qualitative assessment of change between off-drug and on-drug walks.

### Two Minute Walk Test (2MWT)

The subjects will walk without assistance for 2 minutes and the distance will be measured and timed by the use of a stop watch. The distance covered by walking for 2 minutes will be measured in meters. Verbal confirmation of start and stop will be given. If assistive device is needed it can be used, but no physical assistance is allowed. If assistive device is used, the same device will be used for all tests (retrieved 2012-02-09 from Rehabilitation Measures Database webpage).

### Berg Balance Scale (BBS)

The BBS is a 14-item scale specifically designed to measure balance in the elderly population in a clinical setting (Berg et al., 1989; Tinetti, 1986), but has been used in populations with stroke and traumatic brain injury to assess postural balance (Berg et al., 1995; Newstead et al., 2005). The BBS evaluates subjects ability to sit, stand, reach, maintain single-leg stance, and turn. The scoring is rated from 0 (cannot perform task) to 4 (normal performance of task). This test has reported to give a good prediction of falling with good validity and reliability (Berg et al., 1992a; Berg et al., 1992b; Bogle et al., 1996; Creel et al., 2001; Shumway-Cook et al., 1997). The maximum possible score is 56 and the lowest 0; a score of 45 or below indicates an increased risk of falling (Riddle and Stratford, 1999).

### NeuroCom SMART Balance Master®

The SMART Balance Master® is a machine that provides objective assessments and retraining of the sensory and voluntary motor control of balance with visual feedback on either a stable or unstable support surface and in a stable or dynamic visual environment (Balance Master Family, retrieved 2012-02-09 from NeuroCom SMART Balance Master® webpage).

The system utilizes a dynamic 18" × 18" dual forceplate with rotation capabilities to measure the vertical forces exerted by the subject's feet; and a moveable visual surround.

The following standardized gait and balance assessment protocols will be performed:

### Functional Limitation Assessments:

### Unilateral Stance (US)

This assessment quantifies postural sway velocity with the subject standing quietly on one foot (either right or left) on the forceplate, with eyes open and eyes closed. The relative absence of sway is "stability", i.e., when the instruction is to "hold still" with greater sway indicates less stability, while less sway indicates greater stability. With verbal direction from the test instructor, the subject either lifts their right or left leg and closes or opens their eyes and will try and stand as steadily as possible for ten seconds. The test consists of four conditions, each consisting of three trials, usually is the following order:
a) EO (Eyes Open) Left
b) EO (Eyes Open) Right
c) EC (Eyes Closed) Left
d) EC (Eyes Closed) Right

### Walk Across (WA)

The WA quantifies characteristics of gait as the subject walks across the length of the forceplate. The test characterizes steady state gait by having the subject begin well behind and continuing beyond the forceplate. Because of the length of the forceplate, the test may not be appropriate for highly fit individuals whose stride lengths are greater than five feet (152 cm). Measured parameters are average step width, average step length, speed and step length symmetry.

The following parameters are measured in this test;
*Step Width* - lateral distance in centimeters between the left and right foot on successive steps.

*Step Length* - longitudinal distance in centimeters between successive heel strikes on successive steps.

*Speed* - velocity in centimeters per second of the forward progression.

*Step Length Symmetry* - comparison of right and left step length, expressed as a percentage of the total stride length (right and left length).

### Tandem Walk (TW)

The TW quantifies characteristics of gait as the subject walks heel to toe from one end of the forceplate to the other. Measured parameters are step width, speed, and endpoint sway velocity.

### Step Quick Turn (SQT)

The SQT quantifies characteristic turn performance as the individual takes two steps forward and then quickly turns 180° and then returns to the starting point. Parameters measured are the time to execute the turn and the sway velocity during the turn execution, (turn time and turn sway).

### Sensory Impairment Assessment: Sensory Organization Test (SOT)

Sensory organization (sensory integration; multi-sensory organization) is the ability of an individual to effectively process individual sensory system input cues to maintain balance control. This is done by suppressing inaccurate sensory system inputs while selecting appropriately from other, more accurate sensory cues to generate appropriate motor and postural response strategies. The SOT systematically assesses this ability, objectively isolating and quantifying the use of each sensory system and the adaptive (or maladaptive) responses of the central nervous system.

Sensory Organization Test (SOT) scores are based on the assumption that a normal individual can exhibit anterior to posterior sway over a total range of approximately 12.5 degrees without losing balance. The equilibrium score for each trial is calculated by comparing the angular difference between the subjects maximum anterior to posterior COG displacements to this theoretical maximum displacement. The result is expressed as an inverse percentage between 0 and 100. Scores approaching 0 indicate sway amplitudes approaching the limits of stability with a value of 1 00 indicating perfect stability. A score of 0 indicates that the subject "fell" on that trial.

If a subject's voluntary limits or "cone" of stability is restricted, even small degrees of sway may exceed the available limits of stability and result in a loss of balance or fall during testing. Because of this fact, sensory (SOT) testing will be performed and interpreted in conjunction with motor performance testing. The raw data from which the equilibrium score is calculated also contains significant information about the amplitude, frequency, direction, and regularity of subject sway and will be reviewed accordingly.

### Motor Impairment Tests

### Automatic Motor Assessment: Adaptation Test (ADT)

The ADT assesses a subject's ability to minimize sway when exposed to surface irregularities and unexpected changes in support surface inclination. Sequences of platform rotations in the toes-up or toes-down direction elicit automatic motor responses. For each platform rotation trial, a sway energy score quantifies the magnitude of the force response required to overcome induced postural instability.

Unanticipated toes-up or toes-down rotations elicit automatic responses, which tend to destabilize the subject's balance. During the first (unexpected) trials, the initial disruptive responses are corrected by secondary responses in the opposing muscles. With each subsequent trial, initial reactions are attenuated and secondary responses strengthened to reduce overall sway.

Performance on the ADT requires adequate ankle range of motion and muscle strength, as well as effective motor adaptation.

### Voluntary Motor Assessment: Limits of Stability Test (LOS)

The LOS quantifies the maximum distance a person can intentionally displace their Center of Gravity (COG), i.e. lean their body in a given direction without losing balance, stepping, or reaching for assistance. The measured parameters are reaction time, COG movement velocity, directional control, end point excursion, and maximum excursion. For each of eight trials, the subject maintains their COG centered over the base of support as indicated by a cursor display of the COG position relative to a center target. On command, the subject moves the COG cursor as quickly and accurately as possible towards a second target located on the LOS perimeter (100% of theoretical limits of stability) and then holds a position as close to the target as possible. The subject is allowed up to 8 seconds to complete each trial.

The COG traces for each trial are shown at the top left of the report.

Reaction Time (RT) is the time in seconds between the command to move and the subject's first movement.

Movement Velocity (MVL) is the average speed of COG movement in degrees per second.

Endpoint Excursion (EPE) is the distance of the first movement toward the designated target, expressed as a percentage of maximum LOS distance. The endpoint is considered to be the point at which the initial movement toward the target ceases.

Maximum Excursion (MXE) is the maximum distance achieved during the trial.

Directional Control (DCL) is a comparison of the amount of movement in the intended direction (towards the target) to the amount of extraneous movement (away from the target).

### (b) Study Sequence

The following assessments will be performed in this study:

### Screening: Visit 1 (day -7)

The evaluators will assess eligibility after the following procedures have been performed:
- Obtain signed informed consent
- Assign subject number
- Obtain medical history, MS history, and demographic information
- Complete the Godin Leisure-Time Exercise Questionnaire
- Complete full physical examination including vital signs and height and weight
- Review of concomitant medication and therapies
- Subject must complete gait and balance analysis, BBS, 2MWT and T25FW
- If found eligible, schedule a date and time for the next visit to occur in one week (± 2 days)

### Visit 2 (day 1)

The following procedures will be performed at Visit 2 (day 1):
- Brief physical examination including vital signs
- Record any changes in concomitant medication/therapies
- Subject must complete gait and balance analysis, BBS, 2MWT and T25FW
- Blood draw to determine dalfampridine concentration
- For subjects who volunteer, video recording during the T25FW evaluation (after obtaining written consent)
- Subjects will be instructed to go off dalfampridine
- Schedule a date and time for the next visit that will occur on day 5 (± 2 days)

Visits 1 and 2 correspond to Period 1, which is the defined as the first on-drug period for statistical purposes.

### Visit 3 (day 5)

The following procedures will be performed at Visit 3 (day 5):
- Brief physical examination including vital signs
- Record any changes in concomitant medication/therapies
- Subject must complete gait and balance analysis, BBS, 2MWT and T25FW
- For subjects who volunteer, video recording during the T25FW evaluation
- Schedule a date and time for the next visit that will occur on day 11 (± 2 days)
- Blood draw to determine dalfampridine concentration

### Visit 4 (day 11)

The following procedures will be performed at Visit 4 (day 11):
- Brief physical examination including vital signs
- Record any changes in concomitant medication/therapies
- Subject must complete gait and balance analysis, BBS, 2MWT and T25FW
- For subjects who volunteer, video recording during the T25FW evaluation
- Subjects will be instructed to re-start dalfampridine
- Schedule a date and time for the next visit that will occur on day 15 (± 2 days)
- Blood draw to determine dalfampridine concentration

Visits 3 and 4 correspond to Period 2, which is defined as the off-drug period for statistical purposes.

### Visit 5 (day 15 and Final Visit)

The following procedures will be performed at Visit 5 (day 15):
- Brief physical examination including vital signs
- Record any changes in concomitant medication/therapies
- Subject must complete gait and balance analysis, BBS, 2MWT and T25FW
- Blood draw to determine dalfampridine concentration
- Final status assessment

Visit 5 corresponds to Period 3, which is defined as the second on-drug period for statistical purposes.

### Unscheduled Visits

- Brief physical examination and measurements of vital signs
- Record any change in concomitant medication/therapies
- Review and record any adverse events since last visit
- Complete a final status assessment if visit is for early termination

### 6.1.8 STATISTICS

This section outlines the statistical methods to be used for the analysis of the study data.

All computations will be performed using SAS® Version 9 or higher. Statistically significant treatment differences will be declared if the resulting p-value is less than 0.05. All tests will be two-sided. No interim analysis is planned.

### (a) Statistical Power and Sample Size

An estimated 20 subjects will enroll in this study. This sample size will provide adequate parameter estimates to aid in the design of future studies.

### (b) Subject Populations

The Full Analysis Population (FAP) will be the basis of the primary efficacy analysis and include all subjects who have at least one baseline (Visit 1 or Visit 2) and one post-baseline (Visit 3 or Visit 4) assessment in both on-drug and off-drug periods. A Per-Protocol Population (PPP) is a sub-population of the FAP and will consist of all FAP subjects who complete all visits with no major protocol violations. The Safety Population will include all subjects who are enrolled into the study at Visit 1 since all subjects coming into the study will already be taking dalfampridine.

### (c) Demographic and Background Variables

Demographic, disease background, and baseline data will be summarized using descriptive statistics. Other baseline variables include the following assessments at screening: Concomitant medications/therapies, physical examination, vital signs including height, and weight. For continuous variables, descriptive statistics will include the mean, standard error of the mean, standard deviation, median, minimum and maximum values. For categorical variables, descriptive statistics will include the number and percentage subjects falling in each category.

### (d) Analysis of Efficacy

### Primary Efficacy Variable

The primary endpoint will be determined on an intra-subject basis, predicated on the normative data from the NeuroCom SMART system. The parameter that is closest to normal at baseline will be used as the dependent variable in the analysis. Parameters are listed in Section "Additional Efficacy Variables" below.

### Secondary Efficacy Variable

The key secondary endpoint will be analyzed in a similar manner as the primary endpoint but will be based on the most deviant relative to the normative data.

### Additional Efficacy Variables

Each parameter from the NeuroCom SMART system listed below will also be analyzed in a similar manner to the primary and secondary efficacy variables:
- Walk Across (WA) measuring step width, step length, speed and step length symmetry
- Unilateral Stance (US) measuring mean center of gravity sway velocity
- Tandem Walk (TW) measuring step width, speed and end sway
- Step/Quick turn (SQT) measuring turn time and turn sway
- Sensory Organization Test (SOT) (fixed surface eyes open, fixed surface eyes closed, walls moving eyes open, surface moving eyes open, surface moving eyes closed, surface and walls moving eyes open)
- Adaptation Test (ADT) measuring the averaged, raw sway and center of force during rotational disturbances
- Limits of Stability Test (LOS) measuring reaction time, movement velocity, endpoint excursion, maximum excursion and directional control.

In addition, the following efficacy measures will also be analyzed in a similar manner:
- Berg Balance Scale (BBS)
- Two Minute Walk Test (2MWT)
- Timed 25 Foot Walk Test (T25FW)

### Methods of Analysis

For a single individual, the measurement data for one efficacy variable is displayed in Table 3.

**Table 3: Efficacy Variable for a Single Individual**

| Subject | Period | Visit / Day | Treatment | Efficacy Variable |
|---|---|---|---|---|
| 1 | 1 | 1 / -7 | Dalfampridine | Y₁₁ |
| 1 | 1 | 2 / 1 | Dalfampridine | Y₁₂ |
| 1 | 2 | 3 / 5 | Off-drug | Y₁₃ |
| 1 | 2 | 4 / 11 | Off-drug | Y₁₄ |
| 1 | 3 | 5 / 15 | Dalfampridine | Y₁₅ |

Each efficacy variable will be analyzed in 4 ways:
1. Primary method - A mixed-model analysis of variance (ANOVA) will be used to model each efficacy endpoint.
2. Secondary methods will compare:
   - The change from Visit 4 to Visit 5
   - the change from baseline to Visit 4
   - the change from baseline to Visit 5

For each variable, baseline will be defined as the average of Visits 1 and 2 while the subject is on drug. For a particular variable, if a subject does not have an assessment at Visit 4, the value will be imputed using the assessment at Visit 3.

The correlation between the individual parameters of the NeuroCom SMART system and the other clinical efficacy measures (BBS, 2MWT, T25FW) will be calculated.

### 6.1.9 REFERENCES FOR EXAMPLE 1

- Berg, K., Wood-Dauphinee, S., & Williams, J. I. (1995). The Balance Scale: reliability assessment with elderly residents and patients with an acute stroke. [Research Support, Non-U.S. Gov't]. Scandinavian journal of rehabilitation medicine, 27(1), 27-36.
- Berg, K., Wood-Dauphinee, SL, Williams, JI, Gayton, D. (1989). Measuring balance in the elderly: preliminary development of an instrument. Physiother Canada, 41, 304-311.
- Berg, K. O., Maki, B. E., Williams, J. I., Holliday, P. J., & Wood-Dauphinee, S. L. (1992). Clinical and laboratory measures of postural balance in an elderly population. [Research Support, Non-U.S. Gov't]. Archives of physical medicine and rehabilitation, 73(11), 1073-1080.
- Berg, K. O., Wood-Dauphinee, S. L., Williams, J. I., & Maki, B. (1992). Measuring balance in the elderly: validation of an instrument. [Comparative Study Research Support, Non-U.S. Gov't]. Canadian journal of public health. Revue canadienne de sante publique, 83 Suppl 2, S7-11.
- Bogle Thorbahn, L. D., & Newton, R. A. (1996). Use of the Berg Balance Test to predict falls in elderly persons. Physical therapy, 76(6), 576-583; discussion 584-575.
- Creel, G. L., Light, K. E., & Thigpen, M. T. (2001). Concurrent and construct validity of scores on the Timed Movement Battery. [Clinical Trial Randomized Controlled Trial]. Physical therapy, 81(2), 789-798.
- Frohman, E. M. (2003). Multiple sclerosis. [Case Reports Research Support, Non-U.S. Gov't Review]. The Medical clinics of North America, 87(4), 867-897, viii-ix.
- Goodman A., Brown T., Krupp L., et al. (2009). Sustained-release oral fampridine in multiple sclerosis: A randomised, double-blind, controlled trial. Lancet 373, 732-738
- Goodman A., Brown T., Edwards K., et al. (2010). A phase 3 trial of extended release oral dalfampridine in multiple sclerosis. Ann Neurol 68, 494-502
- Newstead, A. H., Hinman, M. R., & Tomberlin, J. A. (2005). Reliability of the Berg Balance Scale and balance master limits of stability tests for individuals with brain injury. [Clinical Trial Validation Studies]. Journal of neurologic physical therapy : JNPT, 29(1), 18-23.
- Riddle, D. L., & Stratford, P. W. (1999). Interpreting validity indexes for diagnostic tests: an illustration using the Berg balance test. Physical therapy, 79(10), 939-948.
- Shumway-Cook, A., Baldwin, M., Polissar, N. L., & Gruber, W. (1997). Predicting the probability for falls in community-dwelling older adults. [Research Support, Non-U.S. Gov't], Physical therapy, 77(8), 812-819.
- Tinetti, M. E. (1986). Performance-oriented assessment of mobility problems in elderly patients. [Research Support, Non-U.S. Gov't Research Support, U.S. Gov't, P.H.S.]. Journal of the American Geriatrics Society, 34(2), 119-126.

### 6.2 Example 2: A Study Using NeuroCom SMART Balance Master®, Berg Balance Scale, 2MWT and T25FW to Evaluate the Effects of Dalfampridine on Gait and Balance Parameters in Subjects with Multiple Sclerosis (MS)

### 6.2.1 LIST OF ABBREVIATIONS

Refer to Table 1 in Example 1, section 6.1.1, for the abbreviations used in this study protocol.

### 6.2.2 STUDY OBJECTIVES

The objectives for this study were the same as those described in Example 1, section 6.1.2.

### 6.2.3 INVESTIGATIONAL PLAN

For this study, the investigational plan was essentially the same as described in Example 1, section 6.1.3. Brief description of the study schedule is presented below:

Tests were administered at every study visit. The study was subdivided into three study periods as follows:
Period 1 (the first on-drug period lasting 1 week): All eligible subjects were being treated with Dalfampridine-ER for at least two weeks at the start of the study and were considered to be Improvers based on treatment, per the inclusion/exclusion criteria (see section 6.1.4, Example 1): dalfampridine-ER tablets ("D-ER"), 10 mg, given orally twice daily approximately 12 hours apart. Eligible subjects had a screening visit (Day -7, Visit 1) and returned to the study center one week later (Day 1, Visit 2). Dalfampridine-ER was then withdrawn from the study subjects for study Period 2.
Period 2 (the off-drug period lasting 10 days): Following dalfampridine-ER withdrawal, subjects returned to the clinic on Day 5 (Visit 3) and Day 11 (Visit 4). Dalfampridine-ER was then reinitiated for study Period 3.
Period 3 (the second on-drug period lasting 4 days): the final visit was on Day 15 (Visit 5).

### 6.2.4 SELECTION AND WITHDRAWAL OF SUBJECTS

The criteria used for selection and withdrawal of subjects were the same as those described in Example 1, section 6.1.4.

### 6.2.5 DESCRIPTION OF INVESTIGATIONAL PRODUCT

The investigational product used in this study was identical to the one described in Example 1, section 6.1.6.

### 6.2.6 STUDY PROCEDURES

### (a) Assessment of Efficacy

### NeuroCom SMART Balance Master®

The NeuroCom SMART Balance Master® was used to assess gait and balance using the assessments described in Example 1, section 6.1.7 (a), paragraphs [00129]-[00153].

### Timed 25 Foot Walk Test (T25FW) and Two Minute Walk Test (2MWT)

The T25FW and 2MWT tests were used to assess gait parameters using the protocols described in Example 1, section 6.1.7 (a), paragraphs [00123]-[00127].

### Berg Balance Scale (BBS)

The BBS test was used to assess balance parameters using the protocols described in Example 1, section 6.1.7 (a), paragraph [00128].

### (b) Study Sequence

The study sequence for this Example was the same as described in Example 1, section 6.1.7 (b).

### 6.2.7 STATISTICS

The statistical methods used for the analysis of the study data were the same as those described in Example 1, section 6.1.8.

### (a) Statistical Power and Sample Size

Twenty subjects were enrolled in this study to provide adequate parameter estimates to aid in the design of future studies.

### (b) Baseline Demographic Characteristics and Disease History

A total of 20 subjects enrolled into the study. The disposition of the study subjects is presented in Table 4. The baseline demographic characteristics and disease history (including dalfampridine-ER 10 mg use prior to study enrollment) of the study subjects are presented in Tables 5 and 6.

**Table 4: Summary of Subject Disposition (All Subjects)**

| | All Subjects |
|---|---|
| Subjects Screened | 21 |
| Subjects Enrolled | 20 (95.2%) |
| Subjects Enrolled | 20 |
| Safety Population* | 20 (100.0%) |
| Full Analysis Population** | 20 (100.0%) |
| | |
| Subjects That Completed Period 01 | 20 (100.0%) |
| Subjects That Completed Period 02 | 20 (100.0%) |
| Subjects That Completed the Study | 20 (100.0%) |
| Subjects that Discontinued from the Study | 0 |

| | |
|---|---|
| * Safety Population: All subjects who were enrolled in the study at Visit 1. ** Full Analysis Population: All subjects who had at least one baseline (Visit 1 or Visit 2) and one post-baseline (Visit 3 or Visit 4) assessment in both on-drug and off-drug periods. | |

**Table 5: Baseline Demographic Characteristics (Safety Population)**

| | Safety Population (N=20) |
|---|---|
| Gender - n (%) | |
| Female | 12 (60.0%) |
| Male | 8 (40.0%) |
| Age (years) | |
| N | 20 |
| Mean (Standard Error) | 53.1 (2.45) |
| Standard Deviation | 10.96 |
| Median | 56.5 |
| (Min, Max) | (20, 65) |
| Race - n (%) | |
| American Indian or Alaska Native | 1 (5.0%) |
| Black or African American | 1 (5.0%) |
| White | 18 (90.0%) |
| Ethnicity - n (%) | |
| Not Hispanic or Latino | 20 (100.0%) |

| | |
|---|---|
| Safety Population: All subjects who were enrolled in the study at Visit 1. | |

**Table 6: Disease History and Dalfampridine-ER 10 mg Use (Safety Population)**

| | Safety Population (N=20) |
|---|---|
| MS Diagnosis Type - n (%) | |
| Primary-Progressive | 2 (10.0%) |
| Progressive-Relapsing | 2 (10.0%) |
| Relapsing-Remitting | 13 (65.0%) |
| Secondary-Progressive | 3 (15.0%) |
| Duration of Disease (years) | |
| N | 20 |
| Mean (Standard Error) | 11.3 (1.88) |
| Standard Deviation | 8.43 |
| Median | 7.8 |
| (Min, Max) | (3, 29) |
| Time on Dalfampridine-ER 10 mg Prior to Study Enrollment (days) | |
| N | 20 |
| Mean (Standard Error) | 315.3 (60.47) |
| Standard Deviation | 270.45 |
| Median (Min, Max) | 355.5 (14, 685) |

| | |
|---|---|
| Safety Population: All subjects who were enrolled in the study at Visit 1. | |

### (c) Analysis of Efficacy

### First Primary Efficacy Endpoint

The first endpoint was a composite score assessing each subject's gait. The overall gait composite score was predicated on composite scores from three of the tests from the NeuroCom SMART system: Walk Across, Tandem Walk, and Step/Quick Turn.

### Second Primary Efficacy Endpoint

The second endpoint was a composite score assessing each subject's balance. The overall balance composite score was predicated on composite scores from three different tests from the NeuroCom SMART system: Sensory Organization Test, Adaptation Test, and Limits of Stability.

### Calculation of Gait and Balance Scores Using NeuroCom SMART system tests

The overall gait and balance composite scores were calculated based on Z-scores from their three respective NeuroCom SMART system tests. The gait Z-score was computed by taking the average of its three component Z-scores. The balance Z-score used different weights for the three component Z-scores in its computation (50% for Sensory Organization Test, 20% for the Adaptation Test, and 30% for the Limits of Stability). Change in balance, based on individual z-scores from the balance components, was evaluated only if the gait outcome was significant. The gait and balance Z-scores were then transformed into percentile scores using the standard normal distribution. Thus, the Z-scores for gait and for balance were each transformed to a scale from 0 to 100. The primary method of analysis to compare the treatment effect and period effects on gait and balance was a mixed-model analysis of variance (ANOVA).

### Additional Efficacy Endpoints

The additional efficacy measures collected during the study were Berg Balance Scale (BBS), Two Minute Walk Test (2MWT) and Timed 25 Foot Walk (T25FW).

### 6.2.8 EFFICACY RESULTS

### 6.2.8.1. Efficacy Results Using NeuroCom SMART Balance Master®

### (a) Overall Treatment Comparisons

Results for the overall treatment comparisons (pooling dalfampridine-ER across all the on-treatment study visits) are summarized in Table 7. Subjects did significantly better on overall gait while being treated with dalfampridine-ER than when untreated (p = 0.015). On-average, the subjects scored approximately 4 points better while on-treatment. Results for the other endpoint, overall balance, were not significant in this study with respect to treatment since no significant difference in overall balance score between on- and off-drug periods was observed.

**Table 7: Repeated Measures Treatment Comparisons - Composite Scores for Gait and Balance (Full Analysis Population)**

| Parameter Statistic | D-ER vs. D-ER Withdrawn |
|---|---|
| Gait^{(a)} | |
| Difference in LS Means | 4.04 |
| Standard Error | 1.51 |
| p-value | 0.015 |
| | |
| Balance^{(b)} | |
| Difference in LS Means | -2.38 |
| Standard Error | 2.97 |
| p-value | 0.434 |

| | |
|---|---|
| Full Analysis Population: All subjects who had at least one baseline (Visit 1 or Visit 2) and one post-baseline (Visit 3 or Visit 4) assessment in both on-drug and off-drug periods, respectively. Abbreviations: D-ER= Dalfampridine-ER (a): Overall Gait Composite Percentile (b) Overall Balance Composite Percentile NOTE: P-values are based on a mixed model ANOVA with a fixed effect for treatment. | |

### (b) Study Period Treatment Comparisons

Results for the treatment comparisons of the composite scores for overall gait and balance by study period are summarized in Table 8. With respect to overall gait, subjects did significantly better during on-treatment Period 3 than during off-treatment Period 2 (p=0.032) and numerically (but not significantly) better during on-treatment Period 1 than during off-treatment Period 2 (p=0.124). There were no significant differences on how subjects did during the on-treatment periods (Period 1 vs. Period 3; p=0.380).

For overall balance, subjects did significantly better (p=0.029) during Period 3 (on-treatment) than during Period 2 (off-treatment). Subjects also did significantly better (p<0.001) during Period 3 (on-treatment) than during Period 1 (on-treatment), and subjects did numerically better (but not significantly, p=0.114) during Period 2 (off-treatment) than during Period 1 (on-treatment). *A priori* defined repeated measures analysis showed a progressive increase in a balance scores from Period 1 to Period 2 to Period 3.

**Table 8: Repeated Measures Period Comparisons - Composite Scores for Gait and Balance (Full Analysis Population)**

| Parameter Statistic | Period 1 vs. Period 2 | Period 1 vs. Period 3 | Period 2 vs. Period 3 |
|---|---|---|---|
| Gait^{(a)} | | | |
| Difference in LS Means | 3.31 | -3.16 | -6.47 |
| Standard Error | 1.60 | 2.32 | 2.34 |
| p-value | 0.124 | 0.380 | 0.032 |
| | | | |
| Balance^{(b)} | | | |
| Difference in LS Means | -6.47 | -17.14 | -10.66 |
| Standard Error | 3.06 | 3.09 | 3.79 |
| p-value | 0.114 | <.001 | 0.029 |

| | | | |
|---|---|---|---|
| Full Analysis Population: All subjects who had at least one baseline (Visit 1 or Visit 2) and one post-baseline (Visit 3 or Visit 4) assessment in both on-drug and off-drug periods, respectively. Abbreviations: D-ER= Dalfampridine-ER (a): Overall Gait Composite Percentile (b) Overall Balance Composite Percentile NOTE: P-values are based on a mixed model ANOVA with a fixed effect for period. Pairwise comparisons are Tukey adjusted. | | | |

### 6.2.8.2. Efficacy Results Using Berg Balance Scale (BBS), Two Minute Walk Test (2MWT) and Timed 25 Foot Walk (T25FW)

### (a) Overall Treatment Comparisons

Results for the overall treatment comparisons (pooling dalfampridine-ER across all on-treatment study visits) are summarized in Table 9. Subjects performed significantly better on all three measurements while being treated with dalfampridine-ER than when untreated (BBS: p=0.003, 2MWT: p=0.006, T25FW: p<0.001). For the BBS Total Score, on-average, subjects scored 1.7 points higher while being treated. For the 2MWT, subjects on-average walked a total distance of 7.73 meters further while taking dalfampridine-ER. Lastly, for the T25FW, subjects walked 0.36 ft/s faster while on-treatment.

**Table 9: Repeated Measures Treatment Comparisons - Berg Balance Scale, Two Minute Walk Test, Timed 25 Foot Walk (Full Analysis Population*)**

| Parameter Statistic | D-ER vs. D-ER Withdrawn |
|---|---|
| BBS-Total Score | |
| Difference in LS Means | 1.7 |
| Standard Error | 0.5 |
| p-value | 0.003 |
| | |
| 2MWT-Total Distance (m) | |
| Difference in LS Means | 7.73 |
| Standard Error | 2.50 |
| p-value | 0.006 |
| | |
| T25FW-Walking Speed (ft/s) | |
| Difference in LS Means | 0.36 |
| Standard Error | 0.08 |
| p-value | <0.001 |

| | |
|---|---|
| Abbreviations: D-ER= Dalfampridine-ER * Full Analysis Population: All subjects who had at least one baseline (Visit 1 or Visit 2) and one post-baseline (Visit 3 or Visit 4) assessment in both on-drug and off-drug periods. NOTE: P-values are based on a mixed model ANOVA with a fixed effect for treatment. | |

### (b) Study Period Treatment Comparisons

Results for the treatment comparisons of BBS, 2MWT, and T25FW by study period are summarized in Table 10. When comparing Period 1 (on-treatment) to Period 2 (off-treatment), subjects performed significantly better at all three tests when on-treatment. Likewise, when comparing Period 3 (on-treatment) to Period 2 (off-treatment), subjects performed significantly better at all three tests when on-treatment. There were no significant differences in how subjects performed during the on-treatment periods (Period 1 vs. Period 3); however, there was a numerical difference between scores in Period 1 vs. Period 3.

**Table 10: Repeated Measures Period Comparisons - Berg Balance Scale, Two Minute Walk Test, Timed 25 Foot Walk (Full Analysis Population*)**

| Parameter Statistic | Period 1 vs. Period 2 | Period 1 vs. Period 3 | Period 2 vs. Period 3 |
|---|---|---|---|
| BBS- Total Score | | | |
| Difference in LS Means | 1.5 | -0.5 | -2.0 |
| Standard Error | 0.6 | 0.7 | 0.7 |
| p-value | 0.040 | 0.740 | 0.018 |
| | | | |
| 2MWT-Total Distance (m) | | | |
| Difference in LS Means | 7.84 | -3.46 | -11.29 |
| Standard Error | 2.50 | 2.34 | 3.47 |
| p-value | 0.014 | 0.322 | 0.011 |
| | | | |
| T25FW-Walking Speed (ft/s) | | | |
| Difference in LS Means | 0.38 | -0.04 | -0.42 |
| Standard Error | 0.09 | 0.06 | 0.12 |
| p-value | <0.001 | 0.783 | 0.006 |

| | | | |
|---|---|---|---|
| Abbreviations: D-ER= Dalfampridine-ER * Full Analysis Population: All subjects who had at least one baseline (Visit 1 or Visit 2) and one post-baseline (Visit 3 or Visit 4) assessment in both on-drug and off-drug periods. NOTE: P-values are based on a mixed model ANOVA with a fixed effect for period. Pairwise comparisons are Tukey adjusted. | | | |

### 6.2.9 SAFETY RESULTS

A total of 6 (30%) of the subjects reported at least one adverse event in this study: 1 (5%) in Period 1 and 5 (25%) in Period 2. Fall was the only adverse event reported in more than one subject (2 subjects). No adverse events led to study discontinuation and there were no serious adverse events reported among the enrolled subjects during the study.

### 6.2.10 CONCLUSIONS

Dalfampridine-ER had positive and significant effects on gait. Observed trends for improvement in balance with time shown may reflect a previously reported NeuroCom SMART Balance Master® learning effect in addition to pharmacologic effects. Subjects performed significantly better in T25FW, 2MWT, and BBS while on drug than off drug.

### 6.3 Example 3: Detailed Description of the Study to Evaluate the Effects of Dalfampridine on Gait and Balance Parameters in Subjects with Multiple Sclerosis (MS) Presented in Example 2

This example provides a more detailed description of the experimental design of the study presented in Example 2 and the data obtained therein.

### 6.3.1 LIST OF ABBREVIATIONS

The following abbreviations and specialist terms are used in this study protocol (see Table 11).

**Table 11: Abbreviations and Specialist Terms**

| **Abbreviation or Specialist Term** | **Explanation** |
|---|---|
| 2MWT | 2-minute Walk Test |
| ADT | Adaptation Test |
| AE | Adverse event |
| ANOVA | Analysis of Variance |
| BBS | Berg's Balance Scale |
| BMI | Body Mass Index |
| CFR | Code of Federal Regulations |
| cm | Centimeter |
| COG | Center of gravity |
| CRA | Clinical Research Associate |
| CRF | Case Report Form |
| CRO | Contract Research Organization |
| deg | Degree |
| D-ER | Dalfampridine extended release |
| ER | Extended release |
| FAP | Full Analysis Population |
| FDA | Food and Drug Administration |
| ft | Feet |
| 2MWT | 2-minute Walk Test |
| GCP | Good Clinical Practice |
| ICF | Informed Consent Form |
| ICH | International Conference on Harmonization |
| IRB | Institutional Review Board |
| LOS | Limits of Stability |
| m | Meter |
| mmHg | Millimeter of mercury |
| min | Minute |
| MS | Multiple sclerosis |
| NeuroCom or NeuroCom system or NeuroCom SMART system | NeuroCom SMART Balance Master® |
| PPP | Per Protocol Population |
| SAE | Serious adverse event |
| SAP | Statistical analysis plan |
| SD | Standard deviation |
| sec | Seconds |
| SOT | Sensory Organization Test |
| SQT | Step/Quick Turn |
| T25FW | Timed 25-foot Walk Test |
| TEAE | Treatment-emergent adverse event |
| TW | Tandem Walk |
| US | Unilateral Stance |
| V | Visit |
| WA | Walk Across |

### 6.3.2 STUDY OBJECTIVES

The primary objective of this signal detection study was to determine changes in overall gait as well as in multiple gait and balance parameters after withdrawal of dalfampridine-ER 10 mg (i.e., a sustained release formulation of 10 mg 4-aminopyridine) in subjects who received medication consistently for at least two weeks prior to the screening visit as part of their regular clinical care and were considered Improvers based on treatment, defined as having an improvement on the T25FW between an off-drug and on-drug evaluation prior to entry into the study.

Gait and balance parameters were measured using the NeuroCom SMART Balance Master® and included the following tests: Walk Across (WA) measuring step width, step length, speed, and step length symmetry; Unilateral Stance (US) measuring mean center of gravity sway velocity; Tandem Walk (TW) measuring step width, speed, and end sway; Step/Quick Turn (SQT) measuring turn time and turn sway; Sensory Organization Test (SOT) (fixed surface eyes open, fixed surface eyes closed, walls moving eyes open, surface moving eyes open, surface moving eyes closed, surface and walls moving eyes open); Adaptation Test (ADT) measuring the averaged raw sway and center of force during rotational disturbances; and Limits of Stability Test (LOS) measuring reaction time, movement velocity, endpoint excursion, maximum excursion, and directional control.

The secondary objectives of this study were to assess the changes on the Berg's Balance Scale (BBS), Two Minute Walk Test (2MWT), and T25FW after dalfampridine-ER withdrawal.

### 6.3.3 INVESTIGATIONAL PLAN

### 6.3.3.1. OVERALL STUDY DESIGN AND PLAN: DESCRIPTION

This was a single-center, open-label, signal detection study to evaluate the effects of dalfampridine-ER withdrawal on gait and postural balance parameters in subjects diagnosed with MS that are Improvers in response to treatment with dalfampridine-ER.

Figure 2 illustrates the treatment schedule. The study consisted of 3 periods: a 7 day on-drug screening phase, a 10 day off-drug phase, and a second 4 day on-drug phase. See Schedule of Assessments (Table 12) for more details.

Approximately 20 subjects were enrolled in this study.

No interim analyses were planned for this study.

**Table 12: Schedule of Assessments**

| | **Period 1** | | **Period 2** | | **Period 3** |
|---|---|---|---|---|---|
| | **Visit 1 (screening)** | **Visit 2** | **Visit 3** | **Visit 4** | **Visit 5** |
| | **Day -7** | **Day 1 ± 2** | **Day 5 ± 2** | **Day 11 ± 2** | **Day 15 ± 2** |
| Written Informed Consent | X | | | | |
| Medical History | X | | | | |
| Concomitant Medications/Therapy | X | X | X | X | X |
| Godin Leisure-Time Exercise Questionnaire | X | | | | |
| Physical Examination¹ | X | X | X | X | X |
| Vital Signs | X | X | X | X | X |
| NeuroCom gait and balance tests | X | X | X | X | X |
| BBS | X | X | X | X | X |
| 2MWT | X | X | X | X | X |
| T25FW² | X | X | X | X | X |
| Dalfampridine-ER withdrawal³ | | X | | | |
| Dalfampridine-ER re-start⁴ | | | | X | |
| Final status assessment | | | | | X |
| Blood sample for dalfampridine concentration | | X | X | X | X |
| Video recording of T25FW⁵ | | X | X | X | |
| Review and record adverse events | X | X | X | X | X |

| | | | | | |
|---|---|---|---|---|---|
| Full physical at screening; brief physical for the remainder of the visits in the study. ² T25FW: result was the average of 2 trials separated by a rest period of 5 minutes. ³ After administration of all battery of tests. ⁴ After administration of all battery of tests. ⁵ Video recording was voluntary and additional consent was required. | | | | | |

### 6.3.3.2. DISCUSSION OF THE STUDY DESIGN, INCLUDING THE CHOICE OF CONTROL GROUPS

All subjects provided a written informed consent and then were evaluated by their MS physician before being either included or excluded from the study. Eligibility was determined at screening (day -7, Visit 1) through review of medical history, the amount of physical activity level in the past week based on the Godin Leisure-Time Exercise Questionnaire, physical examination, and vital sign measurements. If subjects were eligible at screening, they were included in the study and the following tests were administered: NeuroCom tests, BBS, 2MWT, and the T25FW.

The subjects returned to the MS Center on day 1 (Visit 2) and underwent safety and tolerability assessments, NeuroCom gait and balance testing, BBS, 2MWT, and the T25FW. A blood sample was drawn to determine the concentration of dalfampridine. Subjects were instructed to stop taking dalfampridine-ER and safety recommendations were given. Visits 1 and 2 correspond to Period 1, which was defined as the first on-drug period for statistical purposes.

On day 5 (Visit 3), subjects underwent safety and tolerability assessments, NeuroCom gait and balance testing, BBS, 2MWT, and the T25FW. A blood sample was drawn to determine the concentration of dalfampridine.

On day 11 (Visit 4), subjects underwent safety and tolerability assessments, NeuroCom gait and balance testing, BBS, 2MWT, and the T25FW. A blood sample was drawn to determine the concentration of dalfampridine. Once safety and tolerability evaluation was complete, the subjects were instructed to re-start dalfampridine-ER. Visits 3 and 4 correspond to Period 2, which is defined as the off-drug period for statistical purposes.

On the final visit (day 15, Visit 5), the subjects underwent safety and tolerability assessments, NeuroCom gait and balance testing, BBS, 2MWT, T25FW, and final status assessment. Blood was drawn to determine the concentration of dalfampridine and subjects terminated participation in the study. Visit 5 was Period 3, which was defined as the second on-drug period for statistical purposes.

Although not a requirement for study participation, some subjects were video recorded during the T25FW evaluation at Visits 2, 3, and 4. If a subject volunteered for video recording, an additional signed consent form was obtained.

### 6.3.3.3. SELECTION AND WITHDRAWAL OF SUBJECTS

### 6.3.3.3.1 Inclusion Criteria

For inclusion in the study, subjects had to fulfill all of the following criteria:
- Diagnosis of multiple sclerosis
- Man or woman 18 to 70 years of age, inclusive
- Receiving dalfampridine-ER consistently for at least 2 weeks prior to the screening visit and were considered Improvers based on treatment, defined as an improvement on the T25FW between an off-drug and on-drug evaluation prior to entry into the study
- No moderate or severe renal impairment (CrCl >50 mL/min) as estimated using the Cockcroft-Gault Equation
- No history of seizures except simple febrile seizures
- No urinary tract infection within 4 weeks of screening
- For any concomitant medications, including disease modifying therapies (DMT) or other symptomatic treatment, the participant must be on stable dosing regimen during the 4 weeks prior to screening

### 6.3.3.3.2 Exclusion Criteria

Any of the following was regarded as a criterion for exclusion from the study:
- If any of the above criteria was not met
- Sexually active woman of childbearing potential who was not surgically sterile, <2 years post-menopause or was not using effective birth control methods
- Subject that was pregnant or breastfeeding, as confirmed and documented by Evaluator

### 6.3.3.3.3 Removal of Patients from Therapy or Assessment

Subjects were informed that they had the right to withdraw from the study at any time for any reason, without prejudice to their medical care. The Evaluator also had the right to withdraw subjects from the study for any of the following reasons:
- Subject experienced an adverse event
- Pregnancy
- Subject was non-compliant with the protocol
- Subject was lost to follow-up
- Evaluator's decision, which may include: subject no longer met an eligibility criterion, and, in the judgment of the Evaluator, this affected assessments of clinical status to a significant degree
- Other reason not defined above

### 6.3.3.4. TREATMENTS

### 6.3.3.4.1 Treatments Administered

Subjects were administered dalfampridine-ER tablets, 10 mg twice daily, for at least 2 weeks prior to the day -7 (Visit 1) and during the 7 day screening period; they were withdrawn from dalfampridine-ER on day 1 (Visit 2) for 10 days and were re-started on day 11 (Visit 4) after all required evaluations were performed.

### 6.3.3.4.2 Identity of Investigational Product(s)

Commercial drug (AMPYRA®, NDC 10144-427-60 bottles of 60 tablets) was obtained by the subject, by prescription. The investigational product in this study was commercial drug. The drug was in a form of tablets, film coated, extended release, 10 mg strength.

### 6.3.3.4.3 Method of Assigning Patients to Treatment Groups

This was an open-label study in which all subjects were administered dalfampridine-ER tablets, 10 mg twice daily, on days -7 to -1 and on days 11 to 15.

### 6.3.3.4.4 Selection of Doses in the Study

Dalfampridine-ER tablets 10 mg twice daily.

### 6.3.3.4.5 Selection and Timing of Dose for Each Patient

At screening (days -7 to -1) subjects were administered dalfampridine-ER tablets, 10 mg twice daily, approximately 12 hours apart. Subjects were withdrawn from dalfampridine-ER on day 1 (Visit 1) and were re-started on dalfampridine-ER on days 11 to 15.

### 6.3.3.4.6 Blinding

This was an open-label study.

### 6.3.3.4.7 Prior and Concomitant Therapy

Concomitant medications and therapies were reviewed at every visit.

### 6.3.3.4.8 Treatment Compliance

The subject completed dosage records during participation in the study to ensure that investigational product was taken according to the study schedule. Additionally, treatment compliance was confirmed by measuring dalfampridine plasma concentration.

### 6.3.3.5. EFFICACY AND SAFETY VARIABLES

The following efficacy and safety measures were collected at the times shown in Table 12:

### 6.3.3.5.1 Efficacy Measurements

### Timed 25 Foot Walk Test:

The T25FW test is a measure of ambulatory function that provides quantitative data and is used widely in the MS population. If needed, the subject used their assistive device (cane, crutches, or wheeled walker) that was used on a regular basis while walking as quickly as he or she can from one end to the other on a 25-foot (7.62 meters) course. Non-wheeled walkers were generally not to be used. This test was performed in a clearly marked course, which was used for every T25FW test.

For the test the individual was asked to line up with their toes of their shoes on the marked spot on the starting line and when their first foot crossed the starting line, timing using a stopwatch began. Timing concluded when the first foot crossed the 25 foot line. Time was recorded in seconds and, if appropriate, rounded in seconds to the nearest tenth of a second. There was a maximum 5 minute rest between trials and then the subject would walk the same distance again.

The T25FW was performed once at Visits 1, 2, 3, 4, and 5.

Subjects could volunteer to be video recorded during the T25FW evaluation. However, video recording was not a requirement for study participation. Video recording of the T25FW was conducted at Visits 2, 3, and 4. If a subject volunteered for video recording, an additional consent form was obtained. The video footage was captured using a fixed camera mount to ensure consistent perspective and angle. The raw video footage was edited to ensure that the subjects are unidentifiable. This was accomplished by using a visual effects software program, Adobe® After Effects®, to track the face or any personally distinguishing features of the subject and obscure it for the entire length of the segment. The final videos included title graphics for each segment including the subject number, the phase of the study, and the elapsed time for the T25FW. These video recordings were viewed by the Evaluator to provide a qualitative assessment of change between off-drug and on-drug walks.

### Two Minute Walk Test:

The subjects walked without assistance for 2 minutes and the distance was measured and timed using a stop watch. The distance covered by walking for 2 minutes was measured in meters. Verbal confirmation of start and stop was given. An assistive device could be used, but no physical assistance was allowed. If an assistive device was used, the same device was used for all tests.

The 2MWT was performed once at Visits 1, 2, 3, 4, and 5.

### Berg Balance Scale

The BBS is a 14-item scale specifically designed to measure balance in the elderly population in a clinical setting (Berg et al., 1989, Physiother Canada 41:304-311; Tinetti, 1986, J Amer Geriatrics Society 34(2): 119-126), but has been used in populations with stroke and traumatic brain injury to assess postural balance (Berg et al., 1989; Berg et al., 1995, Scand J Rehab Med 27(1):27-36; Newstead et al., 2005, J Neurol Phys Ther 29(1):18-23. The BBS evaluates subjects ability to sit, stand, reach, maintain single-leg stance, and turn. The scoring is rated from 0 (cannot perform task) to 4 (normal performance of task). This test has reported to give a good prediction of falling with good validity and reliability (Berg et al., 1992, Arch Phys Med Rehab 73(11):1073-1080; Berg et al., 1992, Can J Pub Health 83 (suppl 2):S7-11; Bogle Thorbahn, 1996, Phys Ther 76(6):576-583; Creel et al., 2001, Phys Ther 81(2):789-798; Shumway-Cook et al., 1997, Phys Ther 77(8):812-819). The maximum possible score is 56 and the lowest 0; a score of 45 or below indicates an increased risk of falling (Riddle & Startford, 1999, Phys Ther 79(10):939-948.

The BBS was performed once at Visits 1, 2, 3, 4, and 5.

### NeuroCom SMART Balance Master®:

The SMART Balance Master is a device that provides objective assessments and retraining of the sensory and voluntary motor control of balance with visual feedback on either a stable or unstable support surface and in a stable or dynamic visual environment (see webpage of Balance and Mobility Services/NeuroCom®, a division of Natus®; NeuroCom Products for Balance and Mobility; Balance Master® Family; SMART Balance Master®). The system utilizes a dynamic 18 inches by 8 inches dual forceplate with rotation capabilities to measure the vertical forces exerted by the subject's feet and a moveable visual surround.

Balance and gait variables measured using the NeuroCom were:
Unilateral Stance (US): The US quantifies postural sway velocity with the subject standing quietly on one foot (either right or left) on the forceplate, with eyes open and eyes closed. The relative absence of sway is "stability," when the instruction is to "hold still," greater sway indicates less stability, while less sway indicates greater stability. With verbal direction from the test instructor, the subject either lifts their right or left leg and closes or opens their eyes and will try and stand as steadily as possible for 10 seconds. The test consists of 4 conditions, each consisting of 3 trials, usually is the following order:
   - Left - eyes open
   - Right - eyes open
   - Left - eyes closed
   - Right - eyes closed
Walk Across (WA): The WA quantifies characteristics of gait as the subject walks across the length of the forceplate. The test characterizes steady state gait by having the subject begin well behind and continuing beyond the forceplate. Because of the length of the forceplate, the test may not be appropriate for highly fit individuals whose stride lengths are greater than 5 feet (152 cm). The following variables were measured in this test:
   - Step Width - lateral distance in centimeters between the left and right foot on successive steps
   - Step Length - longitudinal distance in centimeters between successive heel strikes on successive steps
   - Speed - velocity in centimeters per second of the forward progression
   - Step Length Symmetry - comparison of right and left step length, expressed as a percentage of the total stride length (right and left length)
Tandem Walk (TW): The TW quantifies characteristics of gait as the subject walks heel to toe from one end of the forceplate to the other. Measured variables were step width, speed, and endpoint sway velocity.
Step/Quick Turn (SQT): The SQT quantifies turn performance as the individual takes 2 steps forward and then quickly turns 180° and then returns to the starting point. Variables measured were the time to execute the turn and the sway velocity during the turn execution (turn time and turn sway).
Sensory Organization Test (SOT): SOT scores were based on the assumption that a normal individual could exhibit anterior to posterior sway over a total range of approximately 12.5° without losing balance. The equilibrium score for each trial was calculated by comparing the angular difference between the subjects' maximum anterior to posterior center of gravity (COG) displacements to this theoretical maximum displacement. The result was expressed as an inverse percentage between 0 and 100. Scores approaching 0 indicate sway amplitudes approaching the limits of stability with a value of 100 indicating perfect stability. A score of 0 indicates that the subject fell on that trial. If a subject's voluntary limits or "cone of stability" was restricted, even small degrees of sway may exceed the available limits of stability and result in a loss of balance or fall during testing. Because of this fact, SOT should always be performed and interpreted in conjunction with motor performance testing. The raw data from which the equilibrium score was calculated also contains significant information about the amplitude, frequency, direction, and regularity of subject sway and should be reviewed accordingly. Variables measured were fixed surface eyes open, fixed surface eyes closed, walls moving eyes open, surface moving eyes open, surface moving eyes closed, and surface and walls moving eyes open.
Adaptation Test (ADT): The ADT assesses a subject's ability to minimize sway when exposed to surface irregularities and unexpected changes in support surface inclination. Sequences of platform rotations in the toes-up or toes-down direction elicit automatic motor responses. For each platform rotation trial, a sway energy score quantifies the magnitude of the force response required to overcome induced postural instability. Unanticipated toes-up or toes-down rotations elicit automatic responses, which tend to destabilize the subject's balance. During the first (unexpected) trials, the initial disruptive responses were corrected by secondary responses in the opposing muscles. With each subsequent trial, initial reactions were attenuated and secondary responses strengthened to reduce overall sway. Variables measured were averaged raw sway and center of force during rotational disturbances.
Limits of Stability Test (LOS): The LOS quantifies the maximum distance a person can intentionally displace their COG, i.e., lean their body in a given direction without losing balance, stepping, or reaching for assistance. For each of 8 transitions, the subject maintained their COG centered over the base of support as indicated by a cursor display of the COG position relative to a center target. On command, the subject moved the COG cursor as quickly and accurately as possible toward a second target located on the LOS perimeter (100% of theoretical limits of stability) and then held a position as close to the target as possible. The subject was allowed up to 8 seconds to complete each trial. Reaction time is the time in seconds between the command to move and the subject's first movement. Movement velocity is the average speed of COG movement in degrees per second. Endpoint excursion is the distance of the first movement toward the designated target, expressed as a percentage of maximum LOS distance. The endpoint is considered to be the point at which the initial movement toward the target ceases. Maximum excursion is the maximum distance achieved during the trial. Directional control is a comparison of the amount of movement in the intended direction (toward the target) to the amount of extraneous movement (away from the target).

The SMART Balance Master tests were performed once at Visits 1, 2, 3, 4, and 5.

### 6.3.3.5.2 Safety Measurements

Adverse events (AEs) were assessed at every visit. Adverse events, including serious adverse events (SAEs) were recorded in the CRFs. All AEs were monitored until they resolved or until the subject's completed or discontinued the study, whichever came first.

Blood pressure, pulse, respiratory rate, and temperature were collected at every visit. Height and weight were collected only at the screening visit. Before vital sign measurements, the subject was resting in a supine position for 5 minutes.

A full physical examination was done at screening and then a brief physical exam was performed for the remainder of the visits of the study.

Concomitant medications and therapies were recorded at every visit.

### 6.3.3.5.3 Appropriateness of Measurements

The NeuroCom system, which was used to measure the efficacy assessments (gait and balance), is a well documented tool (see webpage of Balance and Mobility Services/NeuroCom®, a division of Natus®; NeuroCom Products for Balance and Mobility; Balance Master® Family; SMART Balance Master®). Gait and balance are the result of complex mechanisms that integrate sensory input, central sensory integration, and an appropriate motor response. Abnormalities in any of these functions negatively impact mobility and safety. The NeuroCom system allows the objective identification of impairment of specific systems and /or functional limitations. Different assessment protocols can be used. For this study, protocols that more closely represented regular daily activities were selected. Consequently, evaluations representing steady state ambulation, direction change, adaptation to surface irregularities, reaching, and integration of sensory inputs were chosen.

### 6.3.3.5.4 Primary Efficacy Variable(s)

The primary efficacy was determined based on two co-primary efficacy variables: the composite score for gait and the composite score for balance while taking dalfampridine-ER compared to withdrawal. Gait and balance variables were measured using the NeuroCom and included the following tests: WA, TW, SQT, SOT, ADT, and LOS.

### 6.3.3.5.5 Drug Concentration Measurements

Blood was drawn from the subjects at Visits 2, 3, 4, and 5 for analysis of dalfampridine concentration. Analysis was performed by Covance Analytical Laboratories (Wisconsin) in accordance with Covance SOPs and the validated method. Samples were originally analyzed singly. At a minimum, each batch included a calibration curve, a matrix blank, a control zero (matrix blank containing internal standard), a reagent blank, and duplicate quality control (QC) samples at three concentrations within the calibration range. The samples were interspersed with calibration standards and QC samples within the batch. Dilution QC samples were also included in batches where samples were diluted prior to analysis. Study samples were re-assayed if values exceeded the curve range. All the results were reported according to Covance SOPs.

### 6.3.3.6. STATISTICAL METHODS PLANNED IN THE PROTOCOL AND DETERMINATION OF SAMPLE SIZE

### 6.3.3.6.1 Statistical and Analytical Plans

All computations were performed using SAS® Version 9 or higher. Statistically significant treatment differences were declared if the resulting p-value was less than 0.05. All tests were two-sided. No interim analysis was planned or performed.

### 6.3.3.6.2 Determination of Sample Size

The sample size of 20 subjects was selected to provide adequate parameter estimates to aid in the design of future studies.

### 6.3.3.6.3 Analysis Populations

The following definitions were used to identify different sets of subjects:
- The Full Analysis Population (FAP) was the basis of the primary efficacy analysis and included all subjects who had at least 1 baseline (Visit 1 or Visit 2) and 1 post-baseline (Visit 3 or Visit 4) assessment in both on-drug and off-drug periods.
- The Safety Population included all subjects who were enrolled into the study at Visit 1 since all subjects entering the study were taking dalfampridine-ER.

### 6.3.3.6.4 Demographic and Background Variables

Demographic, disease background, and baseline data were summarized using descriptive statistics. Other baseline variables included the following assessments at screening: medical history, concomitant medications and therapies, physical examination, vital signs including height, and weight. For continuous variables, descriptive statistics included the mean, standard error of the mean, standard deviation, median, and minimum and maximum values. For categorical variables, descriptive statistics included the number and percentage of subjects falling in each category.

### 6.3.3.6.5 Analysis of Efficacy

### Primary Efficacy Endpoints:

The two primary endpoints for this study were tested in a step down procedure and were based on comparing on-drug versus off-drug periods. The first endpoint was a composite score assessing the subject's gait. The gait composite score was predicated on composite scores from three of the tests from the NeuroCom system: WA, TW, and SQT. The second endpoint was a composite score assessing the subject's balance. The balance composite score was predicated on composite scores from three different tests from the NeuroCom system: SOT, ADT, and LOS. Both the gait and balance composite scores are endpoints that have not been previously documented in literature. These endpoints were developed to create single measurements for assessing gait and balance.

The overall gait and balance composite scores were calculated based on Z-scores from their respective NeuroCom system tests. The gait Z-score was computed by taking the average of its three component Z-scores. The balance Z-score was computed as a weighted average of the three component Z-scores (50% for SOT, 20% for ADT, and 30% for LOS). The gait and balance Z-scores were then transformed into percentile scores (from 0 to 100) using the standard normal distribution.

### Secondary Efficacy Variables:

There were a number of secondary efficacy variables examined in this study:
Individual NeuroCom Subcomponents Scores to Measure Gait and Balance. The following individual subcomponents of each test from the NeuroCom system were analyzed separately in a similar manner to the primary and secondary efficacy variables: WA, US, TW, SQT, SOT, ADT, and LOS.
Other Measures of Gait and Balance. Three additional measures of gait and balance were analyzed in a similar manner to the primary and secondary efficacy variables: BBS (balance), 2MWT (gait), and T25FW (gait).
Additional Standardized NeuroCom Scores. Three additional efficacy variables were created on an intra-subject basis, predicated on standardized data from the NeuroCom system. All variables (WA, US, TW, SQT, SOT, ADT. and LOS) were eligible to be included in the computation of the following three secondary efficacy variables:
   1. A composite score from the standardized data of all seven NeuroCom tests ("sensitivity").
   2. The test that was closest to normal at baseline ("best").
   3. The test that was most deviant from normal at baseline ("worst").

### Methods of Analysis:

Each efficacy variable (NeuroCom composite and individual test scores; BBS, 2MWT, and T25FW scores) was subjected to 4 types of analysis:
- Descriptive statistics for each time point (Visit 1/Day -7, Visit 2/Day 1, Visit 3/Day 5, Visit 4/Day 11, Visit 5/Day 15), including sample size (n), mean, standard error, standard deviation (SD), median, minimum, and maximum values, and the 95% confidence interval. Descriptive statistics were also generated separately for the treatment comparison (dalfampridine-ER on-drug in Periods 1 and 3 versus dalfampridine-ER withdrawn in Period 2) and the period comparisons (Period 1 versus Period 2 [withdrawal], Period 2 versus Period 3 [reinitiation], and Period 1 versus Period 3 [both on-drug periods]).
- Mixed-model analysis of variance (ANOVA) by use of dalfampridine-ER (dalfampridine-ER on-drug in Periods 1 and 3 versus dalfampridine-ER withdrawn in Period 2); this was the primary statistical analysis. For each category and the difference between dalfampridine-ER on-drug and dalfampridine-ER withdrawn, the summary of results includes the least squares mean, standard error, 95% confidence interval for the least squares mean, and the p-value comparing the difference in least squares means.
- Mixed-model ANOVA by study period. For each period (Period 1 - dalfampridine-ER, Period 2 - dalfampridine-ER withdrawn, and Period 3 - dalfampridine-ER resumed) and the difference between each period (Period 1 - Period 2, Period 1 - Period 3, and Period 2 - Period 3), the summary of results includes the least squares mean, standard error, 95% confidence interval for the least squares mean, and the p-values comparing the differences in least squares means. In addition, the summary includes p-values and confidence intervals for the difference in least squares mean adjusted for the multiple comparisons.
- Paired t-tests comparing baseline (average of Visits 1 and 2) with Visits 4 and 5 and comparing Visit 4 with Visit 5. P-values from a paired t-tests are displayed, as well as sample size (n), mean, standard error, standard deviation (SD), median, minimum, and maximum values, and the 95% confidence interval.

A p-value less than 0.05 was judged as providing evidence of efficacy in this study population. All efficacy analyses were performed on the FAP. Adjustments for multiplicity are discussed in Section 6.3.5.4.4, "Multiple Comparisons or Multiplicity." The adjustments included a step-down procedure for testing the 2 co-primary efficacy variables. If the p-value for overall gait from Periods 1 and 3 versus Period 2 (i.e., the overall treatment comparison) was less than 0.05, then the overall score for balance could be tested in the same manner. However, if the p-value for the overall score for gait was not significant at the 0.05 level, then the testing procedure would stop.

Additional analyses of efficacy variables included the following:
- The correlation between the individual subscales of the NeuroCom system variables (overall gait, balance, and individual subscales) and the other clinical efficacy measures (BBS, 2MWT, and T25FW) was assessed using Pearson's correlation coefficient, if the assumption of linearity was met and a nonparametric method (e.g., Spearman's) if the assumption was not met. P-values, testing whether the correlation significantly differs from 0, are displayed for informational purposes; there was no adjustment for multiplicity.
- Analysis of evaluator's qualitative assessment of change, summarized as the number and percentage of subjects in each change category for the following comparisons: Visit 2 to Visit 3, Visit 3 to Visit 4, and Visit 2 to Visit 4.

### 6.3.3.6.6 Safety Assessment

Safety and tolerability were assessed primarily by reviewing AE data and changes from baseline in vital signs.

Adverse Events. All AEs were coded using Version 14.0 of the Medical Dictionary of Regulatory Activities (MedDRA), and were classified by MedDRA system organ class (SOC) and preferred term. All summaries of AEs were based on treatment-emergent AEs (TEAE). TEAE was defined as an AE with date of onset (or worsening) that occurred on or after the Screening Visit (Visit 1) through 30 days after the last dose of investigational product taken in Period 3. A treatment-emergent serious adverse event (TESAE) was defined as a SAE with date of onset (or worsening) that occurred on or after the Screening Visit (Visit 1) through 30 days after the last dose of investigational product taken in Period 3.

Other Safety and Tolerability Variables. Other safety and tolerability variables including vital signs evaluations were summarized by visit. If applicable, descriptive statistics were calculated for changes from baseline. Baseline vital signs were defined as the last assessment prior to withdrawal of study medication. If a subject did not have vital signs collected at Visit 2, the subject's baseline measurements were imputed using the vital signs collected at Visit 1. Clinically significant changes were counted and tabulated.

Adverse events and other safety variables were summarized by treatment (dalfampridine-ER or off-drug) at the time of the event.

### 6.3.4 STUDY PATIENTS

### 6.3.4.1. DISPOSITION OF PATIENTS

A total of 20 subjects were enrolled into the study at 1 center. All 20 subjects completed the study. The disposition of the study subjects is presented in Table 13.

**Table 13: Summary of Subject Disposition (All Subjects)**

| | **All Subjects** |
|---|---|
| Subjects screened | 21 |
| Subjects enrolled | 20 (95.2%) |
| Subjects that completed Period 1 | 20 (100.0%) |
| Subjects that completed Period 2 | 20 (100.0%) |
| Subjects that completed the study | 20 (100.0%) |
| Subjects that discontinued from the study | 0 |

### 6.3.4.2. PROTOCOL DEVIATIONS

All protocol deviations were reviewed and none were determined to be severe enough to warrant a PPP analysis (i.e., analysis of a sub-population of the FAP consisting of all FAP subjects who completed all visits with no major protocol violations). All subjects received an eligibility exemption from the body mass inclusion criteria. Exemptions were given to subjects who had some of the study visits fall outside of the protocol-defined windows due to scheduling and transportation conflicts. Protocol deviations were granted to five subjects who were unable to perform the US or TW assessments, and to one subject who missed the SQT at one visit.

### 6.3.5 EFFICACY EVALUATION

### 6.3.5.1. DATA SETS ANALYZED

The analysis populations in this study included the Safety Population and the FAP. The Safety Population included all subjects who were enrolled in the study at Visit 1. The FAP included all subjects who had at least 1 baseline (Visit 1 or Visit 2) and 1 post-baseline (Visit 3 or Visit 4) assessment in both on-drug and off-drug periods.

The data sets that were analyzed are summarized in Table 14. All 20 enrolled subjects were included in all analyses.

**Table 14: Data Sets Analyzed (All Subjects)**

| | **All Subjects** |
|---|---|
| Subjects Enrolled | 20 |
| Safety Population | 20 (100.0%) |
| Full Analysis Population | 20 (100.0%) |

### 6.3.5.2. DEMOGRAPHIC AND OTHER BASELINE CHARACTERISTICS

The baseline demographic characteristics and disease history including dalfampridine-ER 10 mg use prior to study enrollment of the study subjects are presented in Table 15 and Table 16, respectively. Sixty percent of the subjects were female, 90% were white, and the mean age was 53 years. On average, subjects were taking dalfampridine-ER for approximately 1 year prior to study enrollment.

**Table 15: Baseline Demographic Characteristics (Safety Population)**

| | **Safety Population (N=20)** |
|---|---|
| Gender - n (%) | |
| Female | 12 (60.0) |
| Male | 8 (40.0) |
| Age (years) | |
| n | 20 |
| Mean | 53.1 |
| Standard Deviation (Minimum, Maximum) | 10.96 (20, 65) |
| Race - n (%) | |
| American Indian or Alaska Native | 1 (5.0) |
| Black or African American | 1 (5.0) |
| White | 18(90.0) |
| Ethnicity - n (%) | |
| Not Hispanic or Latino | 20 (100.0) |

**Table 16: Disease History and Dalfampridine-ER 10 mg Use (Safety Population)**

| | **Safety Population (N=20)** |
|---|---|
| Multiple Sclerosis diagnosis type - n (%) | |
| Primary-Progressive | 2 (10.0) |
| Progressive-Relapsing | 2 (10.0) |
| Relapsing-Remitting | 13(65.0) |
| Secondary-Progressive | 3 (15.0) |
| Duration of disease (years) | |
| n | 20 |
| Mean | 11.3 |
| Standard Deviation (Minimum, Maximum) | 8.43 (3, 29) |
| Time on dalfampridine-ER 10 mg prior to study enrollment (days) | |
| n | 20 |
| Mean | 315.3 |
| Standard Deviation (Minimum, Maximum) | 270.45 (14, 685) |

The most common medical history events are summarized in Table 17.

**Table 17: Most Common Medical History Events (Safety Population)**

| | |
|---|---|
| **Preferred Term** | **Safety Population (N=20)** |
| Number (%) of subjects with any medical history | 19 (95.0) |
| Hypertension | 7 (35.0) |
| Depression | 6 (30.0) |
| Neurogenic bladder | 5 (25.0) |
| Hyperlipidaemia | 4 (20.0) |
| Hysterectomy | 4 (20.0) |
| Tonsillectomy | 4 (20.0) |
| Appendectomy | 3 (15.0) |
| Cholecystectomy | 3 (15.0) |
| Gastrooesophageal reflux disease | 3 (15.0) |
| Hypercholesterolaemia | 3 (15.0) |
| Knee arthroplasty | 3 (15.0) |
| Menopause | 3 (15.0) |
| Migraine | 3 (15.0) |
| Restless legs syndrome | 3 (15.0) |
| Sleep apnoea syndrome | 3 (15.0) |

| | |
|---|---|
| Note: Medical history events that occurred in at least three subjects were considered to be most common. | |

In addition to documenting a baseline amount of physical activity by self-report, there is a health contribution implication with moderate and strenuous activities. The baseline physical activity levels of the study subjects were determined by using the 4-item Godin Leisure Time Exercise Questionnaire and results are summarized in Table 18. On average, subjects participated in mild exercise 4 times a week, moderate exercise twice a week, and strenuous exercise 1 time per week. Overall, all subjects participated in some type of regular exercise regimen during a typical week, although this was primarily a mild form of exercise. Per Table 18, the mean weekly leisure activity score of 20 is reflective of a moderately active group of patients getting some benefits, but not substantial benefits, from their weekly leisure activity.

**Table 18: Summary of Physical Activity Level - Total Score Based on Moderate and Severe (Safety Population)**

| | **Safety Population (N=20)** |
|---|---|
| Mild exercise (Times Per Week) | |
| Mean | 4.3 |
| Standard Deviation (Minimum, Maximum) | 3.31 (0,14) |
| Moderate exercise (Times Per Week) | |
| Mean | 2.1 |
| Standard Deviation (Minimum, Maximum) | 2.77 (0, 9) |
| Strenuous exercise (Times Per Week) | |
| Mean | 1.1 |
| Standard Deviation (Minimum, Maximum) | 2.38 (0, 7) |
| Weekly leisure activity score* | |
| Mean | 20.4 |
| Standard Deviation (Minimum, Maximum) | 3 0.42 (0, 98) |

| | |
|---|---|
| *Weekly leisure activity score= (9 x Strenuous) + (5 x Moderate). Gaston, 2011, Health and Fitness Journal Canada 4(1):18-22 | |

The most common concomitant medications used by the study subjects are summarized in Table 19. It should be noted that most of these medications started prior to study start and continued while on study.

**Table 19: Most Common Concomitant Medications (Safety Population)**

| | **Safety Population (N=20)** |
|---|---|
| Number (%) of subjects who took any prior medication | 20 (100.0) |
| Glatiramer acetate | 8 (40.0) |
| Clonazepam | 6 (30.0) |
| Baclofen | 5 (25.0) |
| Modafinil | 4 (20.0) |
| Tizanidine | 4 (20.0) |
| Betaseron | 3 (15.0) |
| Duloxetine hydrochloride | 3 (15.0) |
| Ergocalciferol | 3 (15.0) |
| Interferon beta-1a | 3 (15.0) |
| Ropinirole | 3 (15.0) |
| Simvastatin | 3 (15.0) |

| | |
|---|---|
| Note: Concomitant medications that were used by at least three subjects were considered to be the most common. | |

### 6.3.5.3. MEASUREMENTS OF TREATMENT COMPLIANCE

A summary of the dalfampridine-ER plasma concentrations is presented by visit in Table 20. During Visits 3 and 4, no subjects took dalfampridine-ER; all subjects were taking dalfampridine-ER during Visits 2 and 5 in accordance to the protocol.

**Table 20: Summary of Dalfampridine-ER Plasma Concentrations (ng/mL) by Visit (FAP)**

| | **FAP (N=20)** |
|---|---|
| Visit 2/Day 1 (D-ER) | |
| Mean | 26.68 |
| Standard Deviation (Minimum, Maximum) | 13.887 (1.6, 54.4) |
| Visit 3/Day 5 (D-ER withdrawn) | |
| Mean | 0.00 |
| Standard Deviation (Minimum, Maximum) | 0.000 (0.0, 0.0) |
| Visit 4/Day 11 (D-ER withdrawn) | |
| Mean | 0.00 |
| Standard Deviation (Minimum, Maximum) | 0.000 (0.0, 0.0) |
| Visit 5/Day 15 (D-ER) | |
| Mean | 30.93 |
| Standard Deviation (Minimum, Maximum) | 12.322 (13.2, 52.8) |

### 6.3.5.4. EFFICACY RESULTS AND TABULATIONS OF INDIVIDUAL SUBJECT DATA

### 6.3.5.4.1 Primary Efficacy Endpoints

Results for the two primary efficacy endpoints are summarized by visit in Figure 3 and statistically in Table 21. Tables 21 through 32 provide a high level summary of the results for each efficacy variable. The purpose of these tables is to indicate which variables showed an efficacy signal for the treatment, period, and visit comparisons.

Statistically, subjects did significantly better on overall gait (p=0.015) while being treated with dalfampridine-ER (Period 1 ("P1") and Period 3 ("P3")) than when untreated (Period 2 ("P2")). Results for the other endpoint, overall balance, were not statistically significant with respect to treatment (p=0.434).

Comparing the individual study periods, overall gait numerically showed deterioration in the subject's gait during dalfampridine-ER withdrawal (Period 1 [on-drug] versus Period 2 [off-drug]), but this difference was not statistically significant. When treatment resumed, there was a statistically significant difference between Period 3 (on-drug) and Period 2 (off-drug) for overall gait. For overall balance, there did not appear to be deterioration in the subject's balance during dalfampridine-ER withdrawal. In contrast, when treatment resumed, there was a statistically significant improvement in balance.

**Table 21: Summary of On-Drug versus Off-Drug Results - Overall Gait and Overall Balance (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Overall Gait | Favors D-ER | ✔ | ✔ | ✔ | ✔ | ✔ |
| | Statistically significant | ✔ | | ✔ | | |
| Overall Balance | Favors D-ER | | | ✔ | | ✔ |
| | Statistically significant | | | ✔ | | |

| | | | | | | |
|---|---|---|---|---|---|---|
| Abbreviations: on=on-drug; off=off-drug; P = Period; V = Visit; vs = versus. ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. | | | | | | |

### 6.3.5.4.2 Secondary Efficacy Variables

Each individual NeuroCom variable in its original scale is summarized by test in this section.

### NeuroCom Gait Variables

### Walk Across ("WA") Test

Results for the WA variables are summarized by visit in Figure 4 and statistically in Table 22.

Statistically, subjects did significantly better on step width, step length, and walking speed measurements while being treated with dalfampridine-ER than when untreated. It should be noted, however, that the conclusions for step width are difficult to interpret in that subjects, on-average, did not have the expected decreases in step width with the re-initiation of dalfampridine-ER in Period 3 (see Period 3 versus Period 2 result in Table 22). There were no statistically notable results for step length symmetry.

**Table 22: Summary of On-Drug versus Off-Drug Results - NeuroCom Walk Across (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Step Width (cm) | Favors D-ER | ✔ | ✔ | | ✔ | |
| | Statistically significant | ✔ | | | | |
| Step Length (cm) | Favors D-ER | ✔ | ✔ | ✔ | ✔ | ✔ |
| | Statistically significant | ✔ | ✔ | ✔ | ✔ | ✔ |
| Speed (cm/sec) | Favors D-ER | ✔ | ✔ | ✔ | ✔ | ✔ |
| | Statistically significant | ✔ | ✔ | ✔ | ✔ | ✔ |
| Step Length Symmetry (%) | Favors D-ER | ✔ | ✔ | ✔ | | |
| | Statistically significant | | | | | |

| | | | | | | |
|---|---|---|---|---|---|---|
| ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. | | | | | | |

### Unilateral Stance ("US") Test

Results for the US variables are summarized by visit in Figure 5 and statistically in Table 23. The US test was not included in the overall gait or overall balance composite scores because, although it was a sensitive measure of instability, it was non-specific and it was difficult for the majority of the subjects to perform. Note that only 17 subjects were able to complete at least one of the on- and off-drug measurements.

Statistically, subjects did significantly better overall on left-eyes open sway velocity while being treated with dalfampridine-ER than when untreated. It should be noted, however, that the conclusions for left-eyes open sway velocity were complicated in that on-average, subjects did not have the expected decreases in left-eyes open sway velocity with the re-initiation of dalfampridine-ER in Period 3 (see Period 3 versus Period 2 result in Table 23). All other results for the US variables either were not statistically notable or were non-estimable.

**Table 23: Summary of On-Drug versus Off-Drug Results - NeuroCom Unilateral Stance (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Left-Eyes Open Sway Velocity (degrees/second) | Favors D-ER | ✔ | ✔ | | ✔ | |
| | Statistically significant | ✔ | | | | |
| Left-Eyes Closed Sway Velocity (degrees/second) | Favors D-ER | Non-est | Non-est | Non-est | Non-est | Non-est |
| | Statistically significant | Non-est | Non-est | Non-est | Non-est | Non-est |
| Right-Eyes Open Sway Velocity (degrees/second) | Favors D-ER | Non-est | Non-est | Non-est | | |
| | Statistically significant | Non-est | Non-est | Non-est | | |
| Right-Eyes Closed Sway Velocity (degrees/second) | Favors D-ER | Non-est | Non-est | Non-est | | |
| | Statistically significant | Non-est | Non-est | Non-est | | |

| | | | | | | |
|---|---|---|---|---|---|---|
| Abbreviations: Non-est = Non-estimable ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note 1: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. Note 2: Several comparisons were non-estimable (could not be calculated) because of the lack of variability or the amount of missing values did not allow the model to converge to estimates. | | | | | | |

### Tandem Walk ("TW") Test

Results for the TW variables are summarized by visit in Figure 6 and statistically in Table 24.

There were no statistically significant results for the treatment comparisons in any of the TW variables. However, there was a statistically significant difference in walking speed during Period 3 (on-drug) versus Period 2 (off-drug) with subjects walking faster in Period 3. It should be noted, however, that the overall conclusions for walking speed (comparing all of the on-drug visits to all of the off-drug visits) were difficult to interpret in that subjects, on-average, did not have the expected decreases in walking speed at off-drug Visit 4 (Figure 6). There were no statistically notable results for the remaining TW variables. However, there was a positive trend for step-width while on-drug.

**Table 24: Summary of On-Drug versus Off-Drug Results - NeuroCom Tandem Walk Variables (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline (on) vs V4 (off)** | **V5 (on) vs V4 (off)** |
| Step Width (cm) | Favors D-ER | ✔ | ✔ | ✔ | ✔ | |
| | Statistically significant | | | | | |
| Speed (cm/sec) | Favors D-ER | ✔ | ✔ | ✔ | | ✔ |
| | Statistically significant | | | ✔ | | |
| End Sway (deg/sec) | Favors D-ER | | | | | |
| | Statistically significant | | | | | |

| | | | | | | |
|---|---|---|---|---|---|---|
| ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. | | | | | | |

### Step/Quick Turn ("SQT") Test

Results for the SQT variables are summarized by visit in Figure 7 and statistically in Table 25.

Both the left-turn sway and the right-turn sway results were statistically significant when subjects were treated with dalfampridine-ER compared to when they were untreated. There were no statistically notable results for the remaining SQT variables (i.e., left turn time and right turn time).

**Table 25: Summary of On-Drug versus Off-Drug Results - NeuroCom Step/Quick Turn (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Left Turn Time (sec) | Favors D-ER | ✔ | ✔ | ✔ | | ✔ |
| | Statistically significant | | | | | |
| Right Turn Time (sec) | Favors D-ER | ✔ | ✔ | ✔ | | ✔ |
| | Statistically significant | | | | | |
| Left Turn Sway (deg) | Favors D-ER | ✔ | ✔ | ✔ | | ✔ |
| | Statistically significant | ✔ | | | | |
| Right turn Sway (deg) | Favors D-ER | ✔ | ✔ | ✔ | ✔ | ✔ |
| | Statistically significant | ✔ | | | | |

| | | | | | | |
|---|---|---|---|---|---|---|
| ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note: On-drug (dalfampridine-ER) = Periods 1 (Visits 3 and 2) and Period 3 (Visit 5); Off-drug (withdrawal) = Period 2 (Visits 3 and 4): Baseline = Visits 1 and 2. | | | | | | |

### NeuroCom Balance Variables

### Sensory Organization Test ("SOT")

Results for the SOT equilibrium composite score variable are summarized by visit in Figure 8 and statistically in Table 26.

There was not a statistically significant difference equilibrium composite score when subjects were treated with dalfampridine-ER versus when they were untreated. Although this finding was not statistically significant, subjects seemed to perform better over time. In this study, when the subjects re-initiated dalfampridine-ER use during Period 3, there was a statistically significant difference (approximately 8 points) with Period 2 (off-drug) with subjects having a higher equilibrium composite score, suggesting a possible drug effect beyond a learning effect. In contrast, the subjects had numerically lower scores during the first on-drug period (Period 1) than they did when untreated.

**Table 26: Summary of On-Drug versus Off-Drug Results - NeuroCom Sensory Organization Test (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Equilibrium Composite Score | Favors D-ER | ✔ | | ✔ | | ✔ |
| | Statistically significant | | | ✔ | | |

| | | | | | | |
|---|---|---|---|---|---|---|
| ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. | | | | | | |

### Adaptation Test ("ADT")

Results for the ADT variables are summarized by visit in Figure 9 and statistically in Table 27.

There was a statistically significant difference in the toes up score when subjects were treated with dalfampridine-ER versus when they were untreated; however, this difference was in the unexpected direction with average scores being significantly higher while on-drug. When comparing the individual periods for the toes up score, the on-drug Period 1 scores were higher than the off-drug Period 2 scores. This result was statistically significant. The on-drug Period 3 scores were numerically, but not significantly, higher than the off-drug Period 2 scores. There were no statistically significant findings for the toes down score.

**Table 27: Summary of On-Drug versus Off-Drug Results - NeuroCom Adaptation Test (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Toes Up Score | Favors D-ER | | | | | |
| | Statistically significant | ✔ | ✔ | | ✔ | ✔ |
| Toes Down Score | Favors D-ER | ✔ | ✔ | | | |
| | Statistically significant | | | | | |

| | | | | | | |
|---|---|---|---|---|---|---|
| ¹Baseline is the average of Visits 1 and 2. ²Favors D-ER A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note 1: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. Note 2: Several comparisons were non-estimable (could not be calculated) because of the lack of variability or the amount of missing values did not allow the model to converge to estimates. | | | | | | |

### Limits of Stability Test ("LOS")

Results for the LOS variables are summarized by visit in Figure 10 and statistically in Table 28.

There was a statistically significant increase in the movement velocity composite score when subjects were treated with dalfampridine-ER versus when they were untreated. When comparing Period 3 (on-drug) to Period 2 (off-drug), there was also a statistically significant increase in the movement velocity composite score. However, this result is confounded by the Period 1 (on-drug) score, which was numerically lower than Period 2 (off-drug). All other results for the LOS were either not statistically significant or non-estimable.

**Table 28: Summary of On-Drug versus Off-Drug Results - NeuroCom Limits of Stability (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Reaction Time (sec) | Favors D-ER | ✔ | | ✔ | | ✔ |
| | Statistically significant | | | | | |
| Movement Velocity (deg/sec) | Favors D-ER | ✔ | | ✔ | | ✔ |
| | Statistically significant | ✔ | | ✔ | | |
| Endpoint Excursion (%) | Favors D-ER | Non-est | Non-est | Non-est | | ✔ |
| | Statistically significant | Non-est | Non-est | Non-est | | |
| Maximum Excursion (%) | Favors D-ER | | | ✔ | | ✔ |
| | Statistically significant | | | | | |
| Directional Control (%) | Favors D-ER | Non-est | Non-est | Non-est | | ✔ |
| | Statistically significant | Non-est | Non-est | Non-est | | |

| | | | | | | |
|---|---|---|---|---|---|---|
| Abbreviations: Non-est = Non-estimable. ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note 1: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. Note 2: Several comparisons were non-estimable (could not be calculated) because of the lack of variability or the amount of missing values did not allow the model to converge to estimates. | | | | | | |

### Other Measures of Gait and Balance

In addition to the NeuroCom variables and the composite scores derived from them, three additional efficacy variables were collected: BBS (balance), 2MWT (gait), and 125FW (gait).

### Berg Balance Scale

Results for the BBS are summarized by visit in Figure 11 and statistically in Table 29.

Statistically subjects did significantly better on the BBS while being treated with dalfampridine-ER compared to when they were untreated. When comparing the total scores by period, subjects also showed a statistically significant improvement in total score when on-drug. Subjects showed a statistically significant improvement in total score in Period 1 (on-drug) compared to Period 2 (off-drug) and in Period 3 (on-drug) compared to Period 2.

**Table 29: Summary of On-Drug versus Off-Drug Results - Berg Balance Scale (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Total Score | Favors D-ER | ✔ | ✔ | ✔ | ✔ | ✔ |
| | Statistically significant | ✔ | ✔ | ✔ | | |

| | | | | | | |
|---|---|---|---|---|---|---|
| ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. | | | | | | |

### Two Minute Walk Test

Results for the 2MWT are summarized by visit in Figure 12 and statistically in Table 30.

Statistically subjects did significantly better in walking distance while being treated with dalfampridine-ER compared to when they were untreated. When comparing walking distance by period, subjects also showed a statistically significant improvement in walking distance when on-drug. Subjects showed a statistically significant improvement in walking distance in Period 1 (on-drug) compared to Period 2 (off-drug) and in Period 3 (on-drug) compared to Period 2.

**Table 30: Summary of On-Drug versus Off-Drug Results - Two Minute Walk Test (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Walking Distance (m) | Favors D-ER | ✔ | ✔ | ✔ | ✔ | ✔ |
| | Statistically significant | ✔ | ✔ | ✔ | ✔ | ✔ |

| | | | | | | |
|---|---|---|---|---|---|---|
| ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0.05. Note: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. | | | | | | |

### Timed 25 Foot Walk Test

Results for the T25FW are summarized by visit in Figure 13 and statistically in Table 31.

Statistically subjects did significantly better on the T25FW while being treated with dalfampridine-ER compared to when they were untreated. When comparing walking speed by period, subjects also showed a statistically significant improvement in speed. Subjects showed a statistically significant improvement in walking speed in Period 1 (on-drug) compared to Period 2 (off-drug) and in Period 3 (on-drug) compared to Period 2.

**Table 31: Summary of On-Drug versus Off-Drug Results - Timed 25 Foot Walk (FAP)**

| **Variable** | **Result^{2,3}** | **By Treatment** | **By Period** | | **By Visit** | |
|---|---|---|---|---|---|---|
| | | **P1 +P3 (on) vs P2 (off)** | **P1 (on) vs P2 (off)** | **P3 (on) vs P2 (off)** | **Baseline¹ (on) vs V4 (off)** | **V5 (on) vs V4 (off)** |
| Walking Speed (ft/s) | Favors D-ER | ✔ | ✔ | ✔ | ✔ | ✔ |
| | Statistically significant | ✔ | ✔ | ✔ | ✔ | ✔ |

| | | | | | | |
|---|---|---|---|---|---|---|
| ¹Baseline is the average of Visits 1 and 2. ²Favors D-ER - A checked box indicates that the estimated treatment difference was numerically in favor of being on-treatment (dalfampridine-ER). An unchecked box indicates that the estimated treatment difference was numerically in favor of being off-treatment (withdrawal). ³Statistically Significant - A checked box indicates p<0.05. An unchecked box indicates p≥0,05. Note: On-drug (dalfampridine-ER) = Periods 1 (Visits 1 and 2) and Period 3 (Visit 5); Off-drug (withdrawal) = Period 2 (Visits 3 and 4): Baseline = Visits 1 and 2. | | | | | | |

### Evaluator's Qualitative Assessment of Change

The Evaluator was not present during the NeuroCom system test. Results from the Evaluator's assessment of performance on the T25FW, based on review of videotapes, are summarized in Table 32. The first 4 (20%) subjects that enrolled into the study were not assessed as they entered before the protocol amendment allowing subjects to be recorded was implemented.

The Evaluator judged that a majority of subjects, 12 (60%), performed 'Somewhat Worse' at the first off-drug visit (Visit 3) compared to the second on-drug visit (Visit 2). One additional subject (5%) was much worse after withdrawal. The remaining 3 subjects (15%) had 'No Change.'

When comparing the second off-drug visit (Visit 4) to the first off-drug visit (Visit 3), the Evaluator judged that 8 (40%) of the subjects were 'Somewhat Worse'. Of the remaining 8 subjects with video recordings, 7 (35%) of the subjects had 'No Change' and 1 (5%) subject was 'Somewhat Improved.'

For the comparison between the second off-drug visit (Visit 4) and the second on-drug visit (Visit 2), the Evaluator judged that a majority of subjects, 13 (65%), performed 'Somewhat Worse' on the T25FW. One (5%) of the subjects was 'Much Worse' and the remaining 2 (10%) subjects that were assessed had 'No Change.'

**Table 32: Summary of the Evaluator's Assessment of Change on the Videotaped Timed 25 Foot Walk (FAP)**

| | **Full Analysis Population (N=20)** |
|---|---|
| Change from Visit 2 to Visit 3 | n (%) |
| Very much improved | 0 (0.0) |
| Much improved | 0 (0.0) |
| Somewhat improved | 0 (0.0) |
| No change | 3 (15.0) |
| Somewhat worse | 12 (60.0) |
| Much worse | 1 (5.0) |
| Very much worse | 0 (0.0) |
| Not done | 4 (20.0) |
| | |
| Change from Visit 3 to Visit 4 | n (%) |
| Very much improved | 0 (0.0) |
| Much improved | 0 (0.0) |
| Somewhat improved | 1 (5.0) |
| No change | 7 (35.0) |
| Somewhat worse | 8 (40.0) |
| Much worse | 0 (0.0) |
| Very much worse | 0 (0.0) |
| Not done | 4 (20.0) |
| | |
| Change from Visit 2 to Visit 4 | n (%) |
| Very much improved | 0 (0.0) |
| Much improved | 0 (0.0) |
| Somewhat improved | 0 (0.0) |
| No change | 2 (10.0) |
| Somewhat worse | 13 (65.0) |
| Much worse | 1 (5.0) |
| Very much worse | 0 (0.0) |
| Not done | 4 (20.0) |

### Additional Standardized NeuroCom Scores

In addition to the primary analyses, three sensitivity analyses were performed (see Section 6.3.3.6.5, "Secondary Efficacy Variables" for details). The three sensitivity analyses did not reveal any signals.

### 6.3.5.4.3 Correlation Analysis

The correlations between the primary efficacy variables and the non-NeuroCom measurements are presented in Table 33. There were high correlations between Overall Gait and all three measurements: BBS (0.76), 2MWT (0.70) and T25FW (0.71). The Overall Balance endpoint was moderately correlated to BBS (0.51), 2MWT (0.46) and T25FW (0.49).

**Table 33: Correlations between the Primary Efficacy Variables and the Non-NeuroCom Measures (FAP)**

| **Variable** | | **Berg Balance Scale** | **Two Minute Walk test** | **Timed 25 Foot Walk Test** |
|---|---|---|---|---|
| Overall Gait | Correlation Coefficient | 0.759 | 0.695 | 0.705 |
| | p-value | <0.001 | <0.001 | <0.001 |
| Overall Balance | Correlation Coefficient | 0.509 | 0.457 | 0.493 |
| | p-value | <0.001 | <0.001 | <0.001 |

### 6.3.5.4.4 Statistical and Analytical issues

Please refer to Section 6.3.3.6 for a discussion of statistical and analytical issues.

### Adjustments for Covariates and Handling of Dropouts or Missing Data

There were no dropouts in this study.

### Multiple Comparisons or Multiplicity

The two co-primary efficacy variables were analyzed using a step-down procedure to maintain an overall alpha-level of 0.05. To present the full clinical picture, p-values for all other analyses were presented although these comparisons were not protected for multiplicity.

For the mixed-model ANOVA comparing periods, all confidence intervals and p-values were adjusted using Tukey's Honest Significant Difference.

### 6.3.5.4.5 Efficacy Conclusions

The results of the primary endpoint analysis showed that there was a statistically significant difference in overall gait when subjects were treated with dalfampridine-ER compared to when they were not treated. Overall balance was not significantly affected based on NeuroCom composite scores.

The results for the individual gait NeuroCom tests were generally consistent with the results for the overall gait suggesting subjects did better while being treated with dalfampridine-ER than while not being treated and that drug withdrawal had a negative effect on gait. No clear pattern of effect on balance was seen on the individual NeuroCom tests.

BBS, 2MWT, and T25FW scores showed subjects did statistically better while treated with dalfampridine-ER than while not being treated and statistically significant deterioration when dalfampridine-ER was withdrawn, indicating detrimental effects of drug withdrawal on both gait and balance. When treatment resumed, subjects performed significantly better on all three measurements.

### 6.3.6 SAFETY EVALUATION

### 6.3.6.1. ADVERSE EVENTS

### 6.3.6.1.1 Brief Summary of Adverse Events

A total of 6 (30%) of the subjects reported at least one treatment-emergent AE (TEAE) in this study. The majority of the TEAEs were mild in severity. None of the TEAEs were deemed to be related to study drug.

No TEAEs led to study discontinuation and there were no SAEs reported among the enrolled subjects.

Table 34 contains an overview of TEAEs reported during the study.

**Table 34: Overall Summary of Adverse Events (Safety Population)**

| | **Safety Population (N=20)** |
|---|---|
| Number (%) of subjects with any TEAE | 6 (30.0) |
| Number (%) of subjects with any related TEAE | 0 |
| Number (%) of subjects with any TEAE by maximum severity | 6 (30.0) |
| Mild | 5 (25.0) |
| Moderate | 1 (5.0) |
| Severe | 0 |
| Number (%) of subjects with any TEAE leading to study discontinuation | 0 |
| Number (%) of subjects with any SAE | 0 |
| Number (%) of subjects with any TEAE leading to death | 0 |

| | |
|---|---|
| Note: TEAEs are defined as an AE with date of onset (or worsening) that occurs on or after the Screening Visit (Visit 1) through 30 days after the last dose of dalfampridine-ER 10 mg taken in Period 3. | |

### 6.3.6.1.2 Display of Adverse Events

Fall was the only TEAE reported in more than one subject. Five of the six TEAEs occurred during the withdrawal period (Period 2). Table 35 summarizes the TEAEs by descending frequency.

**Table 35: Treatment Emergent Adverse Events by Descending Frequency (Safety Population)**

| | **Safety Population (N=20)** | | | |
|---|---|---|---|---|
| **Preferred Term** | **Period 1** | **Period 2** | **Period 3** | **Total** |
| Number (%) of subjects with any TEAE | 1 (5.0) | 5 (25.0) | 0 | 6 (30.0) |
| Fall | 1 (5.0) | 1 (5.0) | 0 | 2 (10.0) |
| Fatigue | 0 | 1 (5.0) | 0 | 1 (5.0) |
| Gait disturbance | 0 | 1 (5.0) | 0 | 1 (5.0) |
| Hypertonia | 0 | 1 (5.0) | 0 | 1 (5.0) |
| Hypokinesia | 0 | 1 (5.0) | 0 | 1 (5.0) |
| Muscular weakness | 0 | 1 (5.0) | 0 | 1 (5.0) |
| Nausea | 0 | 1 (5.0) | 0 | 1 (5.0) |
| Presyncope | 0 | 1 (5.0) | 0 | 1 (5.0) |
| Vertigo | 0 | 1 (5.0) | 0 | 1 (5.0) |
| Vision blurred | 0 | 1 (5.0) | 0 | 1 (5.0) |

### 6.3.6.2. DEATHS, OTHER SERIOUS ADVERSE EVENTS, AND OTHER SIGNIFICANT ADVERSE EVENTS

There were no deaths in this study (Table 34).

### 6.3.6.3. VITAL SIGNS, PHYSICAL FINDINGS, AND OTHER OBSERVATIONS RELATED TO SAFETY

A summary of all clinically significant vital signs is provided in Table 36. There were three clinically significant vital sign findings during the study. Two of the findings, increased pulse rate and increased systolic blood pressure, occurred during the off-drug period (Visits 3 and 4; Period 2). The third finding, increased systolic blood pressure, was during the re-initiation period (Visit 5; Period 3).

**Table 36: Summary of Clinically Significant Vital Signs Results (Safety Population)**

| **Measurement** | **Safety Population** |
|---|---|
| **Study Visit/Day** | **(N=20)** |
| | n (%) |
| Supine Pulse Rate (beats/minute) | |
| Visit 4/Day 11 (D-ER Withdrawn) | |
| ≥105 beats/min and increase from baseline ≥15 beats/min | 1 (5.0) |
| | |
| Supine Systolic Blood Pressure (mmHg) | |
| Visit 3/Day 5 (D-ER Withdrawn) | |
| ≥160 mmHg and increase from baseline ≥20 mmHg | 1 (5.0) |
| | |
| Visit 5/Day 15 (D-ER) | |
| ≥160 mmHg and increase from baseline ≥20 mmHg | 1 (5.0) |

### 6.3.6.4. SAFETY CONCLUSIONS

Withdrawal of subjects' dalfampridine-ER treatment for 10 days did not result in any clinically significant safety findings, as indicated by AE reporting and vital sign measurements.

Subject safety and tolerability profiles were similar while treated with dalfampridine-ER compared to untreated. A total of 30% of the subjects reported one TEAE with the majority of the TEAEs being mild in severity and none were judged as related to the study drug. No TEAEs led to study discontinuation and there were no SAEs or deaths. The tolerability profile was consistent with previous studies.

### 6.3.7 DISCUSSION AND OVERALL CONCLUSIONS

This was a single-center, open-label, signal detection study to evaluate the effects of dalfampridine-ER withdrawal on gait and postural balance variables in subjects diagnosed with MS and Improvers in response to treatment with dalfampridine-ER.

The study consisted of three periods: a 7 day on-drug screening phase, a 10 day off-drug phase, and a second 4 day on-drug phase.

There were 20 subjects enrolled in the study and all completed the study. Sixty percent of the subjects were female, 90% were white, and the mean age was 53 years. On average, subjects were taking dalfampridine-ER for approximately one year prior to study enrollment.

The two primary endpoints for this study were tested in a step down procedure and based on comparing on-drug versus off-drug periods. The first endpoint was a composite score assessing the subject's overall gait. The overall gait composite score was predicated on composite scores from three of the tests from the NeuroCom system: WA, TW, and SQT. The second endpoint was a composite score assessing the subject's overall balance. The overall balance composite score was predicated on composite scores from three different tests from the NeuroCom system: SOT, ADT, and LOS.

Statistically, subjects did significantly better on overall gait p=0.015) while being treated with dalfampridine -ER (Periods 1 and 3) than when untreated (Period 2). Results for the other endpoint, overall balance, were not statistically significant with respect to treatment (p = 0.434).

Comparing the individual study periods, overall gait numerically showed deterioration in the subject's gait during dalfampridine-ER withdrawal (Period 1 [on-drug] versus Period 2 [off-drug]), but this difference was not statistically significant. When treatment resumed, there was a statistically significant difference between Period 3 (on-drug) and Period 2 (off-drug) for overall gait. For overall balance, there did not appear to be deterioration in the subject's balance during dalfampridine-ER withdrawal. When treatment resumed, there was a statistically significant improvement in balance which could be partially explained by a learning effect but also by a possible drug effect. A recent study showed a learning effect when provided five repetitions of the SOT over a 2-week period (Wrisley et al., 2007, Arch Phys Med Rehabil. 88:1049-1054). The learning effect appeared to plateau in the composite score around the 3^{rd} or 4^{th} session. This study showed that improvements of more than 8 points in the composite score indicate recovery beyond the effect of adaptation to the SOT itself.

The results for the individual gait NeuroCom tests were generally consistent with the results for the overall gait suggesting subjects did better while being treated with dalfampridine-ER than while not being treated and that drug withdrawal had a negative effect on gait. No clear pattern of effect on balance was seen on the individual NeuroCom tests.

BBS, 2MWT, and T25FW scores showed statistically significant deterioration when dalfampridine-ER was withdrawn, indicating detrimental effects of drug withdrawal on both gait and balance. When treatment resumed, subjects performed significantly better on all three measurements in comparison to the off-drug period.

### 6.3.8 REFERENCE LIST FOR EXAMPLE 3

- Frohman EM. Multiple sclerosis. The Medical clinics of North America. 2003;87: 867-897.
- Goodman A, Brown T, Krupp L, et al. Sustained-release oral fampridine in multiple sclerosis: A randomised, double-blind, controlled trial. Lancet. 2009;373:732-738.
- Goodman A, Brown T, Edwards K, et al. A phase 3 trial of extended release oral dalfampridine in multiple sclerosis. Ann Neurol. 2010;68:494-502.
- Berg K, Wood-Dauphinée SL, Williams JI, Gayton D. Measuring balance in the elderly: preliminary development of an instrument. Physiother Canada. 1989;41:304-311.
- Tinetti ME. Performance-oriented assessment of mobility problems in elderly patients. [Research Support, Non-U.S. Gov't Research Support, U.S. Gov't, P.H.S.]. Journal of the American Geriatrics Society. 1986;34(2):119-126.
- Berg K, Wood-Dauphinee S, Williams JI. The Balance Scale: reliability assessment with elderly residents and patients with an acute stroke. [Research Support, Non-U.S. Gov't]. Scandinavian journal of rehabilitation medicine. 1995;27(1):27-36.
- Newstead AH, Hinman MR, Tomberlin JA. Reliability of the Berg Balance Scale and balance master limits of stability tests for individuals with brain injury. [Clinical Trial Validation Studies]. Journal of neurologic physical therapy. 2005;29(1):18-23.
- Berg KO, Maki BE, Williams JI, Holliday PJ, Wood-Dauphinee SL. Clinical and laboratory measures of postural balance in an elderly population. [Research Support, Non-U.S. Gov't]. Archives of physical medicine and rehabilitation. 1992;73(11): 1073-1080.
- Berg KO, Wood-Dauphinee SL, Williams JI, Maki B. Measuring balance in the elderly: validation of an instrument. [Comparative Study Research Support, Non-U.S. Gov't]. Canadian journal of public health. Revue canadienne de santé publique. 1992;83(suppl 2):S7-11.
- Bogle Thorbahn LD, Newton RA. Use of the Berg Balance Test to predict falls in elderly persons. Physical therapy. 1996;76(6):576-583;discussion 576-583.
- Creel GL, Light KE, Thigpen MT. Concurrent and construct validity of scores on the Timed Movement Battery. [Clinical Trial Randomized Controlled Trial]. Physical therapy. 2001;81(2):789-798.
- Shumway-Cook A, Baldwin M, Polissar NL, Gruber W. Predicting the probability for falls in community-dwelling older adults. [Research Support, Non-U.S. Gov't]. Physical therapy. 1997;77(8):812-819.
- Riddle DL, Stratford PW. Interpreting validity indexes for diagnostic tests: an illustration using the Berg balance test. Physical therapy. 1999;79(10):939-948.
- Gaston G. The Godin-Shephard Leisure-Time Physical Activity Questionnaire. Health and Fitness Journal of Canada. 2011; 4(1): 18-22.
- Wrisley DM, Stephens MJ, Mosley S. Learning Effects on Repetitive Administrations of the Sensory Organization Test in Healthy Young Adults. Arch Phys Med Rehabil. 2007;88,1049-1054.

### 7. INCORPORATION BY REFERENCE

Various references such as patents, patent applications, and publications are cited herein, the disclosures of which are hereby incorporated by reference herein in their entireties.

The invention is further illustrated by the following non-limiting emdodiments.
1. A method for treating an impairment in gait and/or balance in a patient with multiple sclerosis, said method comprising administering to the patient an aminopyridine or a pharmaceutically acceptable salt thereof.
2. The method of embodiment 1, comprising administering to the patient an aminopyridine.
3. The method of embodiment 1, comprising administering to the patient a pharmaceutically acceptable salt of an aminopyridine.
4. The method of any one of embodiments 1-3, wherein the patient is a human.
5. The method of any one of embodiments 1-4, wherein the aminopyridine is a mono-aminopyridine or a diaminopyridine.
6. The method of any one of embodiments 1-5, wherein the aminopyridine is 4- aminopyridine.
7. The method of any one of embodiments 1-6, wherein the aminopyridine or the pharmaceutically acceptable salt thereof is in a sustained release composition.
8. The method of any one of embodiments 1-6, wherein the aminopyridine or the pharmaceutically acceptable salt thereof is in an immediate release composition.
9. The method of any one of embodiments 1-8, wherein the aminopyridine or the pharmaceutically acceptable salt thereof is administered to the patient once daily.
10. The method of any one of embodiments 1-8, wherein the aminopyridine or the pharmaceutically acceptable salt thereof is administered to the patient twice daily.
11. The method of any one of embodiments 1-10, wherein the aminopyridine or the pharmaceutically acceptable salt thereof is administered in an amount in the range of 5 to 20 mg, 5 to 15 mg, 5 to 10 mg, or 7.5 to 10 mg once or twice daily.
12. The method of embodiment 7, wherein the aminopyridine or the pharmaceutically acceptable salt thereof is administered in a sustained release composition in an amount of the aminopyridine or a pharmaceutically acceptable salt thereof in the range of 5 to 20 mg, 5 to 15 mg, 5 to 10 mg, or 7.5 to 10 mg once or twice daily.
13. The method of embodiment 11 or 12, wherein the aminopyridine or the pharmaceutically acceptable salt thereof is administered in the amount of 5 mg twice daily.
14. The method of embodiment 11 or 12, wherein the aminopyridine or the pharmaceutically acceptable salt thereof is administered in the amount of 7.5 mg twice daily.
15. The method of embodiment 11 or 12, wherein the aminopyridine or the pharmaceutically acceptable salt thereof is administered in the amount of 10 mg twice daily.
16. The method of embodiment 11 or 12, wherein the aminopyridine or the pharmaceutically acceptable salt thereof is administered in the amount of 10 mg once daily.
17. The method of embodiment 11 or 12, wherein the aminopyridine or the pharmaceutically acceptable salt thereof is administered in the amount of 15 mg once daily.
18. The method of embodiment 11 or 12, wherein the aminopyridine or the pharmaceutically acceptable salt thereof is administered in the amount of 20 mg once daily.
19. The method of any one of embodiments 1-18, wherein the aminopyridine or the pharmaceutically acceptable salt thereof is administered orally.
20. The method of embodiment 19, wherein the aminopyridine or the pharmaceutically acceptable salt thereof is formulated in a form of a tablet or capsule.
21. The method of any one of embodiments 1-20, wherein the patient has been diagnosed with an impairment in gait and/or balance.
22. The method of any one of embodiments 1-21, wherein the impairment in gait and/ or balance is an impairment in gait.
23. The method of embodiment 22, wherein the impairment in gait is determined by measuring at least one parameter of gait that is not walking speed.
24. The method of embodiment 22 or 23, wherein the impairment in gait is an impairment in one or more parameters of gait, wherein the one or more parameters of gait are selected from the group consisting of step length, step width, step speed and turn sway, and wherein the impairment in gait is an impairment in at least one of said one or more parameters of gait that is not step speed.
25. The method of embodiment 22, wherein the impairment in gait is an impairment in step length.
26. The method of embodiment 22, wherein the impairment in gait is an impairment in step width.
27. The method of embodiment 22, wherein the impairment in gait is an impairment in turn sway.
28. The method of any one of embodiments 22-27, wherein the impairment in gait is an impairment assayable by the NeuroCom SMART Balance Master®.
29. The method of any one of embodiments 22-28, wherein the patient has been diagnosed with an impairment in gait.
30. The method of embodiment 29, which is effective to improve the impairment in gait in the patient.
31. The method of any one of embodiments 1-21, wherein the impairment in gait and/or balance is an impairment in balance.
32. The method of embodiment 31, wherein the impairment in balance is an impairment in balance during motion.
33. The method of embodiment 32, wherein the motion is walking, jogging or running.
34. The method of embodiment 31, wherein the impairment in balance is an impairment in balance in a stationary position.
35. The method of embodiment 31 or 34, wherein the impairment in balance is an impairment in postural balance.
36. The method of embodiment 35, wherein the impairment in postural balance is an impairment in postural balance during sitting, standing, reaching, maintaining single-leg stance or turning.
37. The method of embodiment 34, wherein the impairment in balance in a stationary position is an impairment in rotational balance.
38. The method of any one of embodiments 31-37, wherein the patient has been diagnosed with an impairment in balance.
39. The method of embodiment 38, which is effective to improve the impairment in balance in the patient.
40. The method of any one of embodiments 1-39, further comprising assessing the level of said impairment after repeated administering of the aminopyridine or a pharmaceutically acceptable salt thereof.
41. The method of any one of embodiments 7 or 12-18, wherein said sustained release composition provides a Tₘₐₓ of about 2 hours to about 6 hours in a human.
42. A method for treating an impairment in stationary balance in a patient with multiple sclerosis, said method comprising administering to the patient 4-aminopyridine in a sustained release composition.
43. The method of embodiment 42, wherein the 4-aminopyridine is administered in an amount of 10 mg twice daily.
44. The method of embodiment 42 or 43, wherein the impairment in balance is an impairment in balance during sitting, standing, reaching, maintaining single-leg stance or turning.
45. The method of any one of embodiments 42-44, wherein the impairment in balance is an impairment assayable by the Berg Balance Scale.
46. The method of any one of embodiments 42-45, wherein the patient has been diagnosed with an impairment in stationary balance.
47. The method of embodiment 46, which is effective to improve the impairment in stationary balance in the patient.
48. A method for increasing step length, decreasing step width or decreasing turn sway in a patient with multiple sclerosis, said method comprising administering to the patient 4-aminopyridine in a sustained release composition.
49. The method of embodiment 48, wherein the 4-aminopyridine is administered in an amount of 10 mg twice daily.
50. The method of embodiment 48 or 49, wherein the step length, step width or turn sway are assayable by the NeuroCom SMART Balance Master®.
51. The method of any one of embodiments 48-50, which is effective to increase step length, decrease step width or decrease turn sway in the patient.
52. The method of any one of embodiments 1-51, wherein the patient has relapsing remitting, secondary progressive, primary progressive, or progressive relapsing type of multiple sclerosis.
53. The method of embodiment 52, wherein the patient has relapsing remitting type of multiple sclerosis.

## Claims

1. An aminopyridine or a pharmaceutically acceptable salt thereof for use in a method for treating an impairment in postural balance or an impairment in balance assayable by the Berg Balance Scale in a human patient with multiple sclerosis in need thereof, said method comprising orally administering to the patient an aminopyridine or a pharmaceutically acceptable salt thereof in a sustained release composition.

2. The aminopyridine or a pharmaceutically acceptable salt thereof for use of claim 1, wherein the aminopyridine or pharmaceutically acceptable salt thereof is the aminopyridine and not the pharmaceutically acceptable salt thereof.

3. The aminopyridine or a pharmaceutically acceptable salt thereof for use of claim 1 or 2, wherein the aminopyridine is a mono-aminopyridine or a diaminopyridine.

4. The aminopyridine or a pharmaceutically acceptable salt thereof for use of any one of claims 1-3, wherein the aminopyridine is 4-aminopyridine.

5. The aminopyridine or a pharmaceutically acceptable salt thereof for use of any one of claims 1-4, wherein said sustained release composition provides a Tmax of about 2 hours to about 6 hours in a human.

6. The aminopyridine or a pharmaceutically acceptable salt thereof for use of any one of claims 1-5, wherein the aminopyridine or the pharmaceutically acceptable salt thereof is administered to the patient once daily.

7. The aminopyridine or a pharmaceutically acceptable salt thereof for use of any one of claims 1-5, wherein the aminopyridine or the pharmaceutically acceptable salt thereof is administered to the patient twice daily.

8. The aminopyridine or a pharmaceutically acceptable salt thereof for use of any one of claims 1-7, wherein the aminopyridine is administered in an amount in the range of 5 to 20 mg, 5 to 15 mg, 5 to 10 mg, or 7.5 to 10 mg once or twice daily, for example, in the amount of 5 mg, 7.5 mg or 10 mg twice daily, or in the amount 10 mg, 15 mg or 20 mg once daily.

9. The aminopyridine or a pharmaceutically acceptable salt thereof for use of claim 8, wherein the aminopyridine is administered in the amount of 10 mg twice daily.

10. The aminopyridine or a pharmaceutically acceptable salt thereof for use of any one of claims 1-9, wherein the aminopyridine or the pharmaceutically acceptable salt thereof is in a form of a tablet or a capsule.

11. The aminopyridine or a pharmaceutically acceptable salt thereof for use of any one of claims 1-10, wherein the patient has been diagnosed with an impairment in balance.

12. The aminopyridine or a pharmaceutically acceptable salt thereof for use of any one of claims 1-11, wherein the use is in a method for treating an impairment in postural balance.

13. The aminopyridine or a pharmaceutically acceptable salt thereof for use of any one of claims 1-12, wherein the impairment in postural balance is an impairment in balance during sitting, standing, reaching, maintaining single-leg stance or turning.

14. The aminopyridine or a pharmaceutically acceptable salt thereof for use of claim 13, wherein the impairment in postural balance is an impairment in balance during sitting, maintaining single-leg stance or turning.

15. The aminopyridine or a pharmaceutically acceptable salt thereof for use of any one of claims 1-11, wherein the use is in a method for treating an impairment assayable by the Berg Balance Scale.

16. The aminopyridine or a pharmaceutically acceptable salt thereof for use of any one of claims 1-15, wherein the patient has relapsing remitting type of multiple sclerosis.
